(19) Europäisches Patentamt
European Patent Office
Office européen des brevets

(11) EP 4 011 865 A1

(12) EUROPEAN PATENT APPLICATION
published in accordance with Art. 153(4) EPC

(43) Date of publication:
**15.06.2022 Bulletin 2022/24**

(21) Application number: **20851903.3**

(22) Date of filing: **05.08.2020**

(51) International Patent Classification (IPC):
***C07D 239/42*** *(2006.01)* ***C07D 403/12*** *(2006.01)*
***A61K 31/506*** *(2006.01)* ***A61P 37/00*** *(2006.01)*
***A61P 35/00*** *(2006.01)*

(52) Cooperative Patent Classification (CPC):
**A61K 31/506; A61P 35/00; A61P 37/00; C07D 239/42; C07D 403/12**

(86) International application number:
**PCT/CN2020/107054**

(87) International publication number:
**WO 2021/027647 (18.02.2021 Gazette 2021/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **09.08.2019 CN 201910733723**

(71) Applicant: **The National Institutes of Pharmaceutical R&D Co., Ltd**
**Changping District**
**Beijing 102206 (CN)**

(72) Inventors:
- **YIN, Huijun**
  **Beijing 102206 (CN)**
- **YAN, Xu**
  **Beijing 102206 (CN)**
- **SHI, Jizhou**
  **Beijing 102206 (CN)**
- **LIU, Guobiao**
  **Beijing 102206 (CN)**
- **FEI, Teng**
  **Beijing 102206 (CN)**
- **DONG, Liuxin**
  **Beijing 102206 (CN)**
- **LIU, Yuanye**
  **Beijing 102206 (CN)**

(74) Representative: **EP&C**
**P.O. Box 3241**
**2280 GE Rijswijk (NL)**

# (54) BRIDGED HETEROCYCLYL-SUBSTITUTED PYRIMIDINE COMPOUND, PREPARATION METHOD THEREFOR, AND PHARMACEUTICAL USE THEREOF

(57) A bridged heterocyclyl-substituted pyrimidine compound, a preparation method therefor, and pharmaceutical use thereof. In particular, the present invention relates to a compound represented by general formula (I), a preparation method for the compound, a pharmaceutical composition containing the compound, use of the compound as a JAK1 and TYK2 kinase inhibitor, and use in treating diseases related to JAK1 and TYK2 kinase activity, such as inflammations, autoimmune diseases, and cancers. The definition of each substituent in general formula (I) is the same as that in the description.

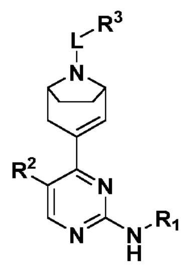

(I)

EP 4 011 865 A1

## Description

### TECHNICAL FIELD

The invention belongs to the technical field of medicine, and specifically relates to a bridged heterocyclyl-substituted pyrimidine compound, a preparation method therefor and a pharmaceutical composition containing the same, as well as use thereof for regulating Janus kinase 1 (JAK1) and tyrosine protein kinase 2 (TYK2) activity and use thereof in treating and/or preventing diseases related to JAK1 and TYK2 activity.

### BACKGROUND

The process of intracellular signaling transduction is an effective way for cells to respond to external stimuli to ultimately trigger specific biological effects. Cytokines can carry out intracellular signaling transduction through a variety of signaling transduction pathways, thereby being involved in the regulation of hematopoietic function and many important biological functions related to immunity. The Janus kinase (JAK) family of protein tyrosine kinases and transcriptional activators (STAT) play an important role in the process of cytokine signaling transduction (J. Immunol. 2015, 194, 21).

The Janus kinase (JAK) family plays a certain role in the cytokine-dependent regulation of cell proliferation and function involved in immune response. Currently, there are four known mammalian JAK family members: JAK1 (also known as Janus kinase-1), JAK2 (also known as Janus kinase-2), JAK3 (also known as Janus kinase, leukocyte, JAKL1, L-JAK and Janus kinase-3), Tyk2 (also known as protein-tyrosine kinase 2). JAK1, JAK2 and Tyk2 are widely present in various tissues and cells, while JAK3 is only present in the bone marrow and lymphatic system (J. Med. Chem. 2014, 57, 5023).

Tyk2 is the first JAK kinase discovered. It plays an important role in regulating the biological response of IL-12 and bacterial lipopolysaccharide (LPS), and is also involved in signal transduction pathways mediated by IL-6, IL-10 and IL-12. Targeting Tyk2 can become a new strategy for treating diseases mediated by IL-12, IL-23 or type I IFN, said diseases include but are not limited to rheumatoid arthritis, multiple sclerosis, lupus, psoriasis, psoriatic arthritis, inflammatory bowel disease, uveitis, sarcoidosis, lupus erythematosus and cancer.

JAK1 plays an important role in regulating the biological response function of multiple cytokine receptor families. JAK1 gene knockout mice have an early postnatal lethal factor phenotype, and the nervous system is also damaged, resulting in birth defects in young mice. Studies have shown that JAK1 gene knockout mice will have thymocyte and B cell secretion defects, and JAK1 gene knockout tissues have significantly weakened response to LIF, IL-6 and IL-10. Clinical trials have shown that JAK1 inhibitors have shown good efficacy in treating inflammatory and autoimmune diseases such as rheumatoid arthritis, ulcerative colitis, Crohn’s disease, lupus erythematosus, alopecia areata, atopic dermatitis.

Upon cytokine binding to receptor, the receptor forms a dimer which approaches the JAK coupled with the receptor to active JAK by phosphorylation of tyrosine residues. Activated JAKs phosphorylate specific tyrosine residues in the intracellular domain of the receptor, creating docking sites for STATs.. Signal Transducer and Activator of Transcription (STAT) is a group of cytoplasmic proteins that can regulate target genes and bind with DNA. The STAT family includes STAT1, STAT2, STAT3, STAT4, STAT5a, STAT5b and StAT6. STAT recognizes the "docking site" through the SH2 domain, and is activated by phosphorylation of its C-terminus tyrosine residue by JAK kinase. The activated STAT factor is transferred into the nucleus and plays an important role in regulating the innate and acquired host immune response.

The activation of JAK/STAT signaling transduction pathway promotes the occurrence of various diseases, including but not limited to many abnormal immune responses, such as allergies, asthma, rheumatoid arthritis, amyotrophic lateral sclerosis, multiple sclerosis and the like. It is also associated with cancers such as leukemia (acute myeloid leukemia and acute lymphoblastic leukemia) and solid tumors (uterine leiomyosarcoma, prostate cancer) (Curr. Opin. Rheumatol. 2014, 26, 237).

In view of the important roles that JAK1 and TYK2 play in the inflammatory signaling pathways, drugs that can simultaneously inhibit both kinases have the potential of further enhancing the efficacy and bringing greater benefits to the patients.

### SUMMARY OF THE INVENTION

Through intensive research, the inventors have designed and synthesized a series of bridged heterocyclyl-substituted pyrimidine compounds, which have been screened for JAK1 and TYK2 activities. The research results show that these compounds have outstanding JAK1 and TYK2 inhibitory activities, and can be developed as a medicament for treating diseases related to JAK1 and TYK2 activity.

Thus, the objective of the present invention is to provide a compound represented by general formula (I), or a

mesomer, racemate, enantiomer, diastereomer thereof, or a mixture thereof, or a prodrug thereof, or a pharmaceutically acceptable salt thereof,

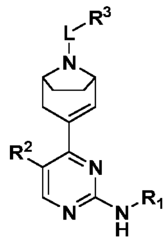

(I)

wherein:

$R^1$ is selected from the group consisting of cycloalkyl, heterocyclyl, aryl and heteroaryl, wherein the cycloalkyl, heterocyclyl, aryl and heteroaryl are each independently optionally further substituted with one or more of $R^4$;
each of $R^4$ is independently selected from the group consisting of halogen, amino, nitro, cyano, hydroxyl, thiol, oxo, alkyl, alkoxyl, cycloalkyl, heterocyclyl, aryl, heteroaryl, $-C(O)R^a$, $-O(O)CR^a$, $-C(O)OR^a$, $-C(O)NR^aR^b$, $NR^aR^b$, $-NHC(O)R^a$, $-S(O)_nR^a$, $-S(O)_nNR^aR^b$ and $-NHS(O)_nR^a$, wherein the alkyl, alkoxyl, cycloalkyl, heterocyclyl, aryl and heteroaryl are each independently optionally further substituted with one or more groups selected from the group consisting of halogen, amino, nitro, cyano, oxo, hydroxyl, thiol, carboxyl, alkoxycarbonylalkoxycarbonyl, alkyl, haloalkyl, alkoxyl, haloalkoxyl, alkenyl, alkynyl, cycloalkyl, heterocyclyl, aryl and heteroaryl;
$R^2$ is selected from the group consisting of hydrogen, halogen, amino, cyano, hydroxyl, thiol, carboxyl, alkyl, alkoxyl and cycloalkyl, wherein the alkyl, alkoxyl and cycloalkyl are each independently optionally further substituted with one or more groups selected from the group consisting of halogen, amino, nitro, cyano, oxo, hydroxyl, thiol, carboxyl, alkoxycarbonyl, alkyl, alkoxyl, alkenyl, alkynyl, cycloalkyl, heterocyclyl, aryl and heteroaryl;
L is selected from the group consisting of single bond, $-CR^5R^6-$, -C(O)-, -C(S)-, $-N(R^a)-$, $-S(O)_n-$, -O-, -S-, $-C(O)N(R^a)-$, $-C(O)-C(O)-N(R^a)-$ and $-S(O)_nN(R^a)-$;
$R^5$ and $R^6$ are each independently selected from the group consisting of hydrogen, halogen, hydroxyl, alkyl, alkoxyl, alkenyl, alkynyl, cycloalkyl, heterocyclyl, aryl and heteroaryl, wherein the alkyl, alkoxyl, alkenyl, alkynyl, cycloalkyl, heterocyclyl, aryl and heteroaryl are each independently optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;
or $R^5$ and $R^6$ together with the atom to which they are attached form a cycloalkyl or heterocyclyl, wherein the cycloalkyl or heterocyclyl is optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;
$R^3$ is selected from the group consisting of alkyl, cycloalkyl, heterocyclyl, aryl and heteroaryl, wherein the alkyl, cycloalkyl, heterocyclyl, aryl and heteroaryl are each independently optionally further substituted with one or more of $R^7$;
each of $R^7$ is independently selected from the group consisting of halogen, amino, nitro, cyano, hydroxyl, thiol, oxo, alkyl, alkoxyl, cycloalkyl, heterocyclyl, aryl, heteroaryl, $OR^a$, $-C(O)R^a$, $-O(O)CR^a$, $-C(O)OR^a$, $-C(O)NR^aR^b$, $NR^aR^b$, $-NHC(O)R^a$, $-S(O)_nR^a$, $-S(O)_nNR^aR^b$ and $-NHS(O)_nR^a$, wherein the alkyl, alkoxyl, cycloalkyl, heterocyclyl, aryl and heteroaryl are each independently optionally further substituted with one or more groups selected from the group consisting of halogen, amino, nitro, cyano, oxo, hydroxyl, thiol, carboxyl, alkoxycarbonyl, alkyl, haloalkyl, alkoxyl, haloalkoxyl, alkenyl, alkynyl, cycloalkyl, heterocyclyl, aryl and heteroaryl;
$R^a$ and $R^b$ are each independently selected from the group consisting of hydrogen, halogen, hydroxyl, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each independently optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;
or $R^a$ and $R^b$ together with the N-atom to which they are attached form a nitrogen-containing heterocyclyl, wherein the nitrogen-containing heterocyclyl is optionally further substituted with one or more groups selected from the group consisting of halogen, amino, nitro, cyano, oxo, hydroxyl, thiol, carboxyl, alkoxycarbonyl, alkyl, alkoxyl, alkenyl,

alkynyl, cycloalkyl, heterocyclyl, aryl and heteroaryl; and
n is 0, 1 or 2.

In a preferred embodiment of the present invention, the compound represented by general formula (I) according to the present invention, or a mesomer, racemate, enantiomer, diastereomer thereof, or a mixture thereof, or a prodrug thereof, or a pharmaceutically acceptable salt thereof, is a compound represented by general formula (II), or a mesomer, racemate, enantiomer, diastereomer thereof, or a mixture thereof, or a prodrug thereof, or a pharmaceutically acceptable salt thereof:

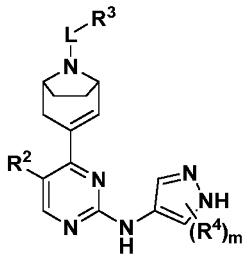

(II)

wherein, $R^2$, $R^3$, $R^4$ and L are as defined in the compound of general formula (I); and
m is 0, 1, 2 or 3.

In a preferred embodiment of the present invention, in the compound represented by general formula (I) according to the present invention, or a mesomer, racemate, enantiomer, diastereomer thereof, or a mixture thereof, or a prodrug thereof, or a pharmaceutically acceptable salt thereof,

$R^3$ is selected from the group consisting of alkyl, cycloalkyl and heterocyclyl, wherein the alkyl, cycloalkyl and heterocyclyl are each independently optionally further substituted with one or more of $R^7$;
and $R^7$ is as defined in claim 1, preferably selected from the group consisting of halogen, cyano, aryl, cycloalkyl and alkyl, wherein the cycloalkyl and alkyl are each independently optionally substituted with one or more halogen(s).

In another preferred embodiment of the present invention, in the compound represented by general formula (I) according to the present invention, or a mesomer, racemate, enantiomer, diastereomer thereof, or a mixture thereof, or a prodrug thereof, or a pharmaceutically acceptable salt thereof,

L is selected from the group consisting of a single bond, $-CR^5R^6-$, -C(O)-, $-S(O)_n-$, -O-, -S-, $-C(O)N(R^a)-$, $C(O)-C(O)-N(R^a)-$ and $-S(O)_nN(R^a)-$, preferably $-S(O)_n-$, -C(O)-, $-C(O)N(R^a)-$ and $-S(O)_nN(R^a)-$, more preferably -C(O)- and $-C(O)N(R^a)-$;
wherein, $R^5$, $R^6$, $R^a$ and n are as defined in the compound of general formula (I).

In a preferred embodiment of the present invention, in the compound represented by general formula (I) according to the present invention, or a mesomer, racemate, enantiomer, diastereomer thereof, or a mixture thereof, or a prodrug thereof, or a pharmaceutically acceptable salt thereof,

$R^2$ is selected from the group consisting of hydrogen, halogen, cyano, hydroxyl, carboxyl, alkyl and cycloalkyl, preferably hydrogen, halogen, cyano and alkyl, more preferably hydrogen and halogen.

In another preferred embodiment of the present invention, in the compound represented by general formula (I) according to the present invention, or a mesomer, racemate, enantiomer, diastereomer thereof, or a mixture thereof, or a prodrug thereof, or a pharmaceutically acceptable salt thereof,

$R^1$ is selected from the group consisting of cycloalkyl, heterocyclyl, aryl and heteroaryl, preferably aryl and heteroaryl, more preferably heteroaryl, wherein the cycloalkyl, heterocyclyl, aryl and heteroaryl are each independently optionally further substituted with one or more of $R^4$; and
$R^4$ is as defined in the compound of general formula (I), preferably alkyl.

Typical compounds of the present invention include, but are not limited to:

| Example No. | Structure and name |
|---|---|
| 1 | 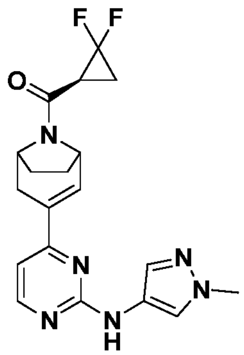 |
| | ((*S*)-2,2-difluorocyclopropyl)(3-(2-((1-methyl-1*H*-pyrazol-4-yl)amino) pyrimidin-4-yl)-8-azabicyclo [3.2.1]oct-2-en-8-yl)methanone |
| 1-a and 1-b | 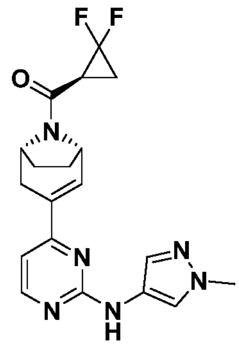 |
| | ((*S*)-2,2-difluorocyclopropyl)((*1S*,*5R*)-3-(2-((1-methyl-1*H*-pyrazol-4-yl) amino)pyrimidin-4-yl)-8-azabicyclo[3.2.1]oct-2-en-8-yl)methanone |
| | 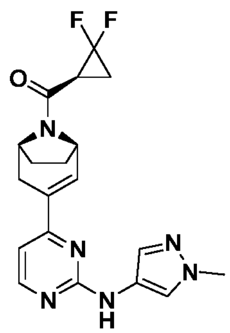 |
| | ((*S*)-2,2-difluorocyclopropyl)((*1R*,*5S*)-3-(2-((1-methyl-1*H*-pyrazol-4-yl) amino)pyrimidin-4-yl)-8-azabicyclo[3.2.1]oct-2-en-8-yl)methanone |
| 2 | 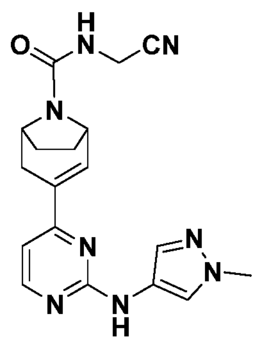 |
| | *N*-(cyanomethyl)-3-(2-((1-methyl-1*H*-pyrazol-4-yl)amino)pyrimidin-4-yl) -8-azabicyclo[3.2.1]oct-2-ene-8-carboxamide |

(continued)

| Example No. | Structure and name |
|---|---|
| 3 | 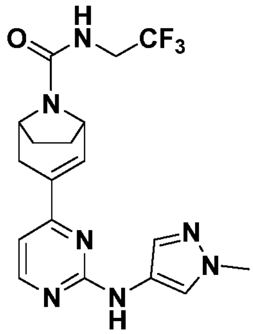 |
| | 3-(2-((1-Methyl-1*H*-pyrazol-4-yl)amino)pyrimidin-4-yl)-*N*-(2,2,2-trifluoroethyl)-8-azabicyclo[3.2.1]oct-2-ene-8-carboxamide |
| 3-a and 3-b | 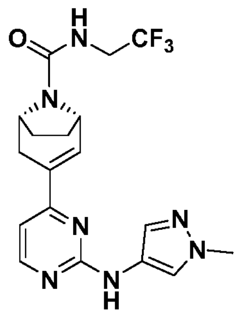 |
| | (*1S,5R)*-3-(2-((1-methyl-1*H*-pyrazol-4-yl)amino)pyrimidin-4-yl)-*N*-(2,2,2 -trifluoroethyl)-8-azabicyclo[3.2.1]oct-2-ene-8-carboxamide |
| | 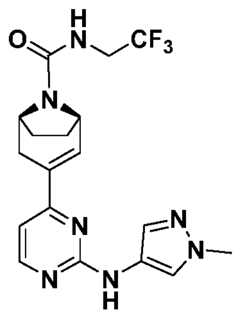 |
| | (*1R,5S*)-3-(2-((1-methyl-1*H*-pyrazol-4-yl)amino)pyrimidin-4-yl)-*N*-(2,2,2 -trifluoroethyl)-8-azabicyclo[3.2.1]oct-2-ene-8-carboxamide |
| 4 | 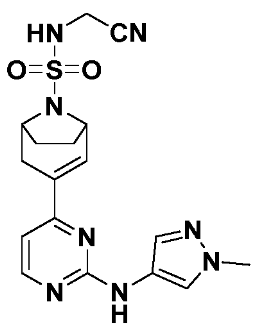 |
| | *N*-(cyanomethyl)-3-(2-((1-methyl-1*H*-pyrazol-4-yl)amino)pyrimidin-4-yl) -8-azabicyclo[3.2.1]oct-2-ene-8-sulfonamide |

(continued)

| Example No. | Structure and name |
|---|---|
| 5 | 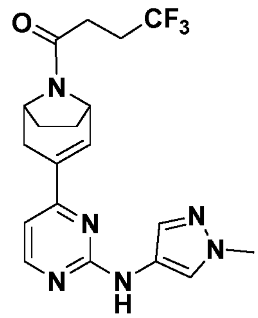 |
| | 4,4,4-Trifluoro-1-(3-(2-((1-methyl-1*H*-pyrazol-4-yl)amino)pyrimidin-4-yl)-8-azabicyclo[3.2.1]oct-2-en-8-yl)butan-1-one |
| 6 | 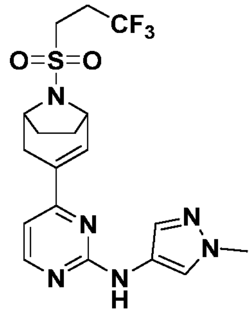 |
| | *N*-(1-methyl-1*H*-pyrazol-4-yl)-4-(8-((3,3,3-trifluoropropyl)sulfonyl)-8-azabicyclo[3.2.1]oct-2-en-3-yl)pyrimidin-2-amine |
| 7 | 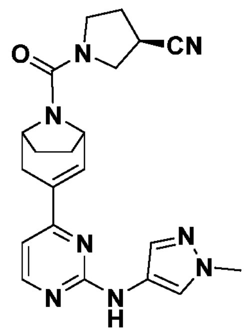 |
| | (*R*)-1-(3-(2-((1-methyl-1*H*-pyrazol-4-yl)amino)pyrimidin-4-yl)-8-aza bicyclo[3.2.1]oct-2-ene-8-carbonyl)pyrrolidine-3-nitrile |
| 8 | 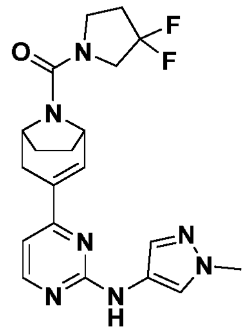 |
| | (3,3-Difluoropyrrolidin-1-yl)(3-(2-((1-methyl-1*H*-pyrazol-4-yl)amino) pyrimidin-4-yl)-8-azabicyclo [3.2.1]oct-2-en-8-yl)methanone |

(continued)

| Example No. | Structure and name |
| --- | --- |
| 9 | 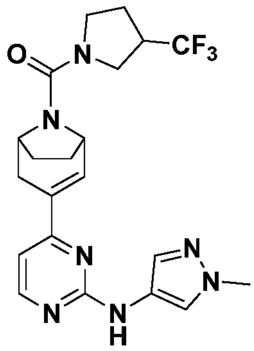 |
| | (3-(2-((1-Methyl-1*H*-pyrazol-4-yl)amino)pyrimidin-4-yl)-8-azabicyclo [3.2.1]oct-2-en-8-yl)(3-(trifluoromethyl)pyrrolidin-1-yl)methanone |
| 10 | 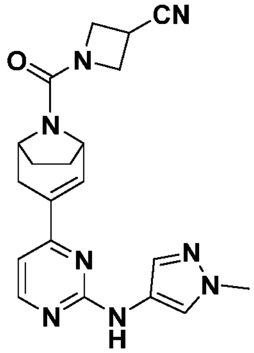 |
| | 1-(3-(2-((1-Methyl-1*H*-pyrazol-4-yl)amino)pyrimidin-4-yl)-8-azabicyclo [3.2.1]oct-2-ene-8-carbonyl)azetidine-3-nitrile |
| 11 | 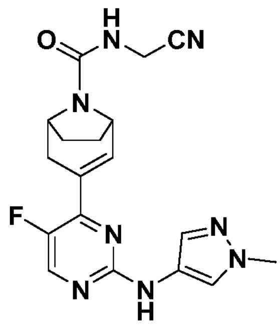 |
| | *N*-(cyanomethyl)-3-(5-fluoro-2-((1-methyl-1*H*-pyrazol-4-yl)amino) pyrimidin-4-yl)-8-azabicyclo [3.2.1]oct-2-ene-8-carboxamide |

(continued)

| Example No. | Structure and name |
|---|---|
| 12 | 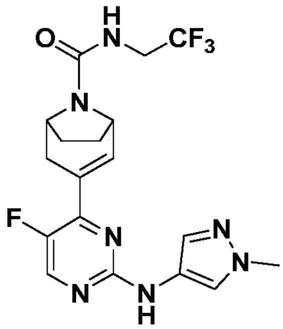 |
| | 3-(5-Fluoro-2-((1-methyl-1*H*-pyrazol-4-yl)amino)pyrimidin-4-yl)-*N*-(2,2, 2-trifluoroethyl)-8-azabicyclo[3.2.1]oct-2-ene-8-carboxamide |
| 12-a and 12-b | 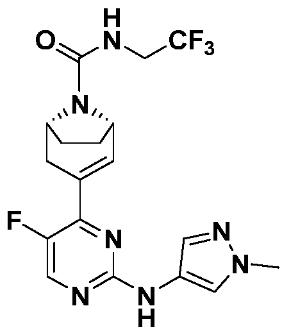 |
| | (*1S,5R*)-3-(5-fluoro-2-((1-methyl-1*H*-pyrazol-4-yl)amino)pyrimidin-4-yl)-*N*-(2,2,2-trifluoroethyl)-8-azabicyclo[3.2.1]oct-2-ene-8-carboxamide |
| | 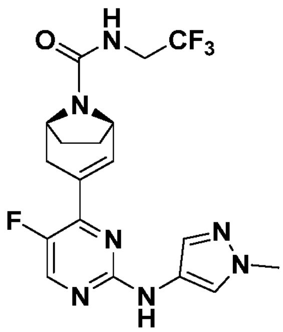 |
| | (*1R,5S*)-3-(5-fluoro-2-((1-methyl-1*H*-pyrazol-4-yl)amino)pyrimidin-4-yl)-*N*-(2,2,2-trifluoroethyl)-8-azabicyclo[3.2.1]oct-2-ene-8-carboxamide |
| 13 | 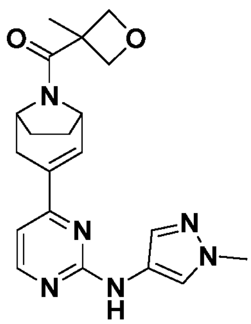 |
| | (3-(2-((1-Methyl-1*H*-pyrazol-4-yl)amino)pyrimidin-4-yl)-8-azabicyclo [3.2.1]oct-2-en-8-yl)(3-methyloxetan-3-yl)methanone |

(continued)

| Example No. | Structure and name |
|---|---|
| 14 | 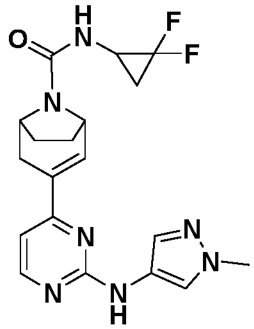 |
| | *N*-(2,2-difluorocyclopropyl)-3-(2-((1-methyl-1*H*-pyrazol-4-yl)amino) pyrimidin-4-yl)-8-azabicyclo [3.2.1]oct-2-ene-8-carboxamide |
| 15 | 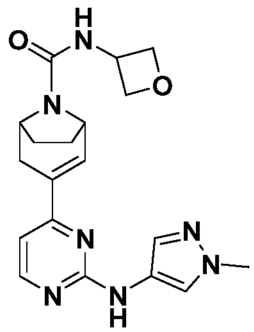 |
| | 3-(2-((1-Methyl-1*H*-pyrazol-4-yl)amino)pyrimidin-4-yl)-*N*-(oxetan-3-yl)-8-azabicyclo[3.2.1]oct-2-ene-8-carboxamide |
| 16 | 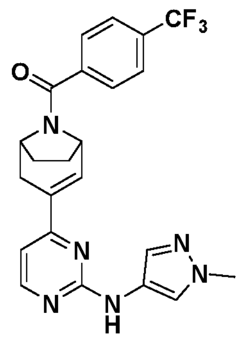 |
| | (3-(2-((1-Methyl-1*H*-pyrazol-4-yl)amino)pyrimidin-4-yl)-8-azabicyclo [3.2.1]oct-2-en-8-yl) (4-(trifluoromethyl)phenyl)methanone |
| 17 | 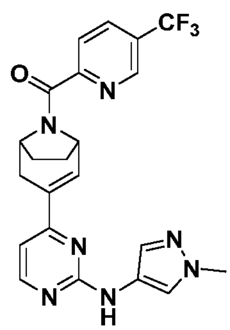 |
| | (3-(2-((1-Methyl-1*H*-pyrazol-4-yl)amino)pyrimidin-4-yl)-8-azabicyclo [3.2.1]oct-2-en-8-yl) (5-(trifluoromethyl)pyridin-2-yl)methanone |

(continued)

| Example No. | Structure and name |
|---|---|
| 18 | 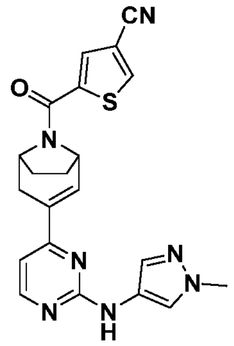 |
| | 5-(3-(2-((1-Methyl-1*H*-pyrazol-4-yl)amino)pyrimidin-4-yl)-8-azabicyclo [3.2.1]oct-2-ene-8-formyl) thiophene-3-carbonitrile |
| 19 | 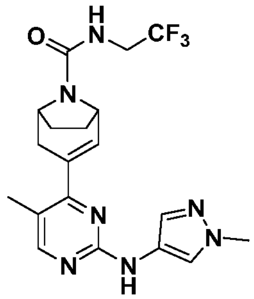 |
| | 3-(5-Methyl-2-((1-methyl-1*H*-pyrazol-4-yl)amino)pyrimidin-4-yl)-*N*-(2,2, 2-trifluoroethyl)-8-azabicyclo[3 .2.1]oct-2-ene-8-carboxamide |
| 19-a and 19-b | 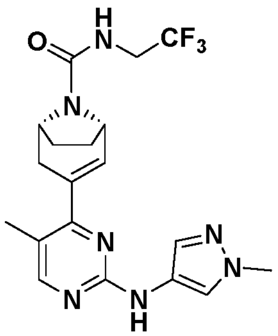 |
| | (*1S,5R*)-3-(5-methyl-2-((1-methyl-*1*H-pyrazol-4-yl)amino)pyrimidin-4-yl) -N-(2,2,2-trifluoroethyl)-8-azabicyclo[3.2.1]oct-2-ene-8-carboxamide |
| | 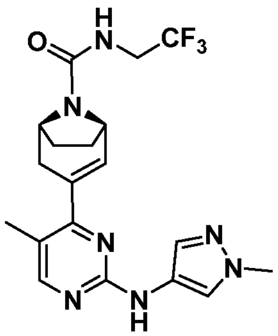 |
| | (*1R,5S*)-3-(5-methyl-2-((1-methyl-1*H*-pyrazol-4-yl)amino)pyrimidin-4-yl) -N-(2,2,2-trifluoroethyl)-8-azabicyclo[3.2.1]oct-2-ene-8-carboxamide |

(continued)

| Example No. | Structure and name |
|---|---|
| 20 | 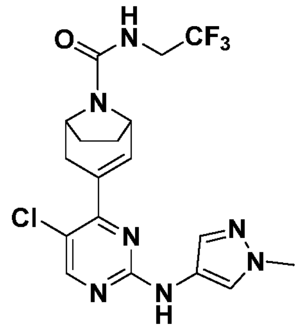 |
| | 3-(5-Chloro-2-((1-methyl-1*H*-pyrazol-4-yl)amino)pyrimidin-4-yl)-*N*-(2,2,2-trifluoroethyl)-8-azabicyclo[3.2.1]oct-2-ene-8-carboxamide |
| 21 | 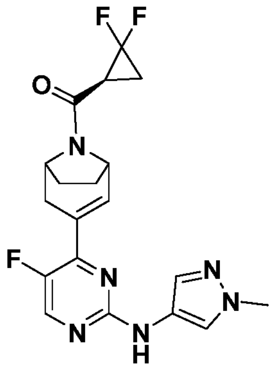 |
| | ((*S*)-2,2-difluorocyclopropyl)(3-(5-fluoro-2-((1-methyl-1*H*-pyrazol-4-yl) amino)pyrimidin-4-yl)-8-azabicyclo[3.2.1]oct-2-en-8-yl)methanone |
| 22 | 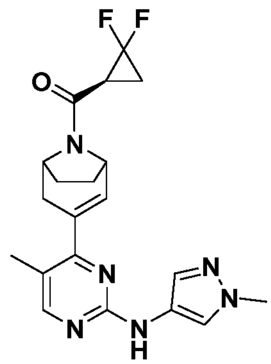 |
| | ((*S*)-2,2-difluorocyclopropyl)(3-(5-methyl-2-((1-methyl-1*H*-pyrazol-4-yl) amino)pyrimidin-4-yl)-8-azabicyclo[3.2.1]oct-2-en-8-yl)methanone |

(continued)

| Example No. | Structure and name |
|---|---|
| 22-a and 22-b | 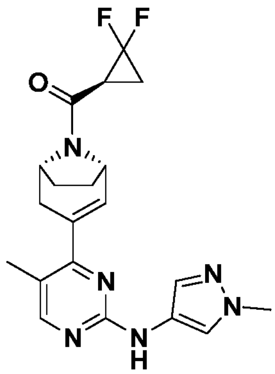 |
| | ((*S*)-2,2-difluorocyclopropyl)((*1S*,*5R*)-3-(5-methyl-2-((1-methyl-1*H*-pyrazol-4-yl)amino)pyrimidin-4-yl)-8-azabicyclo[3.2.1]oct-2-en-8-yl) methanone |
| | 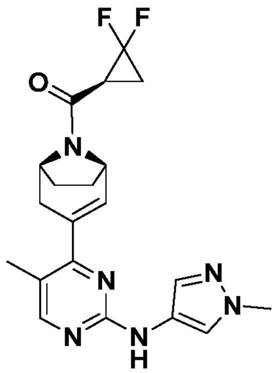 |
| | ((*S*)-2,2-difluorocyclopropyl)((*1R*,*5S*)-3-(5-methyl-2-((1-methyl-1*H*-pyrazol-4-yl)amino)pyrimidin-4-yl)-8-azabicyclo[3.2.1]oct-2-en-8-yl) methanone |
| 23 | 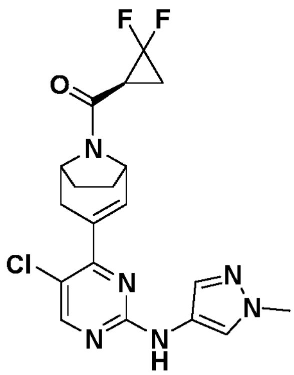 |
| | (3-(5-Chloro-2-((1-methyl-1*H*-pyrazol-4-yl)amino)pyrimidin-4-yl)-8-azabicyclo[3.2.1]oct-2-en-8-yl)((*S*)-2,2-difluorocyclopropyl)methanone |

(continued)

| Example No. | Structure and name |
|---|---|
| 23-a and 23-b | 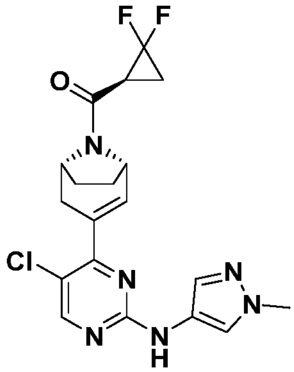 |
| | ((*1S,5R*)-3-(5-chloro-2-((1-methyl-1*H*-pyrazol-4-yl)amino)pyrimidin-4-yl)-8-azabicyclo[3.2.1]oct-2-en-8-yl)((*S*)-2,2-difluorocyclopropyl) methanone |
| | 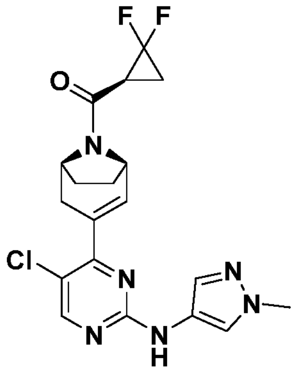 |
| | ((1R,5S)-3-(5-chloro-2-((1-methyl-1H-pyrazol-4-yl)amino)pyrimidin-4-yl)-8-azabicyclo[3.2.1]oct-2-en-8-yl)((S)-2,2-difluorocyclopropyl) methanone |
| 24 | 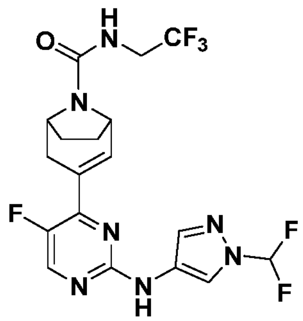 |
| | 3-(2-((1-Difluoromethyl)-1H-pyrazol-4-yl)amino)-5-fluoropyrimidin-4-yl)-N-(2,2,2-trifluoroethyl)-8-azabicyclo[3.2.1]oct-2-ene-8-carboxamide |

(continued)

| Example No. | Structure and name |
|---|---|
| 25 | 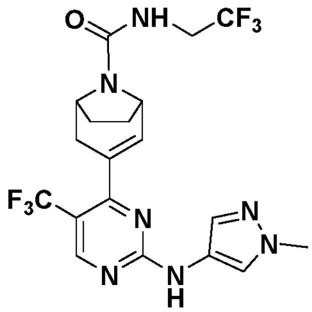 |
| | 3-(2-((1-Methyl-1H-pyrazol-4-yl)amino)-5-(trifluoromethyl)pyrimidin-4-yl)-N-(2,2,2-trifluoroethyl)-8-azabicyclo[3.2.1]oct-2-ene-8-carboxamide |
| 26 | 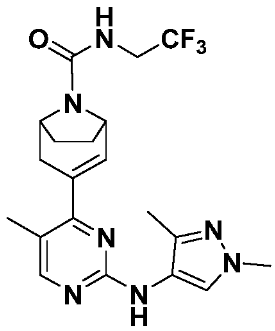 |
| | 3-(2-((1,3-Dimethyl-1H-pyrazol-4-yl)amino)-5-methylpyrimidin-4-yl)-N-(2,2,2-trifluoroethyl)-8-azabicyclo[3.2.1]oct-2-ene-8-carboxamide |
| 26-a and 26-b | 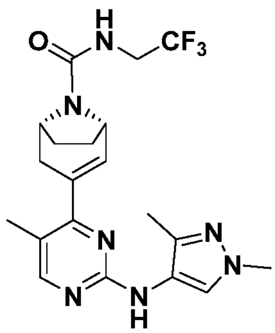 |
| | (1S,5R)-3-(2-((1,3-dimethyl-1H-pyrazol-4-yl)amino)-5-methylpyrimidin-4-yl)-N-(2,2,2-trifluoroethyl)-8-azabicyclo[3.2.1]oct-2-ene-8-carboxamide |
| | 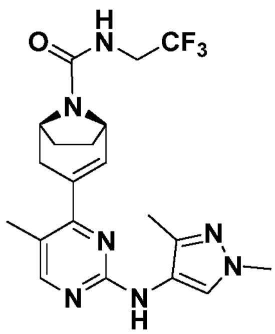 |
| | (1R,5S)-3-(2-((1,3-dimethyl-1H-pyrazol-4-yl)amino)-5-methylpyrimidin-4-yl)-N-(2,2,2-trifluoroethyl)-8-azabicyclo[3.2.1]oct-2-ene-8-carboxamide |

(continued)

| Example No. | Structure and name |
|---|---|
| 27 | 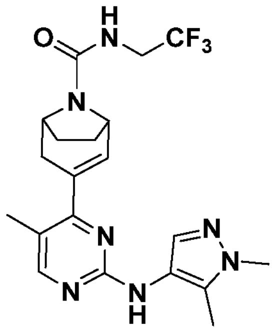 |
| | 3-(2-((1,5-Dimethyl-1H-pyrazol-4-yl)amino)-5-methylpyrimidin-4-yl)-N-(2,2,2-trifluoroethyl)-8-azabicyclo[3.2.1]oct-2-ene-8-carboxamide |
| 28 | 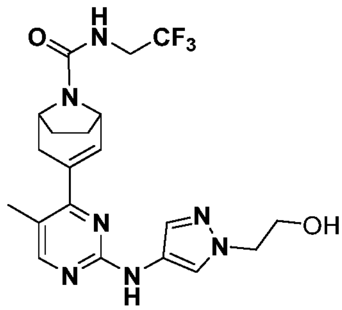 |
| | 3-(2-((1-(2-Hydroxyethyl)-1H-pyrazol-4-yl)amino)-5-methylpyrimidin-4-yl)-N-(2,2,2-trifluoroethyl)-8-azabicyclo[3.2.1]oct-2-ene-8-carboxamide |
| 29 | 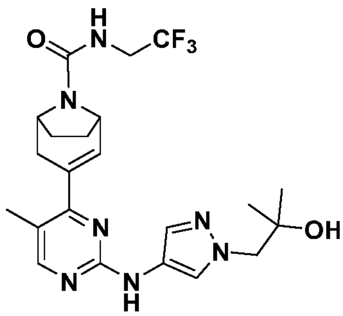 |
| | 3-(2-((1-(2-Hydroxy-2-methylpropyl)-1H-pyrazol-4-yl)amino)-5-methyl pyrimidin-4-yl)-N-(2,2,2-trifluoroethyl)-8-azabicyclo[3.2.1]oct-2-ene-8-carboxamide |
| 29-a and 29-b | 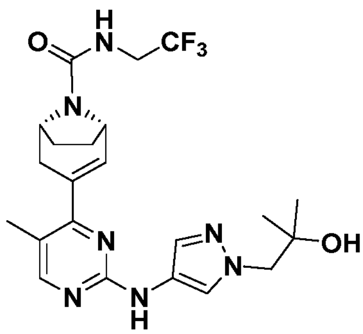 |

(continued)

| Example No. | Structure and name |
|---|---|
| | (1S,5R)-3-(2-((1-(2-Hydroxy-2-methylpropyl)-1H-pyrazol-4-yl)amino)-5-methylpyrimidin-4-yl)-N-(2,2,2-trifluoroethyl)-8-azabicyclo[3.2.1]oct-2-ene-8-carboxamide |
| | 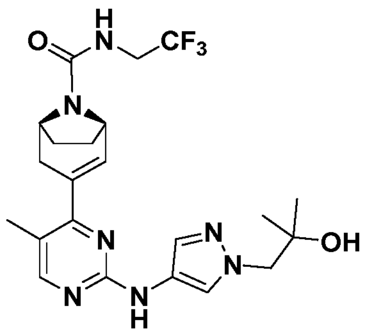 |
| | (1R,5S)-3-(2-((1-(2-Hydroxy-2-methylpropyl)-1H-pyrazol-4-yl)amino)-5-methylpyrimidin-4-yl)-N-(2,2,2-trifluoroethyl)-8-azabicyclo[3.2.1]oct-2-ene-8-carboxamide |
| 30 | 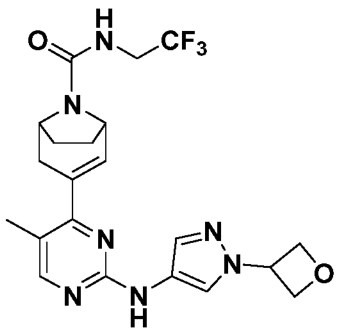 |
| | 3-(5-Methyl-2-((1-(oxetan-3-yl)-1H-pyrazol-4-yl)amino)pyrimidin-4-yl)-N-(2,2,2-trifluoroethyl)-8-azabicyclo[3.2.1]oct-2-ene-8-carboxamide |
| 31 | 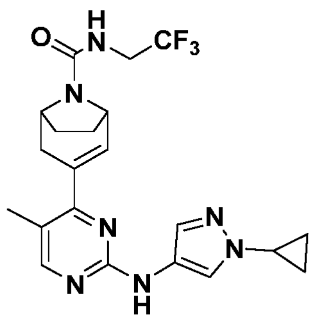 |
| | 3-(2-((1-Cyclopropyl-1H-pyrazol-4-yl)amino)-5-methylpyrimidin-4-yl)-N -(2,2,2-trifluoroethyl)-8-azabicyclo[3.2.1]oct-2-ene-8-carboxamide |
| 32 | 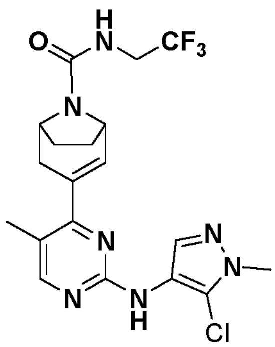 |

(continued)

| Example No. | Structure and name |
|---|---|
| | 3-(2-((5-Chloro-1-methyl-1H-pyrazol-4-yl)amino)-5-methylpyrimidin-4-yl)-N-(2,2,2-trifluoroethyl)-8-azabicyclo[3.2.1]oct-2-ene-8-carboxamide |
| 33 | 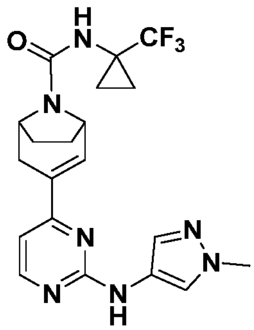 |
| | 3-(2-((1-Methyl-1H-pyrazol-4-yl)amino)pyrimidin-4-yl)-N-(1-(trifluoro methyl)cyclopropyl)-8-azabicyclo[3.2.1]oct-2-ene-8-carboxamide |
| 34 | 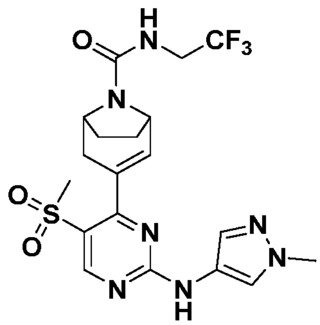 |
| | 3-(2-((1-Methyl-1H-pyrazol-4-yl)amino)-5-(methylsulfonyl)pyrimidin-4-yl)-N-(2,2,2-trifluoroethyl)-8-azabicyclo[3.2.1]oct-2-ene-8-carboxamide |
| 35 | 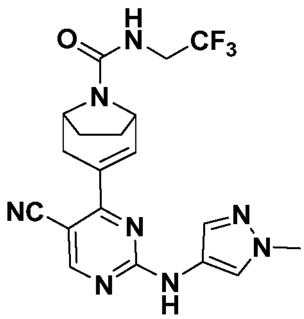 |
| | 3-(5-Cyano-2-((1-methyl-1H-pyrazol-4-yl)amino)pyrimidin-4-yl)-N-(2,2, 2-trifluoroethyl)-8-azabicyclo[3 .2.1]oct-2-ene-8-carboxamide |

(continued)

| Example No. | Structure and name |
| --- | --- |
| 36 | 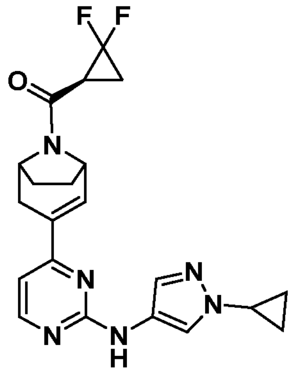 |
| | (3-(2-((1-Cyclopropyl-1H-pyrazol-4-yl)amino)pyrimidin-4-yl)-8-aza bicyclo[3.2.1]oct-2-en-8-yl)((S)-2,2-difluorocyclopropyl)methanone |
| 37 | 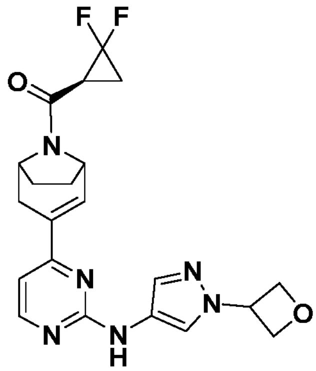 |
| | ((S)-2,2-Difluorocyclopropyl)(3-(2-((1-(oxetan-3-yl)-1H-pyrazol-4-yl) amino)pyrimidin-4-yl)-8-azabicyclo[3.2.1]oct-2-en-8-yl)methanone |
| 38 | 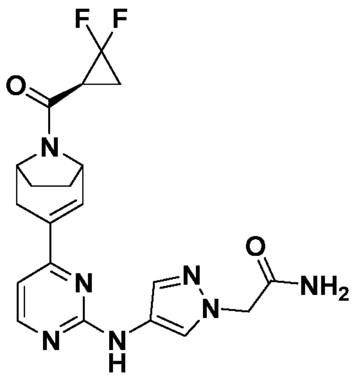 |
| | 2-(4-((4-(8-((S)-2,2-Difluorocyclopropane-1-formyl)-8-azabicyclo[3.2.1] oct-2-en-3-yl)pyrimidin-2-yl)amino)-1H-pyrazol-1-yl)acetamide |

(continued)

| Example No. | Structure and name |
|---|---|
| 39 | 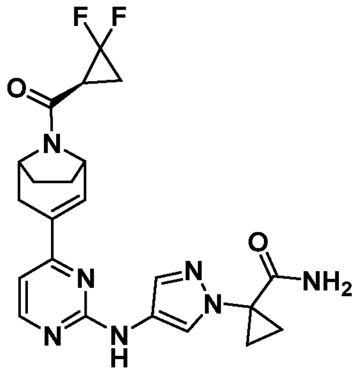 |
| | 1-(4-((4-(8-((S)-2,2-Difluorocyclopropane-1-carbonyl)-8-azabicyclo[3.2.1 ]oct-2-en-3-yl) pyrimidin-2-yl)amino)-1H-pyrazol-1-yl)cyclopropane-1-carboxamide |
| 40 | 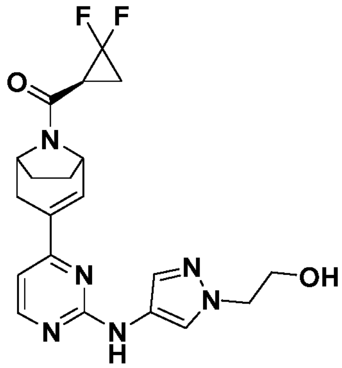 |
| | ((S)-2,2-Difluorocyclopropyl)(3-(2-((1-(2-hydroxyethyl)-1H-pyrazol-4-yl )amino)pyrimidin-4-yl)-8-azabicyclo[3.2.1]oct-2-en-8-yl)methanone |
| 41 | 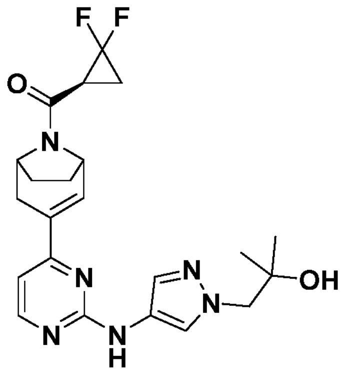 |
| | ((S)-2,2-Difluorocyclopropyl)(3-(2-((1-(2-hydroxy-2-methylpropyl)-1H-pyrazol-4-yl)amino) pyrimidin-4-yl)-8-azabicyclo[3.2.1]oct-2-en-8-yl) methanone |

(continued)

| Example No. | Structure and name |
|---|---|
| 42 | 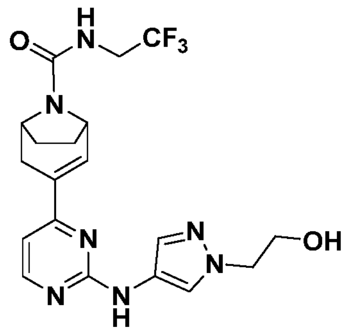 |
| | 3-(2-((1-(2-Hydroxyethyl)-1H-pyrazol-4-yl)amino)pyrimidin-4-yl)-N-(2,2,2-trifluoroethyl)-8-azabicyclo[3.2.1]oct-2-ene-8-carboxamide |
| 43 | 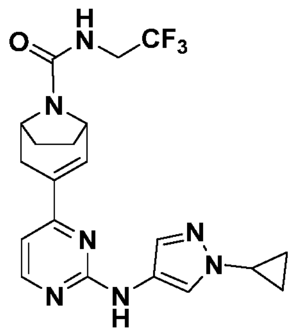 |
| | 3-(2-((1-Cyclopropyl-1H-pyrazol-4-yl)amino)pyrimidin-4-yl)-N-(2,2,2-trifluoroethyl)-8-azabicyclo[3.2.1]oct-2-ene-8-carboxamide |
| 44 | 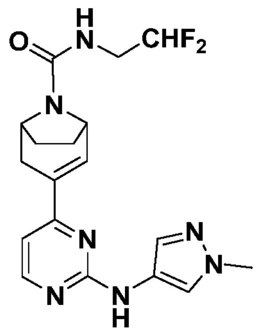 |
| | N-(2,2-Difluoroethyl)-3-(2-((1-methyl-1H-pyrazol-4-yl)amino)pyrimidin-4-yl)-8-azabicyclo[3.2.1]oct-2-ene-8-carboxamide |

(continued)

| Example No. | Structure and name |
| --- | --- |
| 45 | 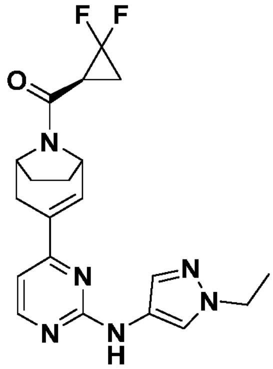 |
| | ((S)-2,2-Difluorocyclopropyl)(3-(2-((1-ethyl-1H-pyrazol-4-yl)amino) pyrimidin-4-yl)-8-azabicyclo [3.2.1]oct-2-en-8-yl)methanone |
| 46 | 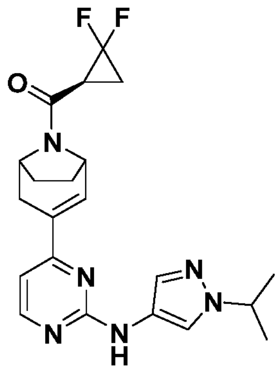 |
| | ((S)-2,2-Difluorocyclopropyl)(3-(2-((1-isopropyl-1H-pyrazol-4-yl)amino) pyrimidin-4-yl)-8-azabicyclo[3.2.1]oct-2-en-8-yl)methanone |
| 47 | 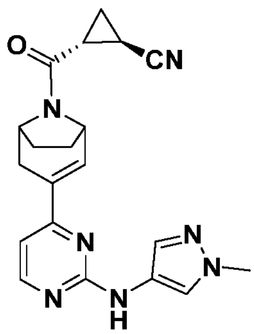 |
| | (1R,2R)-2-(3-(2-((1-Methyl-1H-pyrazol-4-yl)amino)pyrimidin-4-yl)-8-az abicyclo[3.2.1]oct-2-ene-8-formyl)cyclopropane-1-carbonitrile |
| 48 | 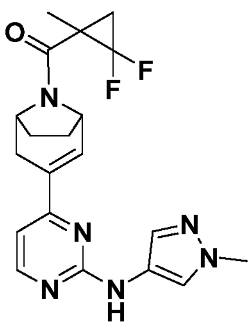 |

(continued)

| Example No. | Structure and name |
|---|---|
| | (2,2-Difluoro-1-methylcyclopropyl)(3-(2-((1-methyl-1H-pyrazol-4-yl) amino)pyrimidin-4-yl)-8-azabicyclo[3.2.1]oct-2-en-8-yl)methanone |
| 49 | 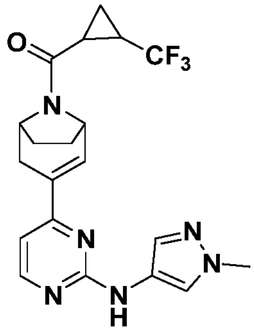 |
| | (3-(2-((1-Methyl-1H-pyrazol-4-yl)amino)pyrimidin-4-yl)-8-azabicyclo [3.2.1]oct-2-en-8-yl)(2-(trifluoromethyl)cyclopropyl)methanone |
| 50 | 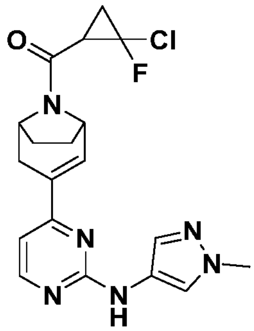 |
| | (2-Chloro-2-fluorocyclopropyl)(3-(2-((1-methyl-1H-pyrazol-4-yl)amino) pyrimidin-4-yl)-8-azabicyclo[3.2.1]oct-2-en-8-yl)methanone |
| 51 | 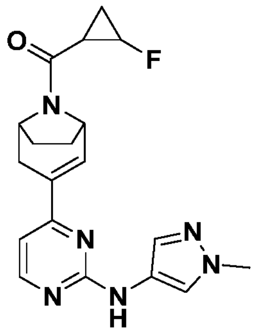 |
| | (2-Fluorocyclopropyl)(3-(2-((1-methyl-1H-pyrazol-4-yl)amino)pyrimidin -4-yl)-8-azabicyclo[3.2.1]oct-2-en-8-yl)methanone |

(continued)

| Example No. | Structure and name |
|---|---|
| 52 | 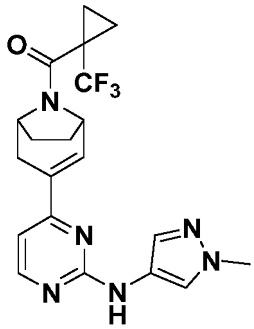 |
| | (3-(2-((1-Methyl- 1H-pyrazol-4-yl)amino)pyrimidin-4-yl)-8-azabicyclo [3.2.1]oct-2-en-8-yl)(1-(trifluoromethyl)cyclopropyl)methanone |
| 53 | 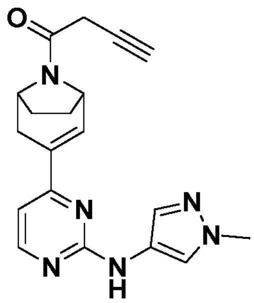 |
| | 1-(3-(2-((1-Methyl-1H-pyrazol-4-yl)amino)pyrimidin-4-yl)-8-azabicyclo[ 3.2.1]oct-2-en-8-yl)but-3-yn-1-one |
| 54 | 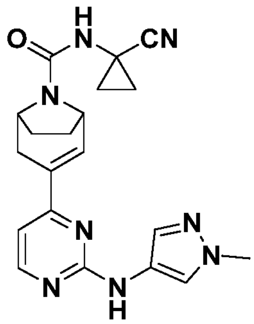 |
| | N-(1-Cyanocyclopropyl)-3-(2-((1-methyl-1H-pyrazol-4-yl)amino) pyrimidin-4-yl)-8-azabicyclo [3.2.1]oct-2-ene-8-carboxamide |
| 55 | 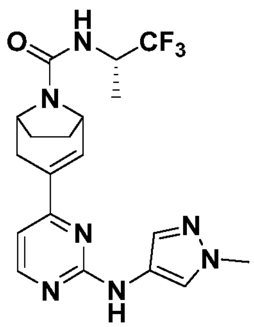 |
| | 3-(2-((1-Methyl-1H-pyrazol-4-yl)amino)pyrimidin-4-yl)-N-((S)-1,1,1-trifluoropropane-2-yl)-8-azabicyclo[3.2.1]oct-2-ene-8-carboxamide |

(continued)

| Example No. | Structure and name |
|---|---|
| 56 | 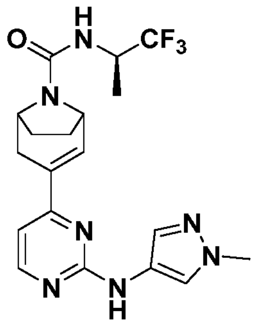 |
| | 3-(2-((1-Methyl-1H-pyrazol-4-yl)amino)pyrimidin-4-yl)-N-((R)-1,1,1-trifluoropropane-2-yl)-8-azabicyclo[3.2.1]oct-2-ene-8-carboxamide |
| 57 | 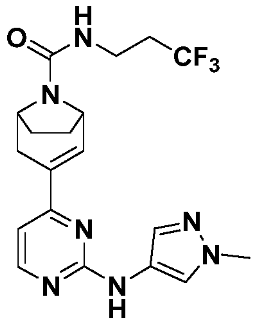 |
| | 3-(2-((1-Methyl-1H-pyrazol-4-yl)amino)pyrimidin-4-yl)-N-(3,3,3-trifluor opropyl)-8-azabicyclo [3.2.1]oct-2-ene-8-carboxamide |
| 58 | 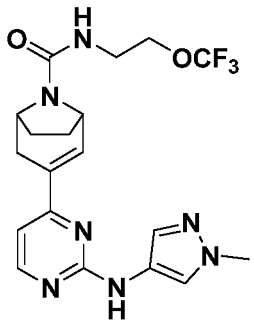 |
| | 3-(2-((1-Methyl-1H-pyrazol-4-yl)amino)pyrimidin-4-yl)-N-(2-(trifluorom ethoxy)ethyl)-8-azabicyclo[3.2.1]oct-2-ene-8-carboxamide |
| 59 | 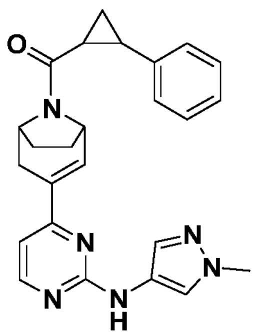 |
| | 3-(2-((1-Methyl-1H-pyrazol-4-yl)amino)pyrimidin-4-yl)-8-azabicyclo [3.2.1]oct-2-en-8-yl)(2-phenylcyclopropyl)methanone |

(continued)

| Example No. | Structure and name |
|---|---|
| 60 | 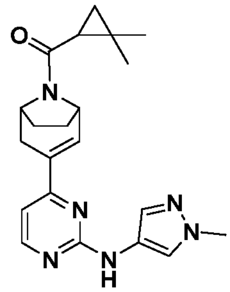 |
| | (2,2-Dimethylcyclopropyl)(3-(2-((1-methyl-1H-pyrazol-4-yl)amino) pyrimidin-4-yl)-8-azabicyclo [3.2.1]oct-2-en-8-yl)methanone |
| 61 | 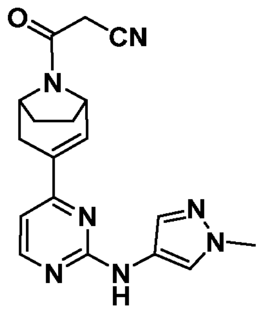 |
| | 3-(3-(2-((1-Methyl-1H-pyrazol-4-yl)amino)pyrimidin-4-yl)-8-azabicyclo [3.2.1]oct-2-en-8-yl)-3-oxopropionitrile |
| 62 | 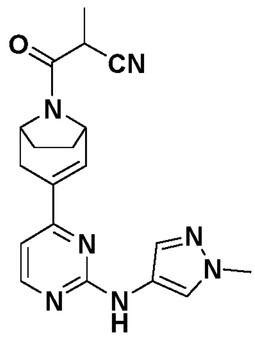 |
| | 2-Methyl-3-(3-(2-((1-Methyl-1H-pyrazol-4-yl)amino)pyrimidin-4-yl)-8-azabicyclo[3.2.1]oct-2-en-8-yl)-3-oxopropionitrile |
| 63 | 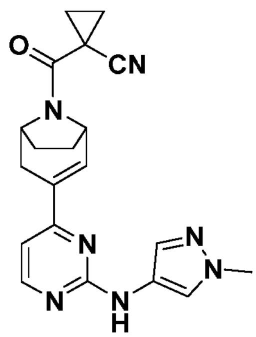 |
| | 1-(3-(2-((1-Methyl-1H-pyrazol-4-yl)amino)pyrimidin-4-yl)-8-azabicyclo [3.2.1]oct-2-ene-8-formyl) cyclopropane-1-carbonitrile |

(continued)

| Example No. | Structure and name |
|---|---|
| 64 | 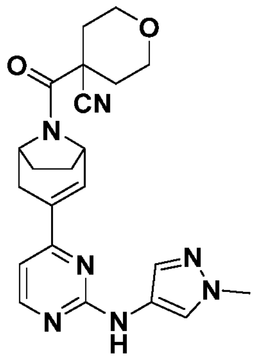 |
| | 4-(3-(2-((1-Methyl-1H-pyrazol-4-yl)amino)pyrimidin-4-yl)-8-azabicyclo [3.2.1]oct-2-ene-8-carbonyl)tetrahydro-2H-pyran-4-cyano |
| 65 | 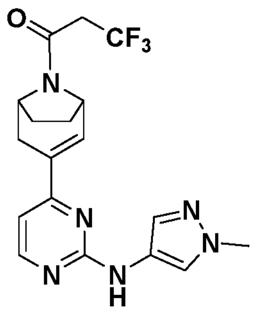 |
| | 3,3,3-Trifluoro-1-(3-(2-((1-methyl-1H-pyrazol-4-yl)amino)pyrimidin-4-yl)-8-azabicyclo[3.2.1]oct-2-en-8-yl)-1-acetone |
| 66 | 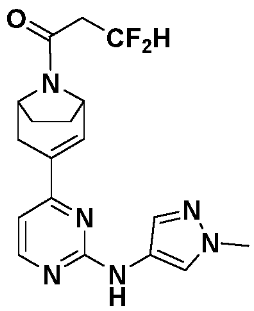 |
| | 3,3-Difluoro-1-(3-(2-((1-methyl-1H-pyrazol-4-yl)amino)pyrimidin-4-yl)-8-azabicyclo[3.2.1]oct-2-en-8-yl)-1-acetone |
| 67 | 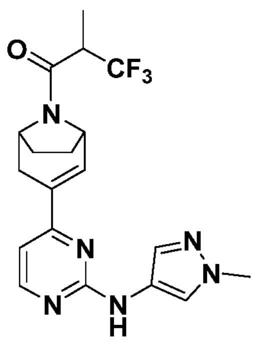 |
| | 3,3,3-Trifluoro-2-methyl-1-(3-(2-((1-methyl-1H-pyrazol-4-yl)amino)pyri midin-4-yl)-8-azabicyclo [3.2.1]oct-2-en-8-yl)-1-acetone |

(continued)

| Example No. | Structure and name |
|---|---|
| 68 | 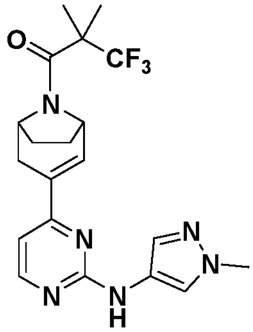 |
| | 3,3,3-Trifluoro-2,2-dimethyl-1-(3-(2-((1-methyl-1H-pyrazol-4-yl)amino) pyrimidin-4-yl)-8-azabicyclo[3.2.1]oct-2-en-8-yl)acetone |
| 69 | 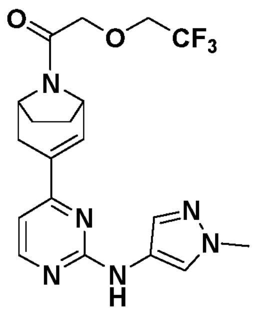 |
| | 1-(3-(2-((1-Methyl-1H-pyrazol-4-yl)amino)pyrimidin-4-yl)-8-azabicyclo [3.2.1]oct-2-en-8-yl)-2-(2,2,2-trifluoroethoxy)-1-one |
| 70 | 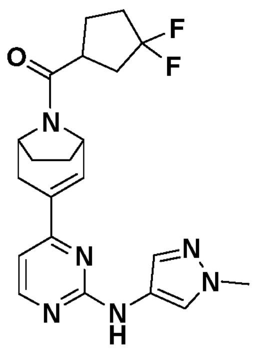 |
| | (3,3-Difluorocyclopentyl)(3-(2-((1-methyl-1H-pyrazol-4-yl)amino) pyrimidin-4-yl)-8-azabicyclo [3.2.1]oct-2-en-8-yl)methanone |

(continued)

| Example No. | Structure and name |
|---|---|
| 71 | 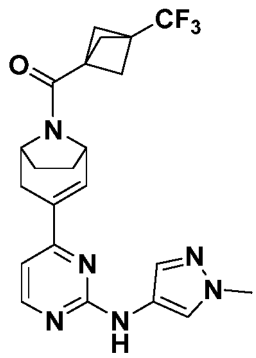 |
| | (3-(2-((1-Methyl- 1H-pyrazol-4-yl)amino)pyrimidin-4-yl)-8-azabicyclo [3.2.1]oct-2-en-8-yl) (3-(trifluoromethyl)bicyclo[1.1.1]pent-1-yl) methanone |
| 72 | 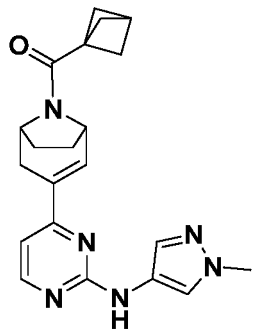 |
| | Bicyclo[1.1.1]pent-1-yl(3-(2-((1-methyl-1H-pyrazol-4-yl)amino) pyrimidin-4-yl)-8-azabicyclo [3.2.1]oct-2-en-8-yl)methanone |
| 73 | 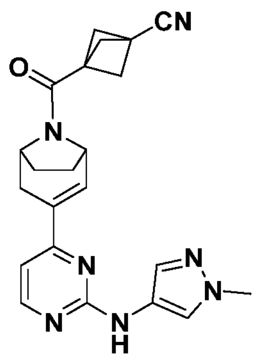 |
| | 3-(3-(2-((1-Methyl-1H-pyrazol-4-yl)amino)pyrimidin-4-yl)-8-azabicyclo [3.2.1]oct-2-ene-8-formyl) bicyclo[1.1.1]pentane-1-carbonitrile |

(continued)

| Example No. | Structure and name |
|---|---|
| 74 | 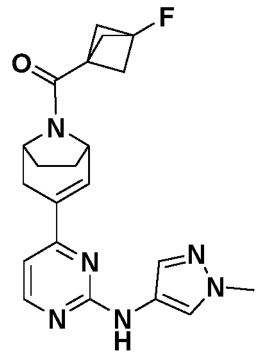 |
| | (3-Fluorobicyclo[1.1.1]pent-1-yl)(3-(2-((1-methyl-1H-pyrazol-4-yl) amino)pyrimidin-4-yl)-8-azabicyclo[3.2.1]oct-2-en-8-yl)methanone |
| 75 | 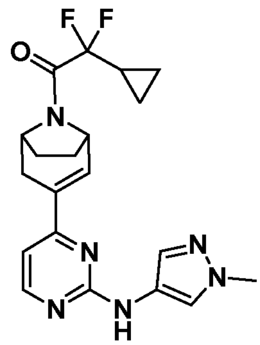 |
| | 2-Cyclopropyl-2,2-difluoro-1-(3-(2-((1-methyl-1H-pyrazol-4-yl)amino) pyrimidin-4-yl)-8-azabicyclo[3.2.1]oct-2-en-8-yl)ethan-1-one |
| 76 | 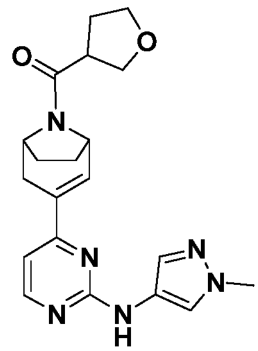 |
| | (3-(2-((1-Methyl- 1H-pyrazol-4-yl)amino)pyrimidin-4-yl)-8-azabicyclo [3.2.1]oct-2-en-8-yl) (tetrahydrofuran-3-yl)methanone |

(continued)

| Example No. | Structure and name |
|---|---|
| 77 | 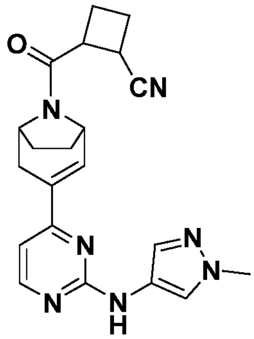 |
| | 2-(3-(2-((1-Methyl-1H-pyrazol-4-yl)amino)pyrimidin-4-yl)-8-azabicyclo [3.2.1]oct-2-ene-8-formyl) cyclobutane-1-carbonitrile |
| 78 | 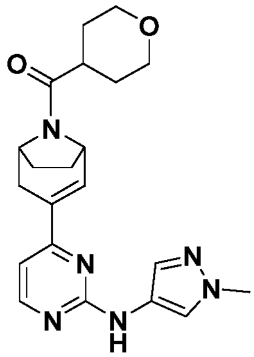 |
| | 3-(2-((1-Methyl-1H-pyrazol-4-yl)amino)pyrimidin-4-yl)-8-azabicyclo [3.2.1]oct-2-en-8-yl) (tetrahydro-2H-pyran-4-yl)methanone |
| 79 | 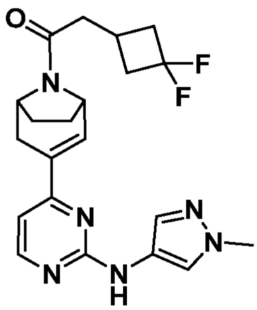 |
| | 2-(3,3-Difluorocyclobutyl)-1-(3-(2-((1-methyl-1H-pyrazol-4-yl)amino) pyrimidin-4-yl)-8-azabicyclo [3.2.1]oct-2-en-8-yl)-1-acetone |

(continued)

| Example No. | Structure and name |
|---|---|
| 80 | 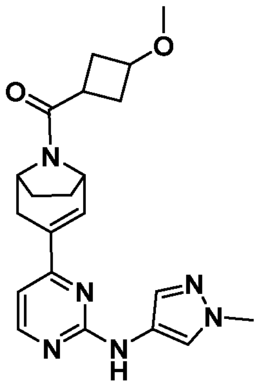 |
| | (3-Methoxycyclopropyl)(3-(2-((1-methyl-1H-pyrazol-4-yl)amino) pyrimidin-4-yl)-8-azabicyclo [3.2.1]oct-2-en-8-yl)methanone |
| 81 | 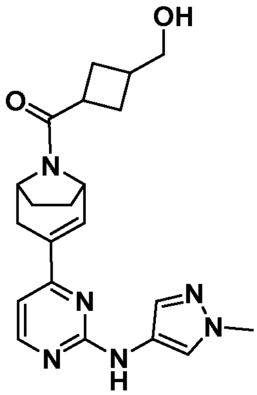 |
| | (3-(Hydroxymethyl)cyclobutyl)(3-(2-((1-methyl-1H-pyrazol-4-yl)amino) pyrimidin-4-yl)-8-azabicyclo[3.2.1]oct-2-en-8-yl)methanone |
| 82 | 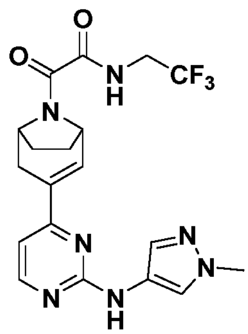 |
| | 2-(3-(2-((1-Methyl-1H-pyrazol-4-yl)amino)pyrimidin-4-yl)-8-azabicyclo [3.2.1]oct-2-en-8-yl)--2-oxo-N-(2,2,2-trifluoroethyl)acetamide |

(continued)

| Example No. | Structure and name |
|---|---|
| 83 | 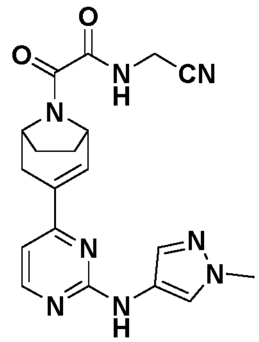 |
| | N-(Cyanomethyl)-2-(3-(2-((1-methyl-1H-pyrazol-4-yl)amino)pyrimidin-4 -yl)-8-azabicyclo[3.2.1] oct-2-en-8-yl)-2-oxoacetamide |
| 84 | 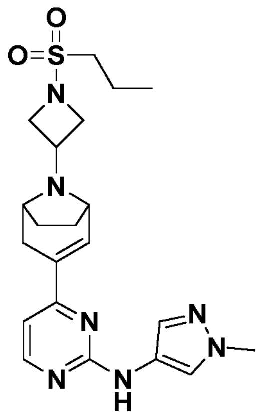 |
| | N-(1-Methyl-1H-pyrazol-4-yl)-4-(8-(1-(propansulfonyl)azetidin-3-yl)-8-azabicyclo[3.2.1]oct-2-en-3-yl)pyrimidin-2-amine |
| 85 | 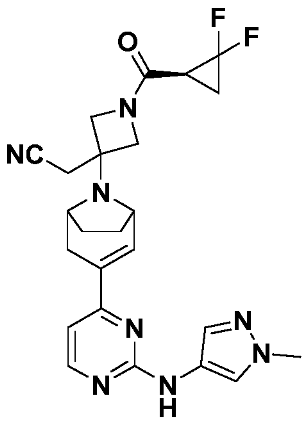 |
| | 2-(1-((S)-2,2-Difluorocyclopropane-1-formyl)-3-(3-(2-((1-methyl-1H-pyrazol-4-yl)amino) pyrimidin-4-yl)-8-azabicyclo[3.2.1]oct-2-en-8-yl) azetidin-3-yl)acetonitrile |

(continued)

| Example No. | Structure and name |
|---|---|
| 86 | 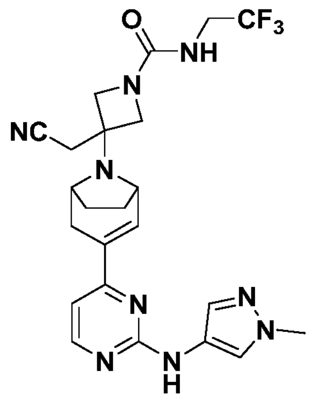 |
| | 3-(Cyanomethyl)-3-(3-(2-((1-methyl-1H-pyrazol-4-yl)amino)pyrimidin-4-yl)-8-azabicyclo[3.2.1]oct-2-en-8-yl)-N-(2,2,2-trifluoroethyl)azetidine-1-carboxamide |
| 87 | 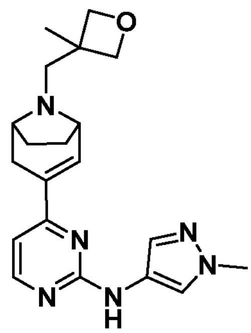 |
| | N-(1-methyl-1H-pyrazol-4-yl)-4-(8-((3-methyloxetan-3-yl)methyl)-8-azabicyclo[3.2.1]oct-2-en-3-yl)pyrimidin-2-amine |

or a mesomer, racemate, enantiomer, diastereomer thereof, or a mixture thereof, or a prodrug thereof, or a pharmaceutically acceptable salt thereof.

The present invention further provides a method for preparing the compound represented by general formula (I), or a mesomer, racemate, enantiomer, diastereomer thereof, or a mixture thereof, or a prodrug thereof, or a pharmaceutically acceptable salt thereof according to the present invention, which includes the following steps:

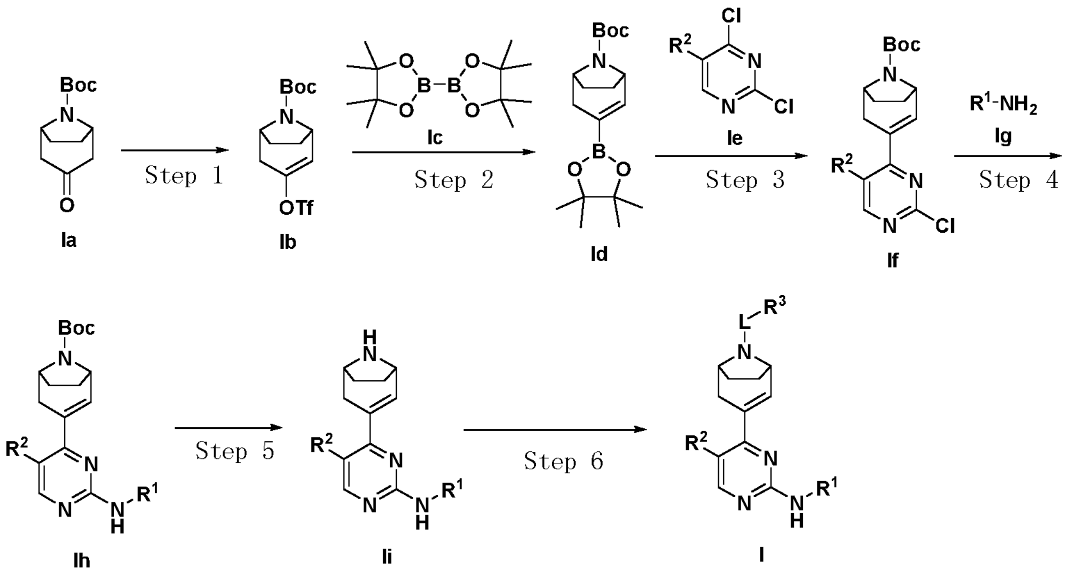


Step 1: Reacting compound Ia with *N*-phenylbis(trifluoromethanesulfonyl)imide under alkaline conditions to obtain compound Ib, wherein the reagent providing alkaline conditions is preferably potassium hexamethyldisilazide;
Step 2: Reacting compound Ib with pinacol diborate (Ic) under alkaline conditions and in the presence of a catalyst to obtain compound Id, wherein the reagent providing alkaline conditions is preferably potassium acetate, and the catalyst is preferably Pd(dppf)$Cl_2$-$CH_2Cl_2$;
Step 3: Reacting compound Id with compound Ie under alkaline conditions in the presence of a catalyst to obtain compound If, wherein the reagent providing alkaline conditions is preferably potassium carbonate, and the catalyst is preferably Pd(dppf)$Cl_2$;
Step 4: Reacting compound If with compound Ig under acidic conditions to obtain compound Ih, wherein the reagent providing acidic conditions is preferably p-toluenesulfonic acid;
Step 5: Compound Ih is subjected to a deprotection reaction under acidic conditions to obtain compound Ii, wherein the reagent providing acidic conditions is preferably trifluoroacetic acid;
Step 6: reacting compound Ii with $R^3$-L-X (X=Cl, Br, I, OPh or

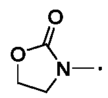

) under alkaline conditions to obtain a compound of general formula (I), wherein the reagent providing alkaline conditions is preferably triethylamine; or reacting compound Ii with $R^3$-L-OH under alkaline conditions in the presence of a catalyst to obtain a compound of general formula (I), wherein the reagent providing alkaline conditions is preferably DIPEA, and the catalyst is preferably HATU,
wherein, $R^1$, $R^2$, $R^3$ and L are as defined in the compound of general formula (I).

The present invention further provides a pharmaceutical composition comprising a therapeutically effective amount of the compound represented by general formula (I), or a mesomer, racemate, enantiomer, diastereomer thereof, or a mixture thereof, or a prodrug thereof, or a pharmaceutically acceptable salt thereof according to the present invention, as well as a pharmaceutically acceptable carrier.
The present invention further relates to a use of the compound represented by general formula (I), or a mesomer, racemate, enantiomer, diastereomer thereof, or a mixture thereof, or a prodrug thereof, or a pharmaceutically acceptable salt thereof, or the pharmaceutical composition containing the same according to the present invention in the preparation of JAK1 and TYK2 inhibitors.
The present invention further relates to a use of the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention in the preparation of medicaments for the prevention and/or treatment of diseases related to JAK1 and TYK2 activity, wherein the disease is selected from the group consisting of inflammation, autoimmune disease and cancer, and the inflammation is preferably

selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

The present invention further relates to the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention, for use as a drug.

The present invention further relates to the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention, for use as a JAK1 and TYK2 inhibitor.

The present invention further relates to the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention, for use in the prevention and/or treatment of related to JAK1 and TYK2 activity, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

The present invention further relates to a method for inhibiting JAK1 and TYK2, comprising contacting the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention with JAK1 and TYK2.

The present invention further relates to a method for preventing and/or treating diseases related to JAK1 and TYK2 activity, comprising administering a therapeutically effective amount of the compound represented by general formula (I), or a mesomer, racemate, enantiomer, diastereomer thereof, or a mixture thereof, or a prodrug thereof, or a pharmaceutically acceptable salt thereof, or a pharmaceutical composition containing the same according to the present invention to a subject in need thereof, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis, the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

According to the conventional methods in the field of the present invention, the compound represented by general formula (I) of the present invention can be formed in a pharmaceutically acceptable acid addition salt with an acid. The acid includes inorganic acids and organic acids, particularly preferably hydrochloric acid, hydrobromic acid, sulfuric acid, phosphoric acid, methanesulfonic acid, ethanesulfonic acid, p-toluenesulfonic acid, benzenesulfonic acid, naphthalene disulfonic acid, acetic acid, propionic acid, lactic acid, trifluoroacetic acid, maleic acid, citric acid, fumaric acid, oxalic acid, tartaric acid, benzoic acid and the like.

According to conventional methods in the field of the present invention, the compound represented by general formula (I) of the present invention can be formed in a pharmaceutically acceptable basic addition salt with a base. The base includes inorganic bases and organic bases. Acceptable organic bases include diethanolamine, ethanolamine, N-methylglucamine, triethanolamine, tromethamine and the like, and acceptable inorganic bases include aluminum hydroxide, calcium hydroxide, potassium hydroxide, sodium carbonate, sodium hydroxide and the like.

Moreover, the present invention also includes a prodrug of the compound represented by general formula (I) of the present invention. The prodrug according to the present invention is a derivative of the compound represented by general formula (I). They may have relatively weak or even no activity *per se,* but can be converted into the corresponding biologically active form under physiological conditions (for example, metabolism, solvolysis or other ways)

after administration.

The pharmaceutical composition comprising the active ingredient can be in a form suitable for oral administration, for example a tablet, troche, lozenge, aqueous or oily suspension, dispersible powder or granule, emulsion, hard or soft capsule, syrup or elixir. An oral composition can be prepared according to any method known in the art for preparing a pharmaceutical composition, and such compositions can also comprise one or more components selected from the group consisting of sweetener, flavoring agent, coloring agent and preservative, in order to provide a pleasing and palatable pharmaceutical preparation. The tablet contains the active ingredient in admixture with non-toxic pharmaceutically acceptable excipients suitable for the manufacture of tablets. These excipients can be inert excipients, such as calcium carbonate, sodium carbonate, lactose, calcium phosphate or sodium phosphate; granulating and disintegrating agents, for example microcrystalline cellulose, croscarmellose sodium, corn starch or alginic acid; binders, for example starch, gelatin, polyvinylpyrrolidone or arabic gum; and lubricants, for example magnesium stearate, stearic acid or talc. The tablet can be uncoated or coated by a known technique to mask the taste of the drug or delay the disintegration and absorption of the active ingredient in the gastrointestinal tract, thereby providing a sustained release over a long period of time. For example, water-soluble taste-masking substances such as hydroxypropyl methylcellulose or hydroxypropyl cellulose, or time-extending substances such as ethylcellulose or cellulose acetate butyrate can be used.

An oral formulation can also be provided as a hard gelatin capsules in which the active ingredient is mixed with an inert solid diluent such as calcium carbonate, calcium phosphate or kaolin, or a soft gelatin capsules in which the active ingredient is mixed with a water-soluble carrier such as polyethylene glycol, or an oil solvent such as peanut oil, liquid paraffin or olive oil.

An aqueous suspension comprises an active ingredient in admixture with excipients suitable for the manufacture of an aqueous suspension. Such excipients are suspending agents such as sodium carboxymethylcellulose, methylcellulose, hydroxypropylmethylcellulose, sodium alginate, polyvinylpyrrolidone and arabic gum; dispersing or wetting agents, which may be a naturally occurring phospholipid, such as lecithin, or a condensation product of alkylene oxide with fatty acid, such as polyoxyethylene stearate, or a condensation product of ethylene oxide with long chain fatty alcohol, such as heptadecylethyleneoxy cetanol, or a condensation product of ethylene oxide with partial ester derived from fatty acid and hexitol, such as polyethylene oxide sorbitol monooleate, or a condensation product of ethylene oxide with partial ester derived from fatty acid and hexitol anhydride, such as polyethylene oxide sorbitan monooleate. The aqueous suspension can also comprise one or more preservatives, such as ethylparaben or n-propylparaben, one or more coloring agents, one or more flavoring agents and one or more sweetening agents, such as sucrose, saccharin or aspartame.

An oil suspension can be formulated by suspending the active ingredient in a vegetable oil such as peanut oil, olive oil, sesame oil or coconut oil, or a mineral oil, such as liquid paraffin. The oil suspension can comprise a thickener, such as beeswax, hard paraffin or cetyl alcohol. The aforementioned sweeteners and flavoring agents can be added to provide a palatable preparation. The compositions can be kept by adding an antioxidant, such as butylated hydroxyanisole or alpha-tocopherol.

The dispersible powders or granules suitable for the preparation of an aqueous suspension can provide the active ingredient in admixture with the dispersants or wetting agents, suspending agent or one or more preservatives by adding water. Suitable dispersants or wetting agents and suspending agents are as described above. Additional excipients, such as sweeteners, flavoring agents and colorants can also be added. The compositions can be kept by adding an antioxidant, such as ascorbic acid.

The pharmaceutical composition of the present invention can also be in the form of an oil-in-water emulsion. The oil phase can be a vegetable oil, such as olive oil or peanut oil, or a mineral oil, such as liquid paraffin, or a mixture thereof. Suitable emulsifiers can be naturally occurring phospholipids, such as soy lecithin, and esters or partial esters derived from fatty acids and hexitol anhydrides, such as sorbitan monooleate, and condensation products of the partial ester and ethylene oxide, such as polyethylene oxide sorbitol monooleate. The emulsion can also comprise sweeteners, flavoring agents, preservatives and antioxidants. Acceptable sweeteners are for example syrups and elixirs prepared with glycerol, propylene glycol, sorbitol or sucrose. Such preparations can also comprise demulcents, preservatives, colorants and antioxidants.

The pharmaceutical composition of the present invention can also be in the form of a sterile injectable aqueous solution. Acceptable vehicles or solvents that can be used are water, Ringer's solution or isotonic sodium chloride solution. The sterile injectable preparation can be a sterile injectable oil-in-water microemulsion in which the active ingredient is dissolved in the oil phase. For example, the active ingredient is dissolved in a mixture of soybean oil and lecithin, which is then added to a mixture of water and glycerol to form a microemulsion. The injection solution or microemulsion can be introduced into the bloodstream of patients by local bolus injection. Alternatively, the solution and micro-emulsion are preferably administered in a manner that maintains a constant circulating concentration of the compound of the present invention. In order to maintain this constant concentration, a continuous intravenous delivery device can be used.

The pharmaceutical composition of the present invention can be in the form of a sterile injectable aqueous or

oil suspension for intramuscular and subcutaneous administration. Such a suspension can be formulated with suitable dispersants or wetting agents and suspending agents as described above according to known techniques. The sterile injectable formulation can also be a sterile injectable solution or suspension prepared in a non-toxic parenterally acceptable diluent or solvent, for example a solution prepared in 1,3-butanediol. In addition, a sterile fixed oil can be conveniently used as a solvent or suspension medium. For this purpose, any blended fixed oil including synthetic mono- or diglycerides can be used. In addition, fatty acids, for example oleic acid, can also used to prepare the injections.

The compound of the present invention can be administered in the form of a suppository for rectal administration. These pharmaceutical compositions can be prepared by mixing the drug with a suitable non-irritating excipient, which is solid at ordinary temperature but is liquid in the rectum, thereby melting in the rectum to release the drug. Such substances include cocoa butter, glycerin gelatin, hydrogenated vegetable oil, and a mixture of polyethylene glycol and fatty acid esters of polyethylene glycol of various molecular weights.

It is well known to those skilled in the art that the dosage of a drug depends on a variety of factors including, but not limited to the following factors: the activity of the specific compound, the age, weight, health condition, behavior and diet of the patient, administration time, administration route, excretion rate, drug combination and the like. Moreover, the optimal treatment, such as treatment mode, daily dose of the compound or the type of the pharmaceutically acceptable salt, can be verified according to the conventional therapeutic regimens.

The present invention can comprise a composition prepared with the compound represented by general formula (I), or a pharmaceutically acceptable salt, hydrate or solvate thereof as an active ingredient in admixture with a pharmaceutically acceptable carrier or excipient, which can be formulated into a clinically acceptable formulation. The derivatives of the present invention can be used in combination with other active ingredients, as long as they do not exert adverse effects, for example allergic reactions and the like. The compound of the present invention can be used as the only active ingredient, or can also be used in combination with other active ingredient for the treatment of diseases related to JAK1 and TYK2 activity. Combination therapy is achieved by administering each active ingredients simultaneously, separately or sequentially.

## DETAILED DESCRIPTION OF THE INVENTION

Unless otherwise stated, the terms used in the specification and claims have the following meanings.

The term "alkyl" refers to a saturated aliphatic hydrocarbon group, which is a straight or branched group comprising 1 to 20 carbon atoms, preferably an alkyl containing 1 to 12 carbon atoms, more preferably an alkyl containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-decyl, 2-methyl-2-ethylhexyl, 2-methyl-3-ethylhexyl, 2,2-diethylpentyl, n-decyl, 3,3-diethylhexyl, 2,2-diethylhexyl and various branched chain isomers thereof. The alkly is more preferably lower alkyl groups containing 1 to 6 carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl and the like. The alkyl can be substituted or unsubstituted. When substituted, the substituent group(s) can be substituted at any available connection point. The substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, oxo, carboxyl and carboxylate.

The term "alkenyl" refers to an alkyl as defined above consisting of at least two carbon atoms and at least one carbon-carbon double bond, for example vinyl, 1-propenyl, 2-propenyl, 1-, 2- or 3-butenyl and the like. The alkenyl can be substituted or unsubstituted. When substituted, the substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio and heterocyclylthio.

The term "alkynyl" refers to an alkyl as defined above consisting of at least two carbon atoms and at least one carbon-carbon triple bond, for example ethynyl, propynyl, butynyl and the like. The alkynyl can be substituted or unsubstituted. When substituted, the substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio and heterocyclylthio.

The term "cycloalkyl" refers to a saturated or partially unsaturated monocyclic or polycyclic hydrocarbon substituent. The cycloalkyl ring comprises 3 to 20 carbon atoms, preferably 3 to 12 carbon atoms, more preferably 3 to 6 carbon atoms. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptantrienyl, cyclooctyl and the like. Polycyclic cycloalkyl includes a cycloalkyl having a spiro ring, fused ring or bridged ring.

The term "spiro cycloalkyl" refers to a 5 to 20 membered polycyclic group with individual rings connected through one shared carbon atom (called a spiro atom), wherein the rings can contain one or more double bonds, but none of the rings has a fully conjugated π electron system. The spiro cycloalkyl is preferably a 6 to 14 membered spiro cycloalkyl, more preferably a 7 to 10 membered spiro cycloalkyl. The spiro cycloalkyl can be classified into mono-spiro cycloalkyl, di-spiro cycloalkyl, or poly-spiro cycloalkyl according to the number of the spiro atoms shared between the rings, preferably mono-spiro cycloalkyl or di-spiro cycloalkyl, and more preferably a 4 membered/4 membered, 4 membered/5 membered, 4 membered/6 membered, 5 membered/5 membered or 5 membered/6 membered mono-spiro cycloalkyl. Non-limiting examples of spiro cycloalkyl include:

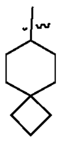 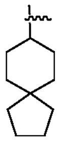 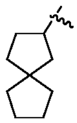 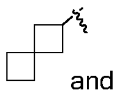 and 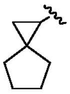 .

The term "fused cycloalkyl" refers to a 5 to 20 membered all-carbon polycyclic group in which each ring in the system shares an adjacent pair of carbon atoms with another ring in the system, wherein one or more of the rings can contain one or more double bonds, but none of the rings has a fully conjugated π-electron system. The fused cycloalkyl is preferably a 6 to 14 membered fused cycloalkyl, and more preferably a 7 to 10 membered fused cycloalkyl. The fused cycloalkyl can be classified into bicyclic, tricyclic, tetracyclic or polycyclic fused cycloalkyl according to the number of membered rings, preferably a bicyclic or tricyclic fused cycloalkyl, and more preferably a 5 membered/5 membered or 5 membered/6 membered bicyclic fused cycloalkyl. Non-limiting examples of fused cycloalkyl include:

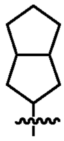 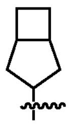 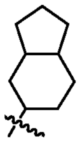 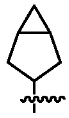 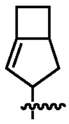 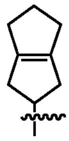 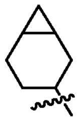 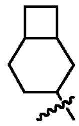 and 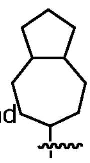 .

The term "bridged cycloalkyl" refers to a 5 to 20 membered all-carbon polycyclic group in which any two rings in the system share two disconnected carbon atoms, which can contain one or more double bonds, but none of the rings has a fully conjugated π-electron system. The bridged cycloalkyl is preferably a 6 to 14 membered bridged cycloalkyl, and more preferably a 7 to 10 membered bridged cycloalkyl. The bridged cycloalkyl can be classified into bicyclic, tricyclic, tetracyclic or polycyclic bridged cycloalkyl according to the number of membered rings, preferably a bicyclic, tricyclic or tetracyclic bridged cycloalkyl, and more preferably a bicyclic or tricyclic bridged cycloalkyl. Non-limiting examples of bridged cycloalkyl include:

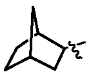 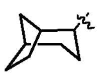 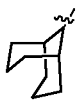 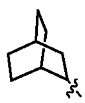 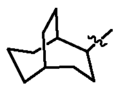

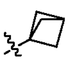 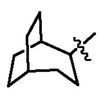 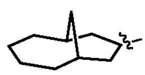 and 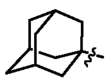 .

The cycloalkyl ring can be fused to an aryl, heteroaryl or heterocyclyl ring, wherein the ring attached to the parent structure is the cycloalkyl. Non-limiting examples include indanyl, tetrahydronaphthyl, benzocycloheptyl and the like. The cycloalkyl can be optionally substituted or unsubstituted. When substituted, the substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, oxo, carboxyl and carboxylate.

The term "heterocyclyl" refers to a 3 to 20 membered saturated or partially unsaturated monocyclic or polycyclic hydrocarbon substituent group, wherein one or more ring atoms are heteroatoms selected from the group consisting of nitrogen, oxygen and $S(O)_m$ (wherein m is an integer of 0 to 2), but excluding the ring moiety of -O-O-, -O-S- or -S-S-, and the rest ring atoms are carbon atoms. The heterocyclyl preferably comprises 3 to 12 ring atoms, wherein 1 to 4 are heteroatoms; more preferably comprising 3 to 8 ring atoms, wherein 1 to 3 are heteroatoms; most preferably comprising 5 to 7 ring atoms, wherein 1 to 2 or 1 to 3 are heteroatoms. Non-limiting examples of monocyclic heterocyclyl include oxetanyl, azetidinyl, pyrrolidinyl, imidazolidinyl, tetrahydrofuranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl and the like, preferably 1,2,5-oxadiazolyl, pyranyl or morpholinyl. Polycyclic heterocyclyl includes a heterocyclyl having a spiro ring, fused ring or bridged ring.

The term "spiro heterocyclyl" refers to a 5 to 20 membered polycyclic heterocyclyl group with individual rings connected through one shared atom (called a spiro atom), wherein one or more ring atoms are selected from the group consisting of nitrogen, oxygen and $S(O)_m$ (m is an integer of 0 to 2), and the rest ring atoms are carbon atoms. The spiro heterocyclyl can contain one or more double bonds, but none of the rings have a fully conjugated $\pi$-electron system. The spiro heterocyclyl is preferably a 6 to 14 membered spiro heterocyclyl, and more preferably a 7 to 10 membered spiro heterocyclyl. The spiro heterocyclyl can be classified into mono-spiro heterocyclyl, di-spiro heterocyclyl or poly-spiro cyclyl according to the number of the spiro atoms shared between the rings, preferably mono-spiro heterocyclyl and di-spiro heterocyclyl, and more preferably a 4 membered/4 membered, 4 membered/5 membered, 4 membered/6 membered, 5 membered/5 membered or 5 membered/6 membered mono-spiro heterocyclyl. Non-limiting examples of spiro heterocyclyl include:

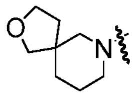 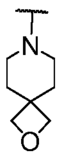 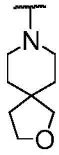 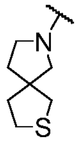 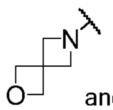 and 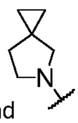.

The term "fused heterocyclyl" refers to a 5 to 20 membered polycyclic heterocyclic group in which each ring in the system shares an adjacent pair of atoms with another ring in the system, and one or more rings can contain one or more double bonds, but none of the rings have a fully conjugated $\pi$-electron system, wherein one or more ring atoms are heteroatoms selected from the group consisting of nitrogen, oxygen and $S(O)_m$ (m is an integer of 0 to 2), and the rest ring atoms are carbon atoms. The fused heterocyclyl is preferably a 6 to 14 membered fused heterocyclyl, and more preferably a 7 to 10 membered fused heterocyclyl. The fused heterocyclyl can be classified into bicyclic, tricyclic, tetracyclic or polycyclic fused heterocyclyl according to the number of membered rings, preferably a bicyclic or tricyclic fused heterocyclyl, and more preferably a 5 membered/5 membered or 5 membered/6 membered bicyclic fused heterocyclyl. Non-limiting examples of fused heterocyclyl include:

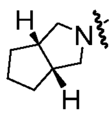 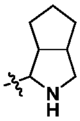 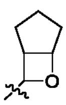 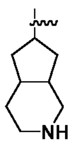 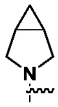 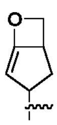 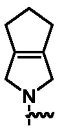 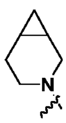

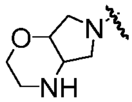 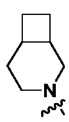 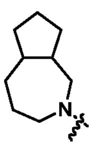 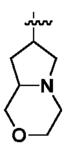 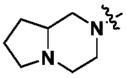 and 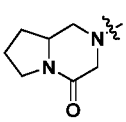.

The term "bridged heterocyclyl" refers to a 5 to 14 membered polycyclic heterocyclyl group in which any two rings share two disconnected atoms, which can contain one or more double bonds, but none of the rings have a fully conjugated $\pi$-electron system, wherein one or more ring atoms are heteroatoms selected from the group consisting of nitrogen, oxygen and $S(O)_m$ (m is an integer of 0 to 2), and the rest ring atoms are carbon atoms. The bridged heterocyclyl is preferably a 6 to 14 membered bridged heterocyclyl, and more preferably a 7 to 10 membered bridged heterocyclyl. The bridged heterocyclyl can be classified into bicyclic, tricyclic, tetracyclic or polycyclic bridged heterocyclyl according to the number of membered rings, preferably a bicyclic, tricyclic or tetracyclic bridged heterocyclyl, and more preferably a bicyclic or tricyclic bridged heterocyclyl. Non-limiting examples of bridge heterocyclyl include:

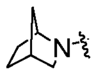 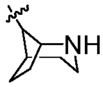 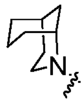 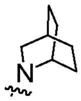 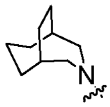

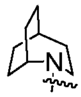 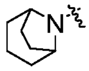 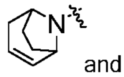 and 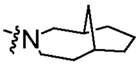 .

The heterocyclic ring can be fused to an aryl, heteroaryl or cycloalkyl ring, wherein the ring attached to the parent structure is heterocyclyl. Non-limiting examples include:

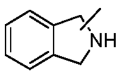 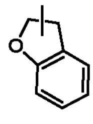 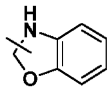 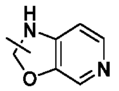 and 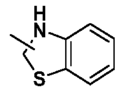

and the like.

The heterocyclyl can be optionally substituted or unsubstituted. When substituted, the substituent group(s) is preferably one or more groups independently selected from the group consisting of alkyl, alkenyl, alkynyl, alkoxyl, alkylthiol, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, oxo, carboxyl and carboxylate.

The term "aryl" refers to a 6 to 14 membered all-carbon monocyclic or polycyclic fused ring (*i.e.* each ring in the system shares an adjacent pair of carbon atoms with another ring in the system) having a conjugated $\pi$-electron system, preferably a 6 to 10 membered aryl, for example phenyl and naphthyl. The aryl is more preferably phenyl. The aryl ring can be fused to a heteroaryl, heterocyclyl or cycloalkyl ring, wherein the ring attached to the parent structure is aryl ring. Non-limiting examples include:

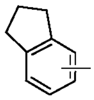 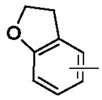 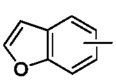 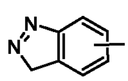 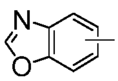 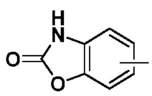 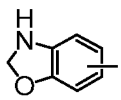 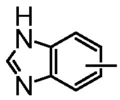 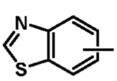

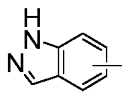 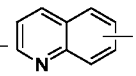 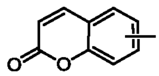 and 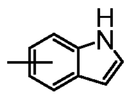 .

The aryl can be substituted or unsubstituted. When substituted, the substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, carboxyl and carboxylate.

The term "heteroaryl" refers to a 5 to 14 membered heteroaromatic system having 1 to 4 heteroatoms selected from the group consisting of oxygen, sulfur and nitrogen and 5 to 14 ring atoms. The heteroaryl is preferably a 5 to 10 membered heteroaryl comprising 1 to 3 heteroatoms; more preferably a 5 or 6 membered heteroaryl comprising 1 to 2 heteroatoms; preferably for example imidazolyl, furyl, thienyl, thiazolyl, pyrazolyl, oxazolyl, pyrrolyl, tetrazolyl, pyridyl, pyrimidinyl, thiadiazole, pyrazinyl and the like, preferably imidazolyl, thiazolyl, pyrazolyl or pyrimidinyl, thiazolyl; more preferably pyrazolyl or thiazolyl. The heteroaryl ring can be fused to an aryl, heterocyclyl or cycloalkyl ring, wherein the ring attached to the parent structure is the heteroaryl ring. Non-limiting examples include:

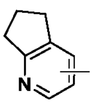 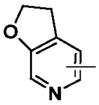 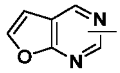 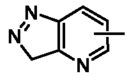 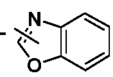 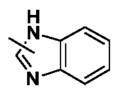 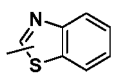 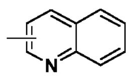 and 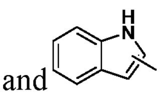 .

The heteroaryl can be optionally substituted or unsubstituted. When substituted, the substituent group(s) is preferably one or more groups independently selected from the group consisting of alkyl, alkenyl, alkynyl, alkoxyl,

alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, carboxyl and carboxylate.

The term "alkoxyl" refers to -O-(alkyl) and -O-(unsubstituted cycloalkyl), wherein the alkyl is as defined above. Non-limiting examples of alkoxyl include methoxyl, ethoxyl, propoxyl, butoxyl, cyclopropoxyl, cyclobutoxyl, cyclopentyloxyl and cyclohexyloxyl. The alkoxyl can be optionally substituted or unsubstituted. When substituted, the substituent group(s) is preferably one or more groups independently selected from the group consisting of alkyl, alkenyl, alkynyl, alkoxyl, alkylthio, alkylamino, halogen, thiol, hydroxyl, nitro, cyano, cycloalkyl, heterocyclyl, aryl, heteroaryl, cycloalkoxyl, heterocycloalkoxyl, cycloalkylthio, heterocyclylthio, carboxyl and carboxylate.

The term "haloalkyl" refers to an alkyl substituted with one or more halogen(s), wherein the alkyl is as defined above.

The term "haloalkoxyl" refers to an alkoxyl substituted with one or more halogen(s), wherein the alkoxyl is as defined above.

The term "hydroxyalkyl" refers to an alkyl substituted with hydroxyl, wherein the alkyl is as defined above.

The term "hydroxyl" refers to an -OH group.

The term "halogen" refers to fluorine, chlorine, bromine or iodine.

The term "amino" refers to $-NH_2$.

The term "cyano" refers to -CN.

The term "nitro" refers to $-NO_2$.

The term "oxo" refers to =O.

The term "carboxyl" refers to -C(O)OH.

The term "thiol" refers to -SH.

The term "alkoxycarbonyl" refers to -C(O)O(alkyl) or -C(O)O(cycloalkyl), wherein the alkyl and cycloalkyl are as defined above.

The term "acyl" refers to a compound containing a -C(O)R group, wherein R is alkyl, cycloalkyl, heterocyclyl, aryl or heteroaryl.

The term "sulfonic acid group" refers to $-S(O)_2OH$.

The term "sulfonate group" refers to $-S(O)_2O$(alkyl) or $-S(O)_2O$(cycloalkyl), wherein the alkyl and cycloalkyl are as defined above.

The term "sulfonyl" refers to compound of $-S(O)_2R$ group, wherein R is alkyl, cycloalkyl, heterocyclyl, aryl or heteroaryl.

The term "aminoacyl" refers to -C(O)-NRR', wherein each of R and R' is independently hydrogen, alkyl, cycloalkyl, heterocyclyl, aryl or heteroaryl.

The term "aminosulfonyl" or "sulfonylamino" refers to $-S(O)_2$-NRR', wherein each of R and R' is independently hydrogen, alkyl, cycloalkyl, heterocyclyl, aryl or heteroaryl.

"Optional" or "optionally" means that the event or circumstance described subsequently can, but need not occur, and such a description includes the situation in which the event or circumstance does or does not occur. For example, "the heterocyclyl optionally substituted by alkyl" means that an alkyl can be, but need not to be present, and such a description includes the situation of the heterocyclyl being substituted by alkyl and the heterocyclyl being not substituted by alkyl.

"Substituted" refers to one or more hydrogen atoms in the group, preferably up to 5, and more preferably 1 to 3 hydrogen atoms, independently substituted by the corresponding number of substituents. It goes without saying that the substituents only exist in their possible chemical positions. Those skilled in the art is able to determine whether the substitution is possible or impossible by experiments or theory without excessive effort. For example, the binding of an amino or a hydroxyl having free hydrogen to a carbon atom having unsaturated bond (such as olefinic) may be unstable.

"Pharmaceutical composition" refers to a mixture containing one or more of the compounds described herein, or a physiologically/pharmaceutically acceptable salt or a prodrug thereof, and other chemical components, as well as other components, such as physiological/pharmaceutically acceptable carrier and excipient. The purpose of the pharmaceutical composition is to facilitate administration of a compound to an organism, which is conducive to the absorption of the active ingredient so as to show the biological activity.

A "Pharmaceutically acceptable salt" refers to a salt of the compound of the present invention, which is safe and effective for use in mammals and possesses the desired biological activity.

METHODS FOR SYNTHESIZING THE COMPOUNDS OF THE PRESENT INVENTION

In order to achieve the objective of the present invention, the following technical solutions are used in the present invention.

The compound represented by general formula (I) of the present invention or a salt thereof can be prepared by the following scheme:

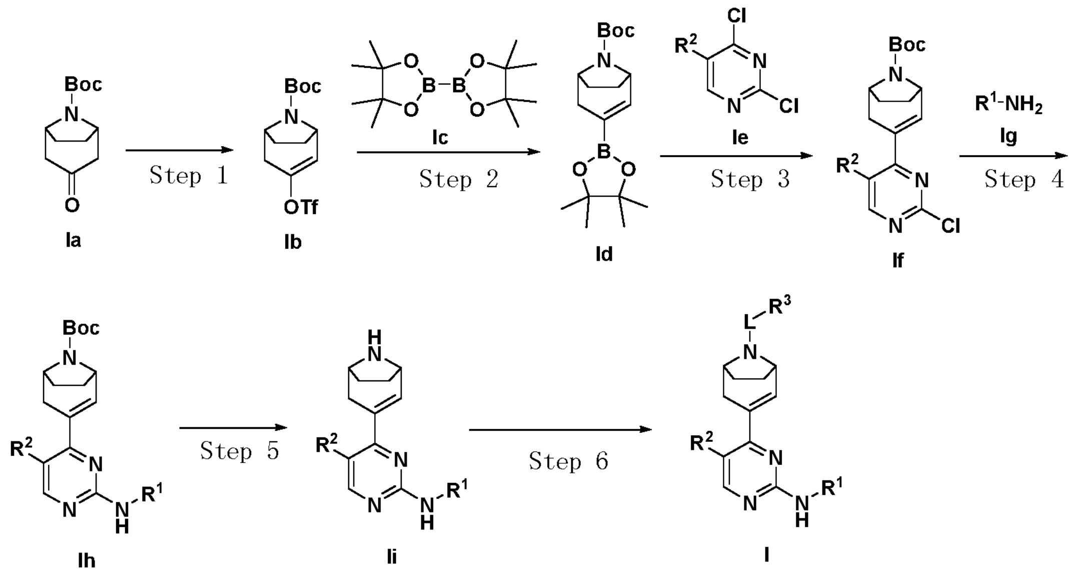


Step 1: Reacting compound Ia with *N*-phenylbis(trifluoromethanesulfonyl)imide under alkaline conditions to obtain compound Ib, wherein the reagent providing alkaline conditions is preferably potassium hexamethyldisilazide;
Step 2: Reacting compound Ib with pinacol diborate (Ic) under alkaline conditions in the presence of a catalyst to obtain compound Id, wherein the reagent providing alkaline conditions is preferably potassium acetate, and the catalyst is preferably Pd(dppf)$Cl_2$-$CH_2Cl_2$;
Step 3: Reacting compound Id with compound Ie under alkaline conditions in the presence of a catalyst to obtain compound If, wherein the reagent providing alkaline conditions is preferably potassium carbonate, and the catalyst is preferably Pd(dppf)$Cl_2$;
Step 4: Reacting compound If with compound Ig under acidic conditions to obtain compound Ih, wherein the reagent providing acidic conditions is preferably p-toluenesulfonic acid;
Step 5: Compound Ih is subjected to a deprotection reaction under acidic conditions to obtain compound Ii, wherein the reagent providing acidic conditions is preferably trifluoroacetic acid;
Step 6: reacting compound Ii with $R^3$-L-X (X=Cl, Br, I, OPh or

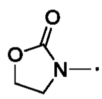
)

under alkaline conditions to obtain a compound of general formula (I), wherein the reagent providing alkaline conditions is preferably triethylamine; or reacting compound Ii with $R^3$-L-OH under alkaline conditions in the presence of a catalyst to obtain a compound of general formula (I), wherein the reagent providing alkaline conditions is preferably DIPEA, and the catalyst is preferably HATU,
wherein, $R^1$, $R^2$, $R^3$ and L are as defined in the compound of general formula (I).

## DETAILED DESCRIPTION

The present invention will be further described with reference to the following examples, but the examples should not be considered as limiting the scope of the present disclosure.
The structures of the compounds are identified by nuclear magnetic resonance (NMR) and/or mass spectrometry (MS). NMR shift is given in $10^{-6}$ (ppm). NMR is determined by a Brukerdps300 nuclear magnetic spectrometer. The solvents for determination are deuterated dimethyl sulfoxide (DMSO-$d_6$), deuterated chloroform ($CDCl_3$), deuterated methanol ($CD_3OD$), and the internal standard is tetramethylsilane (TMS).
MS is determined by a 1100 Series LC/MSD Trap (ESI) mass spectrometer (manufacturer: Agilent).
Unless specified in the examples, a lc3000 high performance liquid chromatograph and a lc6000 high performance liquid chromatograph (manufacturer: ChuangXin TongHeng) are used for preparative liquid chromatograph. The chromatographic column is DaisogelC18 10 μm 60A (20 mm×250 mm). Mobile phase: acetonitrile, water (0.05% formic acid).

HPLC is determined by a Shimadzu LC-20AD high pressure liquid chromatograph (Agilent TC-C18 250×4.6 mm 5 μm column) and a Shimadzu LC-2010AHT high pressure liquid chromatograph (Phenomenex C18 250×4.6mm 5μm column).

Qingdao Haiyang Chemical GF254 silica gel plates are used for thin layer chromatography (TLC), and the specification is 0.15 mm to 0.2 mm for analysis, and 0.4 mm to 0.5 mm for separation and purification of products.

Qingdao Haiyang 100 to 200 mesh and 200 to 300 mesh silica gel are generally used as the carrier for column chromatography.

The known starting materials of the present invention can be synthesized by or according to methods known in the art, or can be purchased from WHall, Beijing Ouhe, Sigma, J&K Scientific, Yishiming (Beijing), Shanghai Shuya, Innochem, Nanjing Pharmablock, Energy Chemical and other companies.

Unless specified in the examples, all reactions can be carried out under argon atmosphere or nitrogen atmosphere.

Argon atmosphere or nitrogen atmosphere means that the reaction flask is connected to an argon or nitrogen balloon with a volume of about 1 L.

A CEM Discover SP microwave reactor is used for microwave reaction.

Unless specified in the examples, a solution refers to an aqueous solution.

Unless specified in the examples, the reaction temperature is room temperature, which is 20°C to 30 °C.

The progress of reactions in the examples is monitored by thin layer chromatography (TLC). The systems of the developing agents used for the reactions are: A: dichloromethane and methanol system, B: n-hexane and ethyl acetate system, C: petroleum ether and ethyl acetate system, D: acetone. The volume ratio of the solvents is adjusted according to the polarity of the compound.

The eluent system of column chromatography and the developing solvent system of thin layer chromatography used to purify the compounds include: A: dichloromethane and methanol system, B: petroleum ether, ethyl acetate and dichloromethane system, C: petroleum ether and ethyl acetate system. The volume ratio of the solvents is adjusted according to the polarity of the compound. A small amount of triethylamine, acetic acid or other basic or acidic reagents can also be added for adjustment.

EXAMPLES

Example 1: Preparation of ((*S*)-2,2-difluorocyclopropyl)(3-(2-((1-methyl-1*H*-pyrazol-4-yl)amino)pyrimidin-4-yl)-8-azabicyclo[3.2.1]oct-2-en-8-yl)methanone (1)

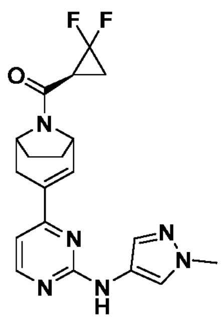

**1**

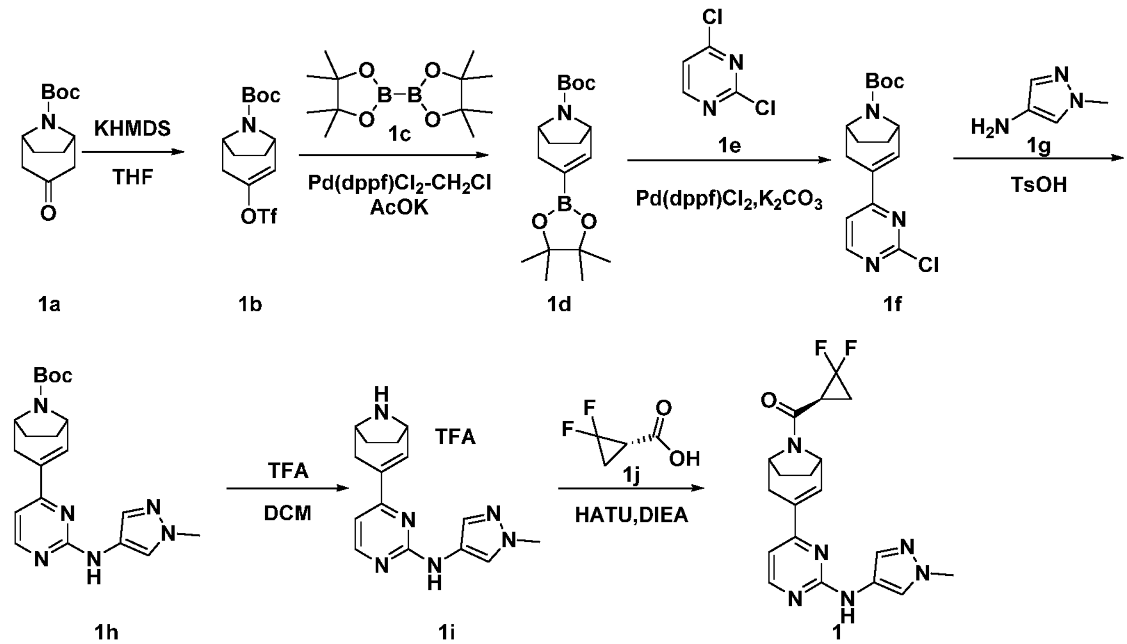


1a 1b 1d 1f

1h 1i 1

**Step 1:** Synthesis of tert-butyl 3-(((trifluoromethyl)sulfonyl)oxy)-8-azabicyclo [3.2.1]oct-2-ene-8-carboxylate (**1b**)

Potassium hexamethyldisilazide (10.7 mL, 10.7 mmol) was added to a mixed solution of tert-butyl 3-oxo-8-azabicyclo[3.2.1]octane-8-carboxylate (2.00 g, 8.88 mmol) in anhydrous tetrahydrofuran (30 mL) at -78°C under nitrogen atmosphere, and the mixture was stirred at -78°C for 0.5 hour. A solution of *N*-phenylbis (trifluoromethanesulfonyl)imide (3.82 g, 10.7 mmol) in anhydrous tetrahydrofuran solution (20 mL) was added dropwise, and after completion of the addition, the mixture was stirred at -78°C for 2 hours. The reaction was quenched by adding a saturated aqueous solution of ammonium chloride (20 mL). The mixture was extracted with ethyl acetate (30 mL*3). The organic phase was washed with a solution of potassium hydroxide (1 mol/L) and saturated brine, dried over anhydrous sodium sulfate and concentrated under reduced pressure. The residues were purified by flash column chromatography (mobile phase: petroleum ether/ethyl acetate, 10/1 to 2/1) to give 3.10 g of the title compound as a pale yellow oil. Yield: 97.8%.

**Step 2:** Synthesis of tert-butyl 3-(4,4,5,5-tetramethyl-1,3,2-dioxaborolan-2-yl)-8-azabicyclo[3.2.1]oct-2-ene-8-carboxylate (**1d**)

Pd(dppf)$Cl_2$ dichloromethane complex (423 mg, 0.518 mmol) was added to a minxture of tert-butyl 3-(((trifluoromethyl)sulfonyl)oxy)-8-azabicyclo[3.2.1]oct-2-ene-8-carboxylate (3.70 g, 10.4 mmol), potassium acetate (3.05 g, 31.1 mmol) and pinacol diborate (2.90 g, 11.4 mmol) in dioxane (50 mL) under nitrogen atmosphere at room temperature, and the mixture was stirred overnight at 80°C. The solvent was removed by rotary evaporation. The mixture was added to water (40 mL) and extracted with ethyl acetate (50 mL*3). The organic phase was washed with saturated brine, dried over anhydrous sodium sulfate and concentrated under reduced pressure. The residues were purified by flash column chromatography (mobile phase: petroleum ether/ethyl acetate = 10/1 to 2/1) to give 1.20 g of the title compound as a yellow oil. Yield: 34.6%.

**Step 3:** Synthesis of tert-butyl 3-(2-chloropyrimidin-4-yl)-8-azabicyclo[3.2.1]oct-2-ene-8-carboxylate (**1f**)

Pd(dppf)$Cl_2$ (262 mg, 0.358 mmol) was added to a mixture of tert-butyl 3-(4,4,5,5-tetramethyl-1,3,2-dioxaborolan-2-yl)-8-azabicyclo[3.2.1]oct-2-ene-8-carboxylic acid (1.20 g, 3.58 mmol), potassium carbonate (1.24 g, 8.95 mmol) and 2,4-dichloropyrimidine (534 mg, 3.58 mmol) in dioxane (40 mL) and water (10 mL) under nitrogen atmosphere at room temperature, and the mixture was stirred overnight at 80°C. The solvent was removed by rotary evaporation. The mixture was added to water (40 mL) and extracted with ethyl acetate (50 mL*3). The organic phase was washed with saturated brine, dried over anhydrous sodium sulfate and concentrated under reduced pressure. The residues were purified by flash column chromatography (mobile phase: petroleum ether/ethyl acetate = 10/1 to 1/1) to give 830 mg of the title compound as a yellow oil. Yield: 72.1 %.

**Step 4:** Synthesis of tert-butyl 3-(2-((1-methyl-1*H*-pyrazol-4-yl)amino)pyrimidin-4-yl)-8-azabicyclo[3.2.1]oct-2-ene-8-carboxylate (**1h**)

p-Toluenesulfonic acid (37.3 mg, 0.217 mmol) was added to a solution of tert-butyl 3-(2-chloropyrimidin-4-yl)-8-azabicyclo[3.2.1]oct-2-ene-8-carboxylate (700 mg, 2.17 mmol) and 1-methyl-1*H*-pyrazol-4-amine (211 mg, 2.17 mmol) in dioxane (10 mL) at room temperature, and the mixture was stirred at 90°C overnight. The solvent was removed by rotary evaporation. The mixture was added to water (40 mL) and extracted with ethyl acetate (50 mL*3). The organic phase was washed with saturated brine, dried over anhydrous sodium sulfate and concentrated under reduced pressure. The residues were purified by flash column chromatography (mobile phase: petroleum ether/ethyl acetate = 10/1 to 1/1) to give 600 mg of the title compound as a brown oil. Yield: 72.4 %.

**Step 5:** Synthesis of 4-(8-azabicyclo[3.2.1]oct-2-en-3-yl)-*N*-(1-methyl-1*H*-pyrazol-4-yl)pyrimidin-2-amine trifluoroacetate (**1i**)

Trifluoroacetic acid (2 mL) was added to a solution of tert-butyl 3-(2-((1-methyl-1*H*-pyrazol-4-yl)amino)pyrimidin-4-yl)-8-azabicyclo[3.2.1]oct-2-ene-8 -carboxylate (200 mg, 0.524 mmol) in dichloromethane (6 mL) at room temperature. The mixture was stirred for 30 minutes and concentrated at low temperature to give 157 mg of the crude title compound as a white solid.

**Step 6:** Synthesis of ((*S*)-2,2-difluorocyclopropyl)(3-(2-((1-methyl-1*H*-pyrazol-4-yl)amino)pyrimidin-4-yl)-8-azabicyclo[3.2.1]oct-2-en-8-yl)methanone (**1**)

2-(7-Oxidobenzotriazole)-*N*;*N*;*N*',*N*'-tetramethylurea hexafluorophosphate (224 mg, 0.590 mmol) was added to a solution of (*S*)-2,2-difluorocyclopropane-1-carboxylic acid (60 mg, 0.492 mmol) in *N*,*N*-dimethylformamide (10 mL) at room temperature, and the mixture was stirred at room temperature for 30 minutes. 4-(8-Azabicyclo[3.2.1] oct-2-en-3-yl)-*N*-(1-methyl-1*H*-pyrazol-4-yl)pyrimidin-2-amine trifluoroacetate (190 mg, 1.48 mmol) and *N*,*N*-diisopropylethylamine (190 mg, 1.48 mmol) were added, and the mixture was stirred at room temperature overnight. The mixture was added to water (50 mL) and extracted with ethyl acetate (30 mL × 3). The organic phase was washed with saturated brine, dried over anhydrous sodium sulfate and concentrated under reduced pressure. The residues were purified by preparative liquid chromatography to give 95.0 mg of the title compound as a yellow solid. Yield: 59.4%.

Purification method by preparative liquid chromatography: column: 30 mm × 250 mm; packing: C18, 10 μm; process: 2-22 min, acetonitrile 10-50%; wavelength: 254 nm; flow rate: 45 mL/min; mobile phase: acetonitrile, water.

LC-MS: m/z 387 $[M+H]^+$;

$^1H$ NMR (300 MHz, $CD_3OD$): δ ppm 8.28 (m, 1 H), 7.89 (s, 1 H), 7.55 (s, 1 H), 7.12-7.22 (m, 1 H), 6.81-6.83 (m, 1 H), 4.94-5.05 (m, 1 H), 4.75-4.77 (m, 1 H),3.86-3.88 (m, 3 H), 2.98-3.10 (m, 2 H), 2.52-2.69 (m, 1 H), 2.16-2.41 (m, 2 H), 2.00-2.12 (m, 2 H), 1.75-1.79 (m, 2 H).

Examples 1-a and 1-b: Preparation of compounds **1-a** and **1-b**

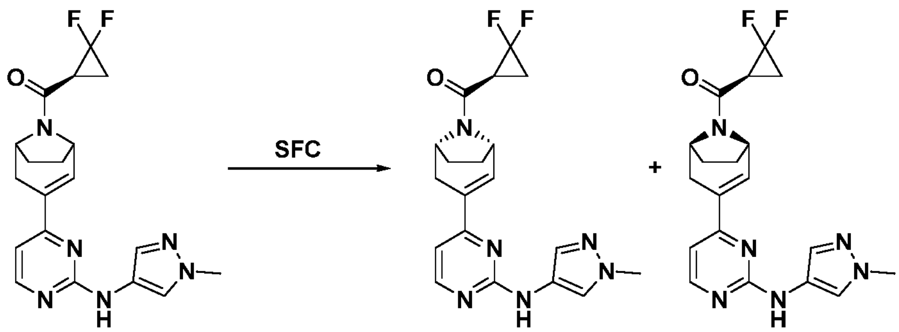


1

**1-a** and **1-b**

Compounds **1-a** and **1-b** were obtained by separation from compound 1 by supercritical liquid chromatography (SFC).

SFC separation conditions:

Column: AS-H 4.6 mm x 250 mm, 5 μm, mobile phase: MeOH (0.2% $NH_3 \cdot H_2O$)/$CO_2$ = 35:65, flow rate: 40 g/min.
**1-a:** retention time: 2.87 min;
LC-MS: m/z 387 $[M+H]^+$
1H NMR (400 MHz, DMSO): δppm9.36 (s, 1H), 8.35-8.29 (m, 1H), 7.83 (s, 1H), 7.49 (s, 1H), 7.18-7.06 (m, 1H), 6.84-6.80 (m, 1H),4.85-4.79 (m, 2H), 3.81 (s, 3H), 3.18-3.05 (m, 1H), 2.88-2.81 (m, 1H), 2.51-2.49 (m, 1H), 2.39-2.29 (m, 1H), 2.10-1.66 (m, 5H).
**1-b:** retention time: 3.79 min.
LC-MS: m/z 387 $[M+H]^+$
$^1$H NMR (400 MHz, DMSO): δppm9.36 (s, 1H), 8.34 (d, $J$ = 5.2 Hz,1H), 7.82 (d, $J$ = 6 Hz, 1H), 7.51 (s, 1H), 7.19-7.15 (m, 1H), 6.83-6.82 (m, 1H),4.90-4.71 (m, 2H), 3.81 (s, 3H), 3.21-3.16 (m, 1H), 3.06-2.90 (m, 1H), 2.51-2.27 (m, 1H), 2.11-2.09 (m, 1H), 1.98-1.67 (m, 5H).

Example 2: Preparation of *N*-(cyanomethyl)-3-(2-((1-methyl-1*H*-pyrazol-4-yl) amino)pyrimidin-4-yl)-8-azabicyclo[3.2.1]oct-2-ene-8-carboxamide (**2**)

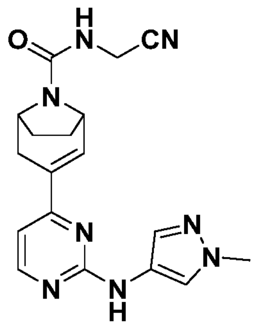

**2**

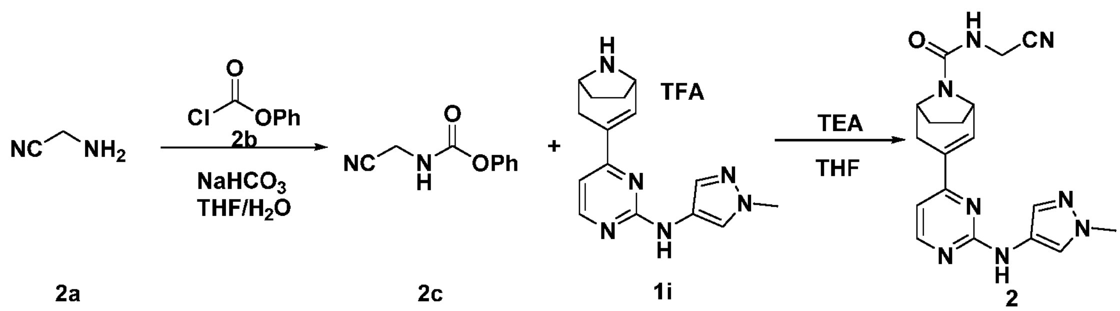


2a 2c 1i 2

**Step 1:** Synthesis of phenyl (cyanomethyl)carbamate (**2c**)

Phenyl chloroformate (844 mg, 5.38 mmol) was added to a mixed solution of 2-aminoacetonitrile (500 mg, 5.38 mmol) in tetrahydrofuran (6 mL) and saturated aqueous solution of sodium bicarbonate (2 mL) at 0°C, and the mixture was stirred at 0°C for 30 min. Water was added (20 mL) and the mixture was extracted with ethyl acetate (30 mL*3). The organic phase was washed with saturated brine, dried over anhydrous sodium sulfate and concentrated under reduced pressure. The residues were purified by flash column chromatography (mobile phase: petroleum ether/ethyl acetate, 100/1 to 10/1) to give 800 mg of the title compound as a white solid. Yield: 84.6%.

**Step 2:** Synthesis of *N*-(cyanomethyl)-3-(2-((1-methyl-1*H*-pyrazol-4-yl)amino) pyrimidin-4-yl)-8-azabicyclo[3.2.1]oct-2-ene-8-carboxamide (**2**)

Triethylamine (102 mg, 1.01 mmol) was added to a solution of 4-(8-azabicyclo[3.2.1]oct-2-en-3-yl)-*N*-(1-methyl-1*H*-pyrazol-4-yl)pyrimidin-2-amine trifluoroacetate (200 mg, 0.504) and phenyl (cyanomethyl)carbamate (106 mg, 0.605 mmol) in tetrahydrofuran (5 mL) at room temperature. The mixture was stirred at 60°C overnight and concentrated under reduced pressure. The residues were purified by preparative liquid chromatography to give 11.0 mg of the title compound as a yellow solid. Yield: 5.99%.

Purification method by preparative liquid chromatography: column: 30 mm × 250 mm; packing: C18, 10 μm; process: 2-22 min, acetonitrile 10-50%; wavelength: 254 nm; flow rate: 45 mL/min; mobile phase: acetonitrile, water.
LC-MS: m/z 365 $[M+H]^+$;
$^1H$ NMR (300 MHz, DMSO-$d_6$): δ ppm9.32 (s, 1H), 8.33-8.31 (m, 1 H), 7.81 (s, 1 H), 7.51 (s, 1 H), 7.40-7.31 (m, 1 H),7.26-7.15 (m, 1 H),6.82-6.80 (m, 1H),4.57-4.48 (m, 2 H),4.04-4.02 (m, 2 H), 3.80 (s, 3 H),2.97-2.92 (m, 1 H),2.33-2.28 (m, 1 H),2.20-2.10 (m, 1 H),2.05-1.85 (m, 2 H), 1.68-1.64 (m, 1 H).

Example 3: Preparation of 3-(2-((1-methyl-1*H*-pyrazol-4-yl)amino)pyrimidin-4-yl)-*N*-(2,2,2-trifluoroethyl)-8-azabicyclo[3.2.1]oct-2-ene-8-carboxamide (**3**)

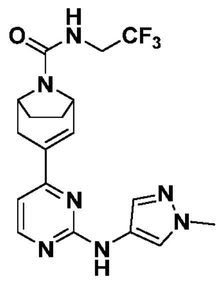

**3**

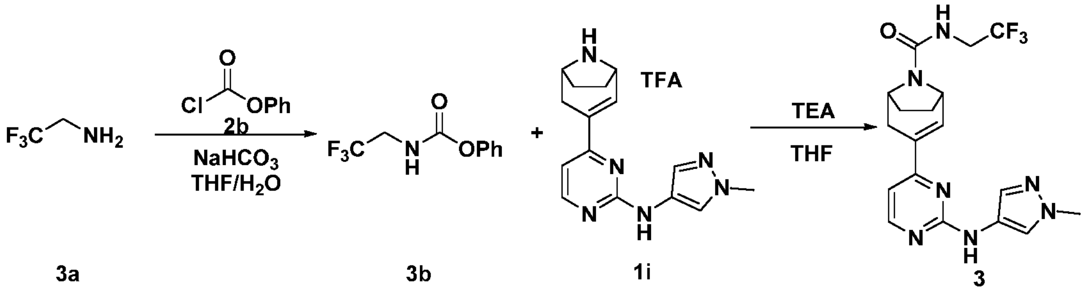


3a 3b 1i 3

**Step 1:** Synthesis of phenyl (2,2,2-trifluoroethyl)carbamate (**3b**)

Phenyl chloroformate (476 mg, 3.03 mmol) was added to a mixed solution of 2,2,2-trifluoroethylamine (300 mg, 3.03 mmol) in tetrahydrofuran (6 mL) and saturated aqueous solution of sodium bicarbonate (2 mL) at 0°C, and the mixture was stirred at 0°C for 30 min. Water was added (20 mL) and the mixture was extracted with ethyl acetate (30 mL*3). The organic phase was washed with saturated brine, dried over anhydrous sodium sulfate, concentrated under reduced pressure and purified by flash column chromatography (mobile phase: PE/EA = 100:1 - 10:1) to give 593 mg white solid. Yield: 89.3%.
LC-MS: m/z=220[M+H].

Step 2: Synthesis of 3-(2-((1-methyl-1*H*-pyrazol-4-yl)amino)pyrimidin-4-yl)-*N*-(2,2,2-trifluoroethyl)-8-azabicyclo[3.2.1]oct-2-ene-8-carboxamide (**3**)

Triethylamine (102 mg, 1.01 mmol) was added to a solution of 4-(8-azabicyclo [3.2.1]oct-2-en-3-yl)-*N*-(1-methyl-1*H*-pyrazol-4-yl)pyrimidin-2-amine trifluoroacetate (200 mg, 0.504 mmol) and phenyl (2,2,2-trifluoroethyl)carbamate (133 mg, 0.605 mmol) in tetrahydrofuran (5 mL) at room temperature. The mixture was stirred at 60°C overnight and concentrated under reduced pressure. The residues were purified by preparative liquid chromatography to give 15 mg of white solid powder.
Purification method by preparative liquid chromatography: column: 30 mm × 250 mm; packing: C18, 10 μm; process: 2-22 min, acetonitrile 10-80%; wavelength: 254 nm; flow rate: 45 mL/min; mobile phase: acetonitrile, water.
LC-MS: m/z 408$[M+H]^+$;
$^1H$ NMR (300 MHz, DMSO-$d_6$): δppm 9.36 (s, 1 H),8.36 (d, *J*= 3 Hz, 1 H), 7.84 (s, 1 H), 7.55 (s, 1 H), 7.32-7.29 (m, 1 H), 7.24 (s, 1 H), 6.84 (d, *J*= 3 Hz, 1 H), 4.64 (s, 1 H), 4.56 (s, 1 H), 3.90-3.76 (m, 5 H), 2.98 (d, *J* = 12 Hz, 1 H),2.33

(d, *J* = 12 Hz, 1 H), 2.20-2.18 (m, 1 H), 2.02-1.96 (m, 2 H), 1.72-1.67 (m, 1 H).

Examples 3-a and 3-b: Preparation of compounds **3-a** and **3-b**

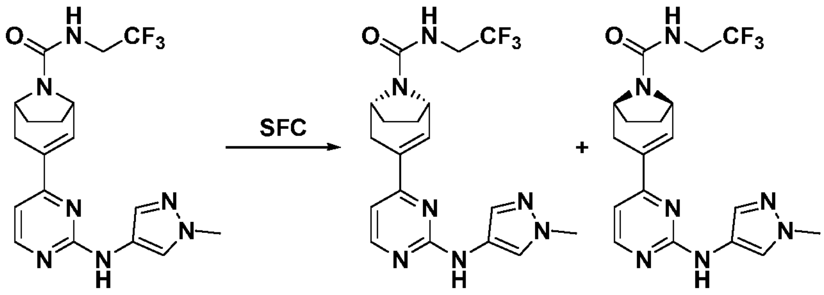


**3** **3-a** and **3-b**

Compounds **3-a** and **3-b** were obtained by resolution from compound **3** by SFC.
SFC separation conditions:

Column: (R,R) whelk-0121.1 mm x 250 mm, 5 μm, mobile phase: MeOH (0.2% $NH_3 \cdot H_2O$)/$CO_2$ = 35:65, flow rate: 40 g/min.
**3-a:** retention time: 5.19 min;

LC-MS: m/z 408 [M+H]+
$^1H$ NMR (400 MHz, CDC13): δppm 8.29 (d, J = 5.2 Hz, 1 H), 7.73 (s, 1 H), 7.54 (s, 1 H), 7.14-7.12 (m, 2 H), 6.66 (d, J = 5.2 Hz, 1 H), 5.04-5.01 (m, 1 H), 4.55-4.48 (m, 2 H), 3.94-3.92 (m, 1 H),3.88 (s, 3 H), 3.85-3.81 (m, 1 H), 3.12-3.07 (m, 1 H), 2.38-2.28 (m, 2 H), 2.02-1.86 (m, 2 H), 1.75-1.72 (m, 1 H).
**3-b:** retention time: 7.42 min.
LC-MS: m/z 408 $[M+H]^+$
$^1H$ NMR (400 MHz, CDC13): δppm 8.30 (d, J = 5.2 Hz, 1 H), 7.74 (s, 1 H), 7.54 (s, 1 H), 7.14 (s, 1 H), 6.87 (s, 1 H), 6.67 (d, J = 5.2 Hz, 1 H), 4.85-4.82 (m, 1 H), 4.55-4.47 (m, 2 H), 3.98-3.94 (m, 1 H),3.90 (s, 3 H), 3.86-3.82 (m, 1 H), 3.13-3.09 (m, 1 H), 2.40-2.29 (m, 2 H), 2.37-2.01 (m, 2 H), 1.79-1.67 (m, 1 H).

Example 4: Preparation of *N*-(cyanomethyl)-3-(2-((1-methyl-1*H*-pyrazol-4-yl) amino)pyrimidin-4-yl)-8-azabicyclo[3.2.1]oct-2-ene-8-sulfonamide (**4**)

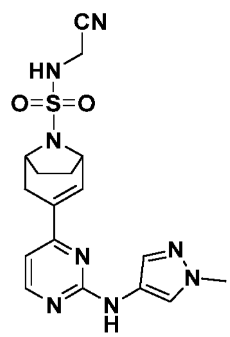

**4**

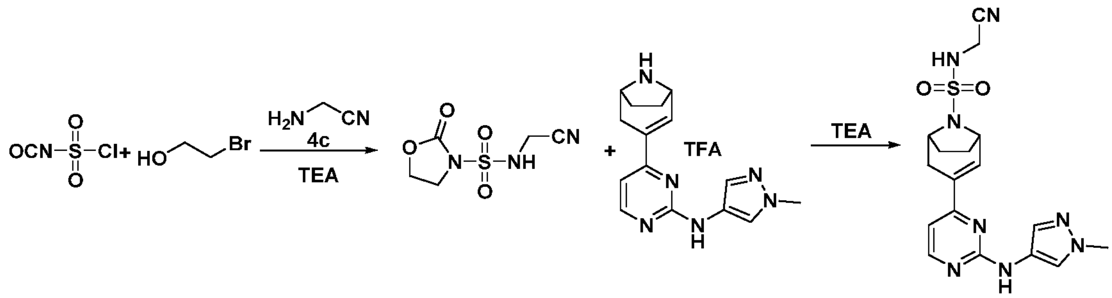


4a 4b 4d 1i 4

**Step 1:** Synthesis of *N*-(cyanomethyl)-2-oxooxazolidine-3-sulfonamide (**4d**)

A solution of 2-bromoethanol (1.25 g, 10.0 mmol) in dichloromethane (2 mL) was added dropwise to a solution of chlorosulfonyl isocyanate (1.42 g, 10 mmol) in dichloromethane (50 mL) at 0°C and reacted at 0°C for 1.5 hours. A solution of 2-aminoacetonitrile hydrochloride (925 mg, 10.0 mmol) and triethylamine (5.10 g, 50.0 mmol) in dichloromethane (40 mL) was added dropwise. After completion of the addition, the mixture was naturally warmed to room temperature and reacted for 30 minutes. An aqueous solution of hydrochloric acid (40 mL, 1 M) was added and the phases were separated. The organic phase was washed with saturated brine, dried and concentrated under reduced pressure to give 600 mg of brown oil, which was used directly in the next step without purification.

**Step 2:** Synthesis of *N*-(cyanomethyl)-3-(2-((1-methyl-1*H*-pyrazol-4-yl)amino) pyrimidin-4-yl)-8-azabicyclo[3.2.1]oct-2-ene-8-sulfonamide (**4**)

*N*-(cyanomethyl)-2-oxooxazolidine-3-sulfonamide (400 mg, 1.95 mmol), 4-(8-azabicyclo[3.2.1]oct-2-ene-3-yl)-*N*-(1-methyl-1*H*-pyrazol-4-yl)pyrimidin-2-amine trifluoroacetate (616 mg, 1.63 mmol) and triethylamine (660 mg, 6.52 mmol) were added to acetonitrile (15 mL) and reacted at 65°C overnight. The reaction solution was cooled to room temperature and concentrated. The residues were purified by preparative liquid chromatography to give 25.0 mg of the title product as a white solid. Yield: 3.83%.

Purification method by preparative liquid chromatography: column: 30 mm × 250 mm; packing: C18, 10 μm; process: 2-22 min, acetonitrile 15-55%; wavelength: 254 nm; flow rate: 45 mL/min; mobile phase: acetonitrile, water.

LC-MS: m/z 401[M+H]$^+$;

$^1$H NMR (300 MHz, DMSO-$d_6$): $\delta$ppm9.34 (s, 1 H), 8.33 (d,*J*= 5.1 Hz, 1 H), 8.26 (s, 1 H),7.83 (s, 1 H),7.50 (s, 1 H),7.16(d,*J*= 5.1 Hz, 1 H),6.82(d,*J*= 5.1 Hz, 1 H), 4.42-4.40 (m, 2 H), 4.06 (s, 2 H),3.80 (s, 3 H),3.06-3.04 (m, 1 H), 2.51-2.43 (m, 1 H),2.27-2.07 (m, 2 H),2.01-1.1.94 (m, 1 H),1.73-1.68 (m, 1 H).

Example 5: Preparation of 4,4,4-trifluoro-1-(3-(2-((1-methyl-1*H*-pyrazol-4-yl) amino)pyrimidin-4-yl)-8-azabicyclo[3.2.1]oct-2-en-8-yl)butan-1-one (**5**)

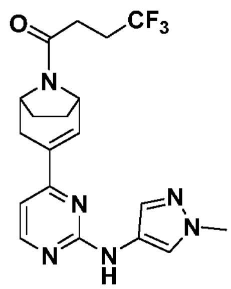

5

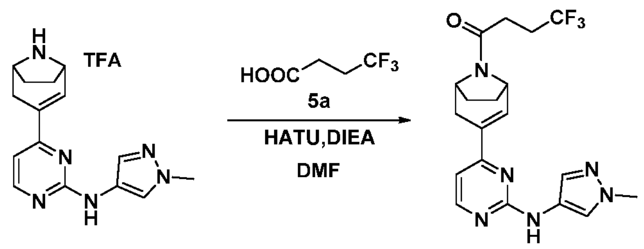


1i 5

2-(7-Oxidobenzotriazole)-*N,N,N;N'*-tetramethylurea hexafluorophosphate (345 mg, 0.907 mmol) was added to a solution of 4,4,4-trifluorobutyric acid (129 mg, 0.907 mmol) in *N*,*N*-dimethylformamide (5 mL) at room temperature, and the mixture was stirred at room temperature for 30 minutes. 4-(8-Azabicyclo[3.2.1]oct-2-en-3-yl)-*N*-(1-methyl-1*H*-pyrazol-4-yl)pyrimidin-2-amine trifluoroacetate (300 mg, 0.756 mmol) and *N*,*N*-diisopropylethylamine (293 mg, 2.27 mmol) were added, and the mixture was stirred at room temperature overnight. The mixture was added to water (50 mL) and extracted with ethyl acetate (30 mL*3). The organic phase was washed with saturated brine, dried over anhydrous sodium sulfate and concentrated under reduced pressure. The residues were purified by preparative liquid chromatography to give 35.0 mg of the title compound as a pale yellow solid. Yield: 11.4%.

Purification method by preparative liquid chromatography: column: 30 mm × 250 mm; packing: C18, 10 μm; process: 2-22 min, acetonitrile 40-90%; wavelength: 210 nm; flow rate: 45 mL/min; mobile phase: acetonitrile, water.

LC-MS: m/z 407 $[M+H]^+$;

$^1H$ NMR (300 MHz, DMSO-$d_6$): δppm 9.34 (s, 1 H), 8.34 (d, *J* = 5.4 Hz, 1 H), 7.83 (d, *J*= 4.8 Hz, 1 H), 7.51 (s, 1 H),7.21 (s, 1 H), 6.82-6.81 (m, 1 H), 4.91-4.63 (m, 2 H), 3.81 (s, 3 H), 2.95-2.72 (m, 1 H), 2.69-2.62 (m, 3 H), 2.41-2.27 (m, 2 H), 2.23-2.09 (m, 1 H), 2.02-1.92 (m, 1 H), 1.88-1.74 (m, 2 H).

Example 6: Preparation of *N*-(1-methyl-1*H*-pyrazol-4-yl)-4-(8-((3,3,3-trifluoropropyl)sulfonyl)-8-azabicyclo[3.2.1]oct-2-en-3-yl)pyrimidin-2-amine (**6**)

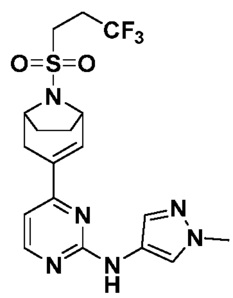

**6**

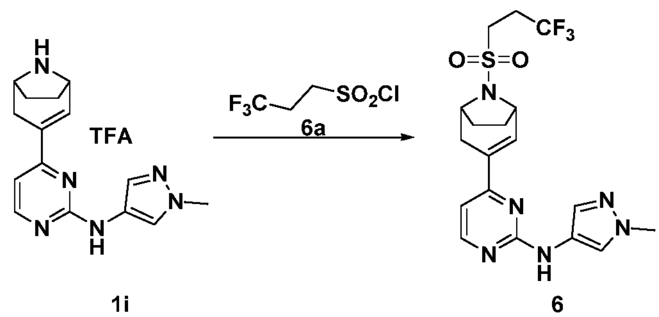


1i 6

3,3,3-Trifluoropropane-1-sulfonyl chloride (119 mg, 0.604 mmol) was added to a solution of 4-(8-azabicyclo[3.2.1]oct-2-en-3-yl)-*N*-(1-methyl-1*H*-pyrazol-4-yl) pyrimidin-2-amine trifluoroacetate (200 mg, 0.526 mmol) in dichloromethane (10 mL) at room temperature. The mixture was stirred at room temperature overnight and concentrated under reduced pressure. The residues were purified by preparative liquid chromatography to give 53.0 mg of the title compound as a yellow solid. Yield: 12.0%.

Purification method by preparative liquid chromatography: column: 30 mm × 250 mm; packing: C18, 10 μm; process: 2-22 min, acetonitrile 20-60%; wavelength: 254 nm; flow rate: 45 mL/min; mobile phase: acetonitrile, water (0.05% formic acid).

LC-MS: m/z 443[M+H]+;

$^1$H NMR (300 MHz, DMSO-$d_6$): δppm 9.41 (s, 1 H), 8.35 (d, *J*= 6 Hz, 1 H), 7.83 (s, 1 H),7.51 (s, 1 H), 7.23-7.17 (m, 1 H), 6.85 (d, *J* = 6 Hz, 1 H), 4.57-4.50 (m, 3 H),3.81 (s, 3 H), 3.49-3.43 (m, 2 H), 3.02-2.96 (m, 1 H), 2.71-2.62 (m, 2 H), 2.19-2.17 (m, 1 H), 2.02-1.98(m, 2 H),1.71-1.67 (m, 1 H).

Example 7: Preparation of (*R*)-1-(3-(2-((1-methyl-1*H*-pyrazol-4-yl)amino) pyrimidin-4-yl)-8-azabicyclo[3.2.1]oct-2-ene-8-carbonyl)pyrrolidine-3-nitrile (**7**)

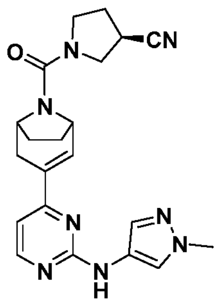

7

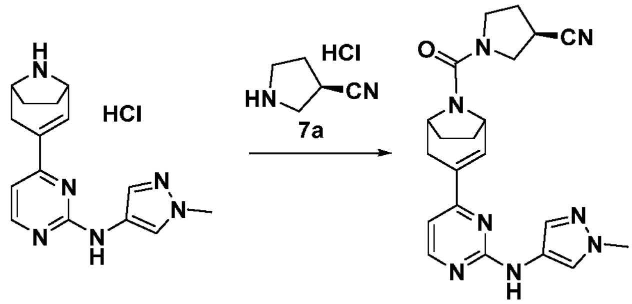


1i 7

Triphosgene (90 mg, 0.302 mmol) was added to a solution of (*R*)-pyrrolidine-3-carbonitrile hydrochloride (100 mg, 0.754 mmol) and pyridine (238 mg, 3.02 mmol) in dichloromethane (10 mL) under nitrogen atmosphere at 0°C, and the mixture was stirred for 2 hours. 4-(8-Azabicyclo[3.2.1]oct-2-en-3-yl)-*N*-(l-methyl-1*H*-pyrazol-4-yl) pyrimidin-2-amine hydrochloride (240 mg, 0.754 mmol) and triethylamine (535 mg, 5.28 mmol) were added at room temperature, and the mixture was stirred at room temperature overnight. The mixture was concentrated under reduced pressure. The residues were purified by preparative liquid chromatography to give 83.0 mg of the title compound as a yellow solid, Yield: 27.2%.

Purification method by preparative liquid chromatography: column: 30 mm × 250 mm; packing: C18, 10 μm; process: 2-22 min, acetonitrile 10-50%; wavelength: 254 nm; flow rate: 45 mL/min; mobile phase: acetonitrile, water.

LC-MS: m/z 405 [M+H]+;

$^1$H NMR (300 MHz, DMSO-$d_6$): δppm 9.30 (s, 1 H), 8.32 (d, *J* = 6 Hz, 1 H), 7.81 (s, 1 H),7.50 (s, 1 H), 7.19-7.17 (m, 1 H), 6.79 (d, *J* = 6 Hz, 1 H),4.49-4.42 (m, 1 H),4.39-4.33 (m, 1 H),3.79 (s, 3 H), 3.69-3.60 (m, 1 H), 3.57-3.51 (m, 1 H), 3.49-3.41 (m, 2 H), 3.38-3.36 (m, 1 H), 3.01-2.95 (m, 1 H), 2.39-2.32 (m, 1 H), 2.23-2.06 (m, 3 H), 1.97-1.85 (m, 2 H), 1.67-1.60 (m, 1 H).

Example 8: Preparation of (3,3-difluoropyrrolidin-1-yl)(3-(2-((1-methyl-1*H*-pyrazol-4-yl)amino)pyrimidin-4-yl)-8-azabicyclo[3.2.1]oct-2-en-8-yl)methanone (8)

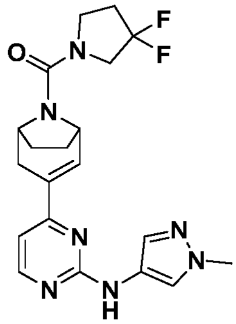

**8**

The same preparation method as that in Example 7 was used to give the title compound **8**, except that 3,3-difluoropyrrolidine hydrochloride was used instead of (*R*)-pyrrolidine-3-carbonitrile hydrochloride.

Purification process by preparative liquid chromatography: column: 30 mm × 250 mm; packing: C18, 10 μm; process: 2-22 min, acetonitrile 20-60%; wavelength: 254 nm; flow rate: 45 mL/min; mobile phase: acetonitrile, water.

LC-MS: m/z 416 $[M+H]^+$;

$^1H$ NMR (300 MHz, DMSO-$d_6$): δ ppm 9.30 (s, 1 H), 8.32 (d, *J*= 6 Hz, 1 H), 7.81 (s, 1 H), 7.50 (s, 1 H), 7.19-7.17 (m, 1 H), 6.79 (d, *J*= 6 Hz, 1 H), 4.48-4.42 (m, 1 H), 4.37-4.36 (m, 1 H), 3.79 (s, 3 H), 3.73-3.69 (m, 1 H), 3.60-3.53 (m, 3 H), 3.00-2.96 (m, 1 H), 2.42-2.28 (m, 3H), 2.08-2.06 (m, 1 H), 1.93-1.91 (m, 2 H), 1.67-1.60 (m, 1 H).

Example 9: Preparation of (3-(2-((1-methyl-1*H*-pyrazol-4-yl)amino)pyrimidin-4-yl) -8-azabicyclo[3.2.1]oct-2-en-8-yl)(3-(trifluoromethyl)pyrrolidin-1-yl)methanone (**9**)

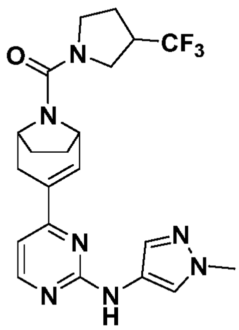

**9**

The same preparation method as that in Example 7 was used to give the title compound **9**, except that 3-(trifluoromethyl)pyrrolidine hydrochloride was used instead of (*R*)-pyrrolidine-3-carbonitrile hydrochloride.

Purification method by preparative liquid chromatography: column: 30 mm × 250 mm; packing: C18, 10 μm; process: 2-22 min, acetonitrile 15-65%; wavelength: 254 nm; flow rate: 45 mL/min; mobile phase: acetonitrile, water.

LC-MS: m/z 448 $[M+H]^+$;

$^1H$ NMR (300 MHz, DMSO-$d_6$): δ ppm 9.30 (s, 1 H), 8.31 (d, *J*= 6 Hz, 1 H), 7.81 (s, 1 H), 7.50 (s, 1 H), 7.22-7.16 (m, 1 H), 6.79 (d, *J*= 6 Hz, 1 H), 4.44-4.36 (m, 2 H), 3.79 (s, 3 H), 3.65-3.39 (m, 4 H), 3.24-3.14 (m, 1 H), 3.02-2.89 (m, 1 H),2.37-2.32 (m, 1H), 2.13-2.06 (m, 2 H), 1.93-1.91 (m, 3 H), 1.66-1.60 (m, 1 H).

Example 10: Preparation of 1-(3-(2-((1-methyl-1*H*-pyrazol-4-yl)amino)pyrimidin-4-yl)-8-azabicyclo[3.2.1]oct-2-ene-8-carbonyl)azetidine-3-nitrile (**10**)

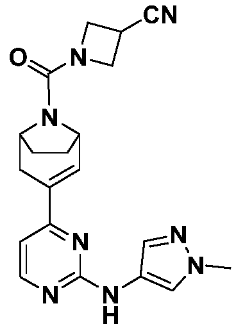

**10**

The same preparation method as that in Example 7 was used to give the title compound **10**, except that azetidine-3-carbonitrile hydrochloride was used instead of (*R*)-pyrrolidine-3-carbonitrile hydrochloride.

Purification method by preparative liquid chromatography: column: 30 mm × 250 mm; packing: C18, 10 μm; process: 2-22-27 min, acetonitrile 10-50-70%; wavelength: 254 nm; flow rate: 45 mL/min; mobile phase: acetonitrile, water.

LC-MS: m/z 391 $[M+H]^+$;

$^1$H NMR (300 MHz, DMSO-$d_6$): $\delta$ppm9.31 (s, 1 H), 8.31 (d, *J*= 6 Hz, 1 H),7.81 (s, 1 H), 7.50 (s, 1 H), 7.18-7.17 (m, 1 H), 6.78 (d, *J* = 6 Hz, 1 H), 4.45-4.39 (m, 1 H), 4.33-4.27 (m, 1 H),4.24-4.16 (m, 2 H), 4.10-4.04 (m, 2 H),3.79 (s, 3 H), 3.76-3.70 (m, 1 H), 2.97-2.92 (m, 1 H), 2.35-2.29 (m, 1 H), 2.11-2.06 (m, 1 H), 1.96-1.85 (m, 2 H), 1.67-1.60 (m, 1 H).

Example 11: Preparation of *N*-(cyanomethyl)-3-(5-fluoro-2-((1-methyl-1*H*-pyrazol-4-yl)amino)pyrimidin-4-yl)-8-azabicyclo[3.2.1]oct-2-ene-8-carboxamide (**11**)

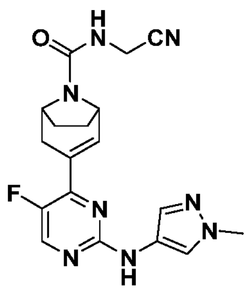

**11**

The same preparation method as that in Examples 1 and 2 was used to give the title compound **11**, except that 2,4-dichloro-5-fluoropyrimidine was used instead of 2,4-dichloropyrimidine (**1e**).

Purification method by preparative liquid chromatography: column: 30 mm × 250 mm; packing: C18, 10 μm; process: 2-22 min, acetonitrile 10-50%; wavelength: 230 nm; flow rate: 45 mL/min; mobile phase: acetonitrile, water.

LC-MS: m/z 383 $[M+H]^+$;

$^1$H NMR (300 MHz, DMSO-$d_6$): $\delta$ppm9.40 (s, 1H), 8.38 (s, 1H), 7.75 (s, 1H), 7.49 (s, 1H), 7.40-7.36 (m, 1H), 7.10-7.09 (m, 1H), 4.59-4.57 (m, 1H), 4.49-4.46 (m, 1H),4.05 (d, *J* = 6.0 Hz, 2H), 3.80 (s, 3H),3.02-2.96 (m, 1H), 2.42-2.33 (m, 1H),2.17-2.09 (m, 1H), 2.02-1.93 (m, 2H), 1.76-1.68 (m, 1H).

Example 12: Preparation of 3-(5-fluoro-2-((1-methyl-1*H*-pyrazol-4-yl)amino) pyrimidin-4-yl)-*N*-(2,2,2-trifluoroethyl)-8-azabicyclo[3.2.1]oct-2-ene-8-carboxamide (**12**)

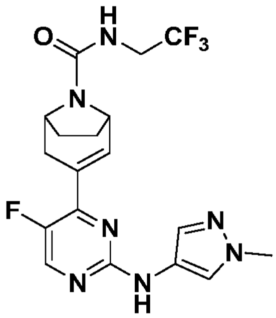

**12**

The same preparation method as that in Examples 1 and 3 was used to give the title compound **12**, except that 2,4-dichloro-5-fluoropyrimidine was used instead of 2,4-dichloropyrimidine (**1e**).

Purification method by preparative liquid chromatography: column: 30 mm × 250 mm; packing: C18, 10 μm; process: 0-2-22 min, acetonitrile 15-15-55%; wavelength: 254 nm; flow rate: 45 mL/min; mobile phase: acetonitrile, water.

LC-MS: m/z 426 [M+H].

$^1$H NMR (300 MHz, DMSO-*d*$_6$) :δppm 9.39 (s, 1H), 8.38 (d, *J*= 4.1 Hz, 1H), 7.75 (s, 1H), 7.49 (s, 1H), 7.29 (t, *J* = 6.2 Hz, 1H), 7.09 (d, *J* = 5.0 Hz, 1H), 4.68-4.60 (m, 1H), 4.57-4.49 (m, 1H), 3.92-3.71 (m, 5H), 3.10-2.97 (m, 1H), 2.35-2.31 (m, 1H), 2.24-2.08 (m, 1H), 2.02-1.87 (m, 2H), 1.78-1.63 (m, 1H).

Examples 12-a and 12-b: Preparation of compounds **12-a** and **12-b**

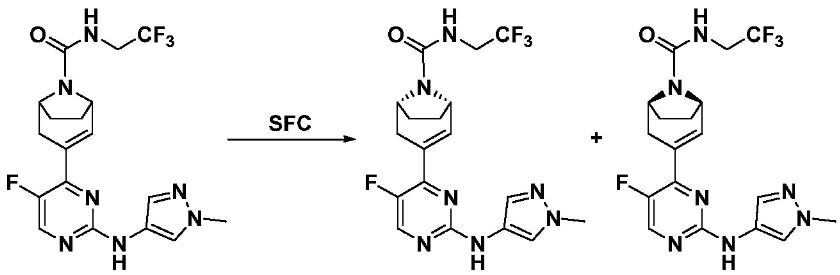


**12** **12-a** and **12-b**

Compounds **12-a** and **12-b** were obtained by separation from compound **12** by SFC.

SFC separation conditions:

Column: AS-H 4.6 mm × 250 mm, 5 μm, mobile phase: MeOH (0.2% $NH_3 \cdot H_2O$)/$CO_2$ = 35:65, flow rate: 40 g/min.
**12-a:** retention time: 2.97 min;

LC-MS: m/z 426 [M+H]

$^1$H NMR (400 MHz, DMSO-*d*$_6$) :δppm 9.40 (s, 1H), 8.38 (d, *J*= 4.4 Hz, 1H),7.75 (s, 1H), 7.50 (s, 1H), 7.28 (t, *J* = 8.4 Hz, 1H), 7.10-7.09 (m, 1H),4.63-4.52 (m, 2H),3.83-3.80 (m, 2H),3.77 (s, 3H), 3.04-3.00 (m, 1H), 2.35-2.31 (m, 1H), 2.24-2.08 (m, 1H), 2.02-1.87 (m, 2H), 1.78-1.63 (m, 1H).

**12-b:** retention time: 5.25 min.

LC-MS: m/z 426 [M+H]

$^1$H NMR (400 MHz, DMSO-*d*$_6$) :δppm9.40 (s, 1H), 8.38 (d, *J* = 4.4 Hz, 1H),7.75 (s, 1H), 7.50 (s, 1H), 7.28 (t, *J* = 8.4 Hz, 1H), 7.10-7.09 (m, 1H), 4.62-4.53 (m, 2H),3.83-3.80 (m, 2H), 3.77 (s, 3H), 3.04-3.00 (m, 1H), 2.35-2.31 (m, 1H), 2.17-2.14 (m, 1H), 2.02-1.87 (m, 2H), 1.74-1.67 (m, 1H).

Example 13: Preparation of (3-(2-((1-methyl-1*H*-pyrazol-4-yl)amino)pyrimidin-4-yl)-8-azabicyclo[3.2.1]oct-2-en-8-yl)(3-methyloxetan-3-yl)methanone (**13**)

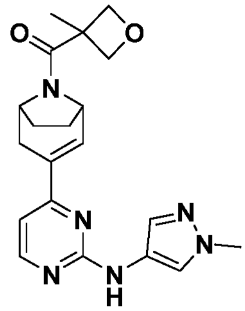

**13**

The same preparation method as that in Example 1 was used to give the title compound **13**, except that 3-methyloxetane-3-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-2-22 min, acetonitrile 10-10-50%; wavelength: 220 nm; flow rate: 45 mL/min; mobile phase: acetonitrile, water.
LC-MS: m/z 381 [M+H].
$^1$H NMR (300 MHz, DMSO-$d_6$): δppm9.34 (s, 1H), 8.34 (d, *J* = 5.2 Hz, 1H), 7.81 (s, 1H), 7.50 (s, 1H), 7.16 (s, 1H), 6.81 (d, *J* = 5.2 Hz, 1H), 5.00-4.65 (m, 3H), 4.41-4.25 (m, 2H), 3.96-3.85 (m, 1H), 3.80 (s, 3H), 2.92-2.89 (m, 1H), 2.42-2.40 (m, 1H), 2.24-1.85 (m, 3H), 1.70-1.64 (m, 1H), 1.53 (s, 1H), 1.48 (s, 2H).

Example 14: Preparation of *N*(2,2-difluorocyclopropyl)-3-(2-((1-methyl-1*H*-pyrazol-4-yl)amino)pyrimidin-4-yl)-8-azabicyclo[3.2.1]oct-2-ene-8-carboxamide (**14**)

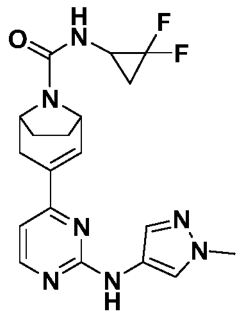

**14**

The same preparation method as that in Example 2 was used to give the title compound **14**, except that 2,2-difluorocyclopropane-1-amine hydrochloride was used instead of 2-aminoacetonitrile (**2a**).
Purification method by preparative liquid chromatography: column: 30 mm × 250 mm; packing: C18, 10 μm; process: 0-22 min, acetonitrile 20-70%; wavelength: 254 nm; flow rate: 45 mL/min; mobile phase: acetonitrile, water (an aqueous solution of 0.05% formic acid).
LC-MS: m/z=402[M+H].
$^1$H NMR (300 MHz, DMSO-$d_6$): δppm 9.31 (s, 1 H),8.33 (d, *J*= 5.2 Hz, 1 H), 7.81 (s, 1 H), 7.52 (s, 1 H), 8.33 (d, *J*= 4.1 Hz, 1 H), 6.99 (s, 1 H), 6.82 (d, *J*= 5.2 Hz, 1 H), 4.57-4.48 (m, 2 H), 3.80 (s, 3 H), 3.14-3.11 (m, 1 H),2.98-2.92 (m, 1 H), 2.51-2.49 (m, 1 H), 2.32-2.42 (m, 1 H),2.13-2.07 (m, 2 H), 1.79-1.76 (m, 1 H), 1.67-1.62 (m, 1 H), 1.47-1.44 (m, 1 H).

Example 15: Preparation of 3-(2-((1-methyl-1*H*-pyrazol-4-yl)amino)pyrimidin-4-yl)-*N*-(oxetan-3-yl)-8-azabicyclo[3.2.1]oct-2-ene-8-carboxamide (**15**)

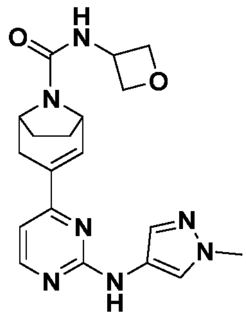

15

The same preparation method as that in Example 2 was used to give the title compound **15**, except that oxetane-3-amine hydrochloride was used instead of 2-aminoacetonitrile (**2a**).

Purification method by preparative liquid chromatography: column: 30 mm × 250 mm; packing: C18, 10 μm; process: 0-2-22 min, acetonitrile 10-10-40%; wavelength: 220 nm; flow rate: 45 mL/min; mobile phase: acetonitrile, water.

LC-MS: m/z 382 [M+H].

$^1$H NMR (300 MHz, DMSO-$d_6$) : δppm9.31 (s, 1H), 8.32 (d, *J*= 5.2 Hz, 1H), 7.81 (s, 1H), 7.52 (s, 1H), 7.31-7.11 (m, 2H), 6.80 (d, *J* = 5.2 Hz, 1H), 4.76-4.56 (m, 4H), 4.55-4.47 (m, 1H), 4.46-4.39 (m, 2H), 3.81 (s, 3H), 3.03-2.89 (m, 1H), 2.35-2.22 (m, 1H), 2.20-2.04 (m, 1H), 1.99-1.86 (m, 2H), 1.72-1.56 (m, 1H).

Example 16: Preparation of (3-(2-((1-methyl-1*H*-pyrazol-4-yl)amino)pyrimidin-4-yl)-8-azabicyclo[3.2.1]oct-2-en-8-yl)(4-(trifluoromethyl)phenyl)methanone (**16**)

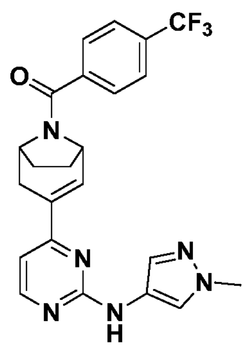

16

The same preparation method as that in Example 1 was used to give the title compound **16**, except that 4-(trifluoromethyl)benzoic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-2-22 min, acetonitrile 40% isocratic; wavelength: 220 nm; flow rate: 45 mL/min; mobile phase: acetonitrile, water.

LC-MS: m/z 455 [M+H].

$^1$H NMR (300 MHz, DMSO-$d_6$):δppm9.35 (s, 1H), 8.35(d, *J* = 5.1 Hz, 1H), 7.83 (s, 3H), 7.76-7.73 (m,1H), 7.66-7.63 (m, 1H), 7.50 (s, 1H), 7.28-7.13 (m,1H), 6.84 (d,*J* = 5.4 Hz 1H), 5.05-4.93 (m, 1H), 4.38-4.24 (m, 1H), 3.80(s, 3H), 3.16-3.10 (m, 1H), 2.87-2.82 (m, 1H), 2.21-2.03 (m, 3H), 1.73 (m, 1H).

Example 17: Preparation of (3-(2-((1-methyl-1*H*-pyrazol-4-yl)amino)pyrimidin-4-yl)-8-azabicyclo[3.2.1]oct-2-en-8-yl)(5-(trifluoromethyl)pyridin-2-yl)methanone (**17**)

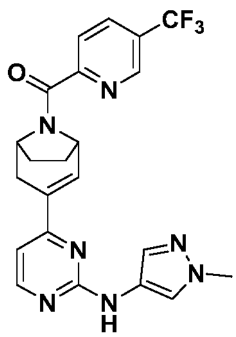

17

The same preparation method as that in Example 1 was used to give the title compound **17**, except that 5-(trifluoromethyl)picolinic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-2-22 min, acetonitrile 40% isocratic; wavelength: 220 nm; flow rate: 45 mL/min; mobile phase: acetonitrile, water.

LC-MS: m/z 456 [M+H].

$^1H$ NMR (300 MHz, DMSO-$d_6$): δppm 9.34 (s, 1H), 9.05 (s, 1H), 8.39-8.35 (m, 2H), 7.96-7.89(m,1H), 7.83 (s, 1H), 7.50 (s, 1H), 7.28-7.14 (m,1H), 6.84-6.82 (m,1H), 5.11-4.73 (m, 2H), 3.80(s, 3H),3.13-3.03(m, 1H), 2.57-2.47 (m, 1H), 2.21-2.03 (m, 3H), 1.77 (m, 1H).

Example 18: Preparation of 5-(3-(2-((1-methyl-1*H*-pyrazol-4-yl)amino)pyrimidin-4-yl)-8-azabicyclo[3.2.1]oct-2-ene-8-formyl)thiophene-3-carbonitrile (**18**)

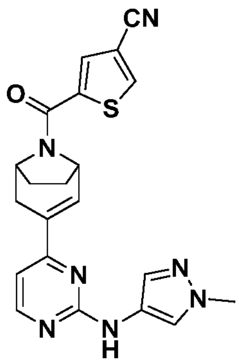

18

The same preparation method as that in Example 1 was used to give the title compound **18**, except that 4-cyanothiophene-2-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-2-22 min, acetonitrile 35% isocratic; wavelength: 220 nm; flow rate: 45 mL/min; mobile phase: acetonitrile, water.

LC-MS: m/z 418 [M+H].

$^1H$ NMR (300 MHz, DMSO-$d_6$): δppm 9.34 (s, 1H), 8.74 (s, 1H), 8.36 (d, *J* = 5.1 Hz, 1H), 8.03 (s, 1H), 7.84 (s, 1H), 7.50 (s, 1H),7.28-7.24 (m,1H),6.84 (d, *J* = 5.1 Hz 1H),5.04-5.01 (m,1H), 4.90-4.85 (m, 1H), 3.81(s, 3H),3.11-3.06(m, 1H), 2.57-2.51 (m, 1H), 2.24-2.05 (m, 3H), 1.77-1.73 (m, 1H).

Example 19: Preparation of 3-(5-methyl-2-((1-methyl-1*H*-pyrazol-4-yl)amino) pyrimidin-4-yl)-*N*-(2,2,2-trifluoroethyl)-8-azabicyclo[3.2.1]oct-2-ene-8-carboxamide (**19**)

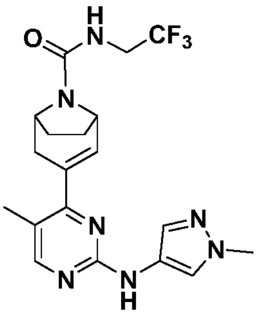

**19**

The same preparation method as that in Examples 1 and 2 was used to give the title compound **19**, except that 2,2,2-trifluoroethylamine hydrochloride was used instead of 2-aminoacetonitrile (**2a**) and 2,4-dichloro-5-methylpyrimidine was used instead of 2,4-dichloropyrimidine (**1e**).

Purification method by preparative liquid chromatography: column: 30 mm × 250 mm; packing: C18, 10 μm; process: 0-2-17 min, acetonitrile 5-5-50%; wavelength: 220 nm; flow rate: 45 mL/min; mobile phase: acetonitrile, 0.05% formic acid in water.

LC-MS: m/z 422 [M+H].

$^1$H NMR (300 MHz, DMSO-$d_6$): $\delta$ppm9.20 (s, 1H), 8.21 (s, 1H), 7.76 (s, 1H), 7.45 (s, 1H), 7.28 (t, $J$ = 6.2 Hz, 1H), 6.31 (d, $J$ = 4.5 Hz, 1H), 4.59-4.43 (m, 2H), 3.92-3.80(m, 2H), 3.78 (s, 3H),3.00-2.84 (m, 1H), 2.26-2.15 (m, 2H), 2.09 (s, 3H), 2.03-1.89 (m, 2H), 1.79-1.66 (m, 1H).

Examples 19-a and 19-b: Preparation of compounds **19-a** and **19-b**

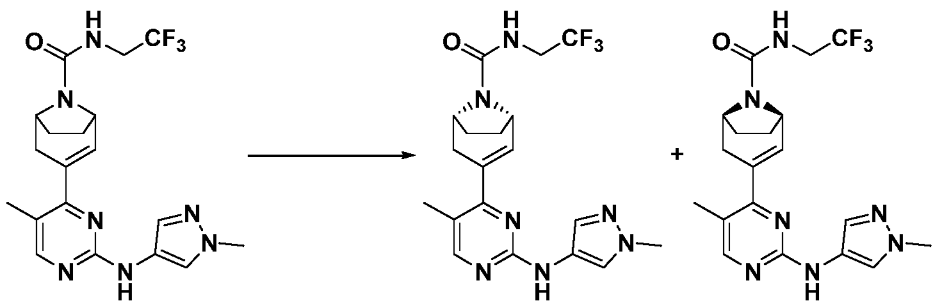

**19** **19-a** and **19-b**

Compounds **19-a** and **19-b** were obtained from compound **19** by chiral separation.

Method for chiral column separation:

Column: IA 4.6 mm × 250 mm, 5 μm, mobile phase: IPA (0.2% $NH_3 \cdot H_2O$)/ hexane = 30:70, flow rate: 14 mL/min.

**19-a:** retention time: 9.89 min;

LC-MS: m/z 422 [M+H].

$^1$H NMR (300 MHz, DMSO-$d_6$):$\delta$ppm9.20 (s, 1H), 8.21 (s, 1H), 7.76 (s, 1H), 7.44 (s, 1H), 7.27 (t, $J$= 12.4 Hz, 1H), 6.30 (d, $J$ = 4.4 Hz, 1H), 4.59-4.50(m, 1H),4.49-4.42 (m, 1H), 3.85-3.80(m, 2H), 3.77 (s, 3H),2.99-2.84 (m, 1H), 2.22-2.15 (m, 2H), 2.10 (s, 3H), 1.95-1.80 (m, 2H), 1.75-1.70 (m, 1H).

**19-b:** retention time: 17.84 min.

LC-MS: m/z 422 [M+H]

$^1$H NMR (400 MHz, DMSO-$d_6$):$\delta$ppm9.20 (s, 1H), 8.21 (s, 1H), 7.76 (s, 1H), 7.44 (s, 1H), 7.27 (t, $J$ = 12.4 Hz, 1H), 6.30 (d, $J$ = 4.8 Hz, 1H), 4.56-4.52(m, 1H),4.48-4.44 (m, 1H), 3.84-3.80(m, 2H), 3.77 (s, 3H),2.95-2.89 (m, 1H), 2.21-2.16 (m, 2H), 2.10 (s, 3H), 1.96-1.81 (m, 2H), 1.76-1.71 (m, 1H).

Example 20: Preparation of 3-(5-chloro-2-((1-methyl-1*H*-pyrazol-4-yl)amino) pyrimidin-4-yl)-*N*-(2,2,2-trifluoroethyl)-8-azabicyclo[3.2.1]oct-2-ene-8-carboxamide (**20**)

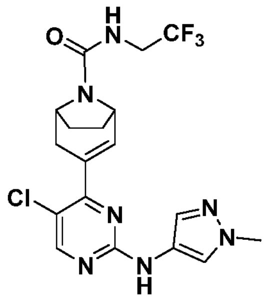

**20**

The same preparation method as that in Examples 1 and 3 was used to give the title compound **20**, except that 2,4,5-trichloropyrimidine was used instead of 2,4-dichloropyrimidine (**1e**).

Purification method by preparative liquid chromatography: column: 30 mm × 250 mm; packing: C18, 10 μm; process: 0-2-22 min, acetonitrile 37% isocratic; wavelength: 220 nm; flow rate: 45 mL/min; mobile phase: acetonitrile, water.

LC-MS: m/z 442 [M+H].

$^1$H NMR (300 MHz, DMSO-$d_6$): δppm9.65 (s, 1H), 8.40(s, 1H), 7.76 (s, 1H), 7.47 (s, 1H), 7.27(s, 1H), 6.70 (s, 1H), 5.60-4.50 (m, 2H), 3.87-3.80 (m, 5H), 3.05-2.99 (m, 1H), 2.23-2.19 (m, 2H), 2.01-1.92 (m, 2H), 1.77-1.71(m, 1H).

Example 21: Preparation of ((*S*)-2,2-difluorocyclopropyl)(3-(5-fluoro-2-((1-methyl-1*H*-pyrazol-4-yl)amino)pyrimidin-4-yl)-8-azabicyclo[3.2.1]oct-2-en-8-yl)me thanone (**21**)

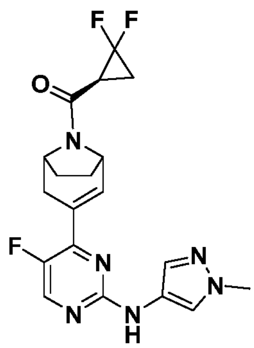

**21**

The same preparation method as that in Example 1 was used to give the title compound **21**, except that 2,4-dichloro-5-fluoropyrimidine was used instead of 2,4-dichloropyrimidine (**1e**).

Purification method by preparative liquid chromatography: column: 30 mm × 250 mm; packing: C18, 10 μm; process: 0-2-22min, acetonitrile 15-15-55%; wavelength: 220 nm; flow rate: 45 mL/min; mobile phase: acetonitrile, water.

LC-MS: m/z 405 [M+H].

$^1$H NMR (300 MHz, DMSO-$d_6$): δppm9.51 (s, 1H), 8.48-8.46 (m, 1H), 7.84-7.82 (m, 1H), 7.55-7.53 (m, 1H), 7.20-7.12 (m, 1H), 4.98-4.90 (m, 2H), 3.87 (s, 3H), 3.28-3.02 (m, 2H), 2.70-2.51 (m, 1H), 2.23-2.16 (m, 2H), 2.06-1.88 (m, 4H).

Example 22: Preparation of ((*S*)-2,2-difluorocyclopropyl)(3-(5-methyl-2-((1-methyl-1*H*-pyrazol-4-yl)amino)pyrimidin-4-yl)-8-azabicyclo[3.2.1]oct-2-en-8-yl) methanone (**22**)

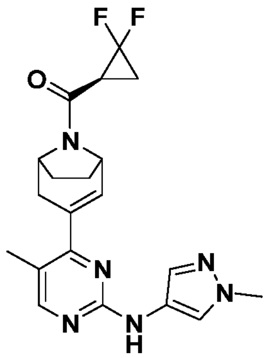

22

The same preparation method as that in Example 1 was used to give the title compound **22**, except that 2,4-dichloro-5-methylpyrimidine was used instead of 2,4-dichloropyrimidine (**1e**).

Purification method by preparative liquid chromatography: column: 30 mm × 250 mm; packing: C18, 10 μm; process: 0-2-22min, acetonitrile 15-15-55%; wavelength: 220 nm; flow rate: 45 mL/min; mobile phase: acetonitrile, water.

LC-MS: m/z 401 [M+H].

$^1$H NMR (300 MHz, DMSO-$d_6$): δppm9.23 (d, *J* = 4.2 Hz, 1H), 8.23 (d, *J* = 3.7 Hz, 1H), 7.77 (d, *J* = 4.8 Hz, 1H), 7.43 (d, *J* = 7.2 Hz, 1H), 6.44-6.27 (m, 1H), 4.88-4.61 (m, 2H), 3.77 (s, 3H), 3.25-2.83 (m, 2H), 2.47-2.21 (m, 2H), 2.21-2.05 (m, 4H), 2.03-1.71 (m, 4H).

Examples 22-a and 22-b: Preparation of compounds **22-a** and **22-b**

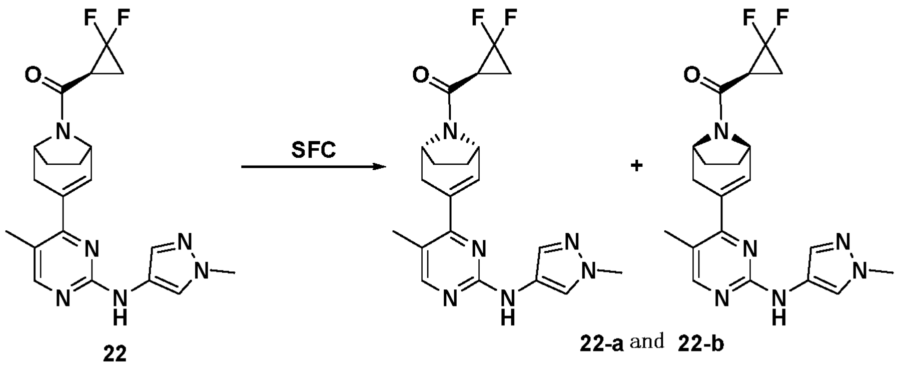


22

22-a and 22-b

Compounds **22-a** and **22-b** were obtained by separation from compound **22** by SFC.

SFC separation conditions:

Column: AS-H 4.6 mm × 250 mm, 5 μm, mobile phase: EtOH (0.2% $NH_3H_2O$)/$CO_2$ = 35:65, flow rate: 40 g/min.

**22-a:** retention time: 2.67 min;

LC-MS: m/z 401 $[M+H]^+$.

$^1$H NMR (400 MHz, CDC13): δppm8.20-8.18 (m, 1H), 7.76-7.71 (m, 1H), 7.46 (s, 1H), 7.00-6.78 (m, 1H), 6.28 (d, *J* = 5.2Hz, 1H), 5.01-4.98 (m, 1H), 4.61-4.56 (m, 1H), 3.89 (s, 3H), 3.11-3.01 (m, 1H), 2.59-2.51 (m, 2H), 2.42-2.31 (m, 1H), 2.27-2.21 (m, 1H), 2.17-2.01 (m, 5H),1.96-1.84 (m, 1H), 1.72-1.64 (m, 1H).

**22-b:** retention time: 3.28 min.

LC-MS: m/z 401 $[M+H]^+$.

$^1$H NMR (400 MHz, CDC13): δppm8.20-8.19 (m, 1H), 7.76-7.71 (m, 1H), 7.48 (s, 1H), 7.00-6.84 (m, 1H), 6.35-6.30 (m,1H), 5.01-4.86 (m, 1H), 4.62-4.57 (m, 1H), 3.89 (s, 3H), 3.07-3.02 (m, 1H), 2.59-2.55 (m, 2H), 2.42-2.31 (m, 1H), 2.27-2.21 (m, 1H), 2.17-2.01 (m, 5H), 1.95-1.89 (m, 1H), 1.73-1.63 (m, 1H).

Example 23: Preparation of (3-(5-chloro-2-((1-methyl-1*H*-pyrazol-4-yl)amino) pyrimidin-4-yl)-8-azabicyclo[3.2.1]oct-2-en-8-yl)((*S*)-2,2-difluorocyclopropyl) methanone (**23**)

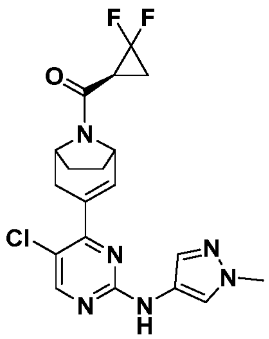

23

The same preparation method as that in Example 1 was used to give the title compound **23**, except that 2,4,5-trichloropyrimidine was used instead of 2,4-dichloropyrimidine (**1e**).

Purification method by preparative liquid chromatography: column: 30 mm × 250 mm; packing: C18, 10 μm; process: 0-2-22 min, acetonitrile 20-20-60%; wavelength: 220 nm; flow rate: 45 mL/min; mobile phase: acetonitrile, water.

LC-MS: m/z 421 [M+H].

$^1$H NMR (300 MHz, DMSO-*d6*):δppm9.68 (d, *J* = 3.3 Hz, 1H), 8.42 (d, *J* = 3.0 Hz, 1H), 7.77 (s, 1H), 7.43 (s, 1H), 6.70 (s, 1H), 4.86-4.69 (m, 2H), 3.80-3.78(m, 3H), 3.23-2.94 (m, 2H), 2.50-2.46 (m, 1H), 2.40-2.29 (m,1H), 2.18-2.07 (m, 1H),2.02-1.88 (m, 4H).

Examples 23-a and 23-b: Preparation of compounds **23-a** and **23-b**

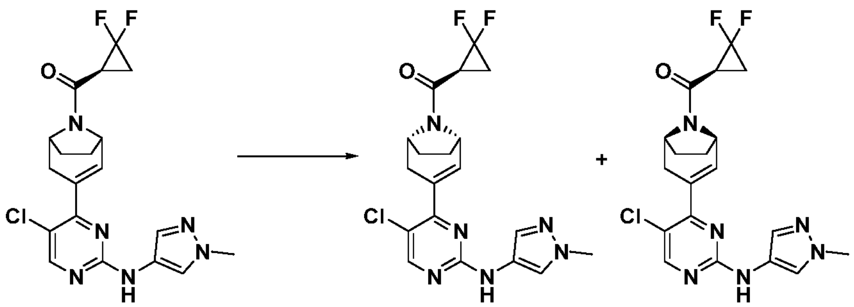

23 — **23-a** and **23-b**

Compounds **23-a** and **23-b** were obtained by separation from compound **23** by SFC.

SFC separation conditions:

Column: AS-H 4.6 mm × 250 mm, 5 μm, mobile phase: EtOH (0.2% $NH_3H_2O$)/$CO_2$ = 35:65, flow rate: 40 g/min.

**23-a:** retention time: 3.67 min;

LC-MS: m/z 421 $[M+H]^+$.

$^1$H NMR (400 MHz, $CDCl_3$): δppm8.31-8.29 (m, 1H), 7.77-7.72 (m, 1H), 7.51-7.45 (m, 1H), 7.03-6.93 (m, 1H), 6.82-6.78 (m, 1H), 5.03-4.97 (m, 1H), 4.63-4.57 (m, 1H), 3.89 (s, 3H), 3.22-3.09 (m, 1H), 2.41-2.24 (m, 3H), 2.18-2.15 (m, 3H), 1.70-1.64 (m, 2H).

**23-b:** retention time: 4.53 min.

LC-MS: m/z 421 $[M+H]^+$.

$^1$H NMR (400 MHz, CDC13): δppm8.31-8.29 (m, 1H), 7.77-7.69 (m, 1H), 7.50-7.45 (m, 1H), 7.00-6.86 (m, 1H), 6.76-6.75 (m, 1H), 5.12-4.87 (m, 1H), 4.63-4.57 (m, 1H), 3.89 (s, 3H), 3.20-3.07 (m, 1H), 2.55-2.52 (m, 3H), 2.25-2.17 (m, 3H), 1.71-1.62 (m, 2H).

Example 24: Preparation of 3-(2-((1-(difluoromethyl)-1*H*-pyrazol-4-yl)amino)-5-fluoropyrimidin-4-yl)-*N*-(2,2,2-trifluoroethyl)-8-azabicyclo[3.2.1]oct-2-ene-8-carboxamide (**24**)

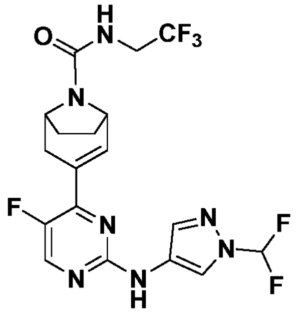

**24**

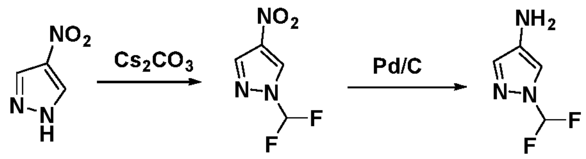


**24a** **24b** **24c**

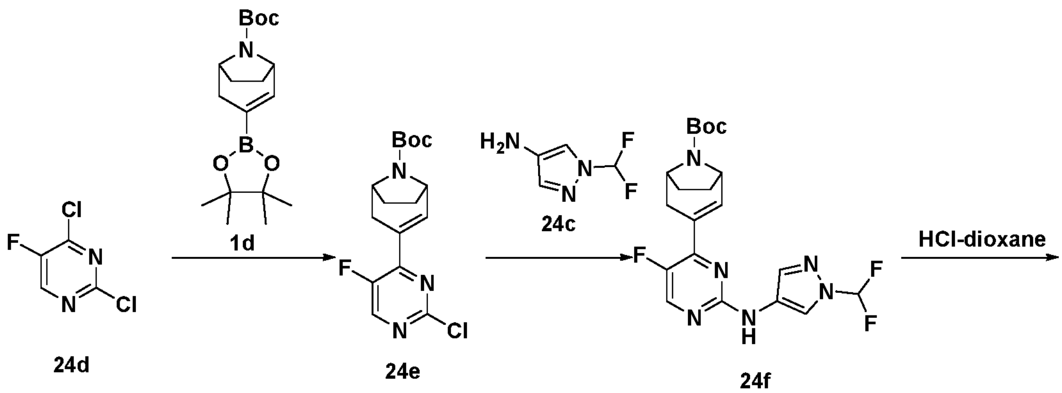


**24d** **24e** **24f**

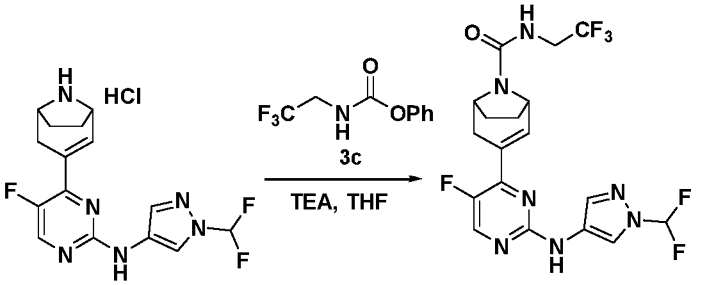


**24g** **24**

**Step 1:** Synthesis of 1-(difluoromethyl)-4-nitro-1*H*-pyrazole (**24b**)

Sodium difluorochloroacetate (13.5 g, 88.4 mmol) was added in portions to a mixture of 4-nitro-1*H*-pyrazole (5.00 g, 44.2 mmol) and cesium carbonate (14.4 g, 44.2 mmol) in *N,N*-dimethylformamide (40 mL) at room temperature. The mixture was stirred and heated in an oil bath at 120°C for 30 minutes. The reaction solution was cooled, diluted with ethyl acetate (300 mL) and washed with water (200 mL*3). The organic phase was washed with saturated brine (100 mL). The organic phase was dried over anhydrous sodium sulfate and concentrated under reduced pressure. The residues were separated and purified by silica gel column chromatography (mobile phase: PE/EA=100:1-10:1) to give 5.7 g of the title compound as a colorless oil. Yield: 79.2%.

LC-MS: m/z 164 $[M+H]^+$.

**Step 2:** Synthesis of 1-(difluoromethyl)-1*H*-pyrazol-4-amine (**24c**)

Palladium on carbon (2.00 g) was added to a solution of 1-(difluoromethyl)-4-nitro-1*H*-pyrazole (5.7 g, 34.9

mmol) in methanol (70 mL) under nitrogen atmosphere at room temperature. The mixture was purged with hydrogen for three times and stirred at room temperature overnight.

The reaction solution was suction filtered, and the filtrate was concentrated under reduced pressure to give 5.5 g of the crude title compound as a yellow oil.

LC-MS: m/z 133 $[M+H]^+$.

Step 3 to Step 6: The same preparation method as that in Examples 1 and 3 was used to give the title compound **24**, except that 1-(difluoromethyl)-1*H*-pyrazole-4-amine was used instead of 1-methyl-1*H*-pyrazole-4-amine (**1g**) and 2,4-dichloro-5-fluoropyrimidine was used instead of 2,4-dichloropyrimidine (**1e**).

Purification method by preparative liquid chromatography: column: 30 mm × 250 mm; packing: C18, 10 μm; process: 0-2-22 min, acetonitrile 10-10-50%; wavelength: 220 nm; flow rate: 45 mL/min; mobile phase: acetonitrile, water.

LC-MS: m/z 462 $[M+H]^+$.

$^1$H NMR (400 MHz, DMSO-$d_6$) δppm 9.72 (s, 1H), 8.44 (d, *J* = 4.1 Hz, 1H), 8.19 (s, 1H), 7.91-7.58 (m, 2H), 7.34-7.25 (m, 1H), 7.13-7.06 (m, 1H), 4.67-4.56 (m, 1H), 4.55-4.44 (m, 1H), 3.88-3.69 (m, 2H), 3.05-2.93 (m, 1H), 2.36-2.28 (m, 1H), 2.21-2.05 (m, 1H), 2.00-1.88 (m, 2H), 1.72-1.67 (m, 1H).

Example 25: Preparation of 3-(2-((1-methyl-1*H*-pyrazol-4-yl)amino)-5-trifluoromethyl)pyrimidin-4-yl)-*N*-(2,2,2-trifluoroethyl)-8-azabicyclo[3.2.1]oct-2-ene-8-carboxamide (**25**)

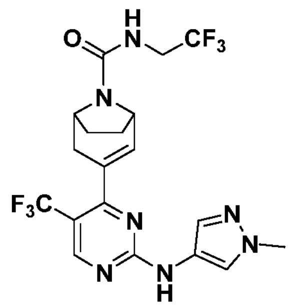

**25**

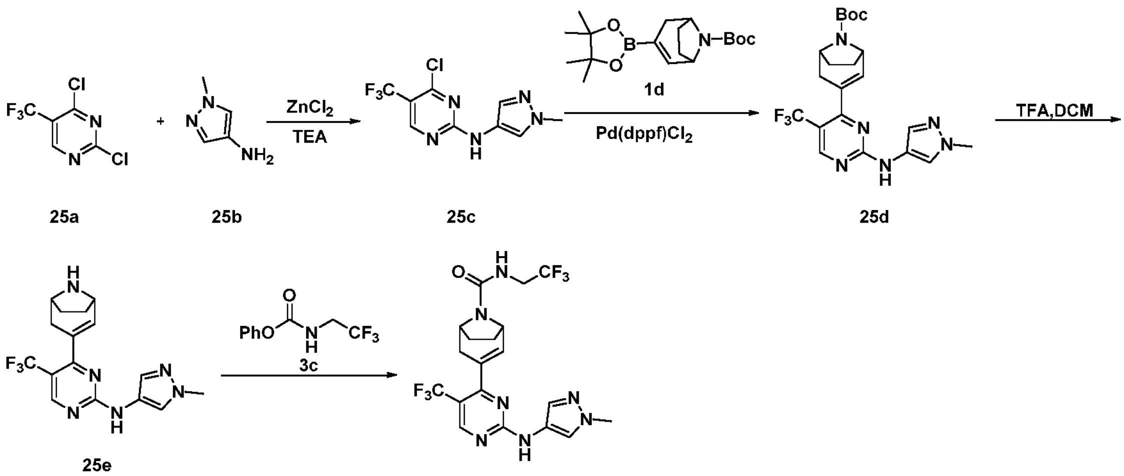


25a 25b 25c 25d

25e

25

**Step 1:** Synthesis of 4-chloro-*N*-(1-methyl-1*H*-pyrazol-4-yl)-5-trifluoromethyl) pyrimidin-2-amine and 2-chloro-*N*-(1-methyl-1*H*-pyrazol-4-yl)-5-(trifluoromethyl) pyrimidin-4-amine (**25c**).

A solution of zinc chloride in tetrahydrofuran (24.7 mL, 24.7 mmol) was added dropwise to a solution of 2,4-dichloro-5-(trifluoromethyl)pyrimidine (4.70 g, 21.6 mmol) in dichloroethane (50 mL) and tert-butanol (50 mL) at 0°C and the mixture was stirred for 10 minutes. A solution of 1-methyl-1*H*-pyrazol-4-amine (2.00 g, 20.6 mmol) and triethylamine (5.00 g, 49.44 mmol) in dichloroethane (25 mL) and tert-butanol (25 mL) was added dropwise. The mixture was naturally warmed to room temperature and stirred overnight. The reaction solution was added to water (200 mL) and extracted

with dichloroethane (100 mL*3). The organic phases were dried and concentrated under reduced pressure. The residues were purified by flash column chromatography (mobile phase: petroleum ether/ethyl acetate = 100/1 to 5/1) to give 1.20 g of the title compound as a brown oil. Yield: 20.0%.

LC-MS: m/z 278 $[M+H]^+$.

**Step 2:** The same preparation method as that in Example If was used to give the title compound **25d**, except that 1-methyl-1*H*-pyrazole-4-amine 4-chloro-*N*-(1-methyl-1*H*-pyrazol-4-yl)-5-(trifluoromethyl)pyrimidin-2-amine was used instead of 2,4-dichloropyrimidine (**1e**).

**Step 3:** The same preparation method as that in Example 1i was used to give the title compound **25e**, except that tert-butyl-3-(2-((1-methyl-1*H*-pyrazol-4-yl)amino)-5-(trifluoromethyl)pyrimidin-4-yl)-8-azabicyclo[3.2.1]oct-2-ene-8-carboxylate was used instead of tert-butyl 3-(2-((1-methyl-1*H*-pyrazol-4-yl)amino)pyrimidin-4-yl)-8-azabicyclo[3.2.1]oct-2-ene-8-carboxylate (**1h**).

**Step 4:** The same preparation method as that in Example 3 was used to give the title compound **25**, except that 4-(8-azabicyclo[3.2.1]oct-2-en-3-yl)-*N*-(1-methyl-1*H*-pyrazol-4-yl)-5-(trifluoromethyl)pyrimidin-2-amine was used instead of 4-(8-azabicyclo [3.2.1]oct-2-en-3-yl)-*N*-(1-methyl-1*H*-pyrazol-4-yl)pyrimidin-2-amine.

Purification method by preparative liquid chromatography: column: 30 mm × 250 mm; packing: C18, 10 μm; process: 0-2-22 min, acetonitrile 40-40-50%; wavelength: 220 nm; flow rate: 45 mL/min; mobile phase: acetonitrile, 0.05% formic acid in water.

LC-MS: m/z 476 $[M+H]^+$.

$^1H$ NMR (300 MHz, DMSO-$d_6$): δppm9.06 (s, 1H), 8.54(s, 1H), 7.93 (s, 1H), 7.76 (s, 1H), 7.47(d,*J* = 6.0 Hz 1H), 7.29-7.24 (m, 1H), 4.63-4.52 (m,2H), 3.85-3.75 (m, 5H), 3.01-2.95 (m,1H),2.38-2.32 (m, 1H), 2.16-1.93 (m, 3H), 1.70-1.23(m, 1H).

Example 26: Preparation of 3-(2-((1,3-dimethyl-1*H*-pyrazol-4-yl)amino)-5-methylpyrimidin-4-yl)-*N*-(2,2,2-trifluoroethyl)-8-azabicyclo[3.2.1]oct-2-ene-8-carboxamide (**26**)

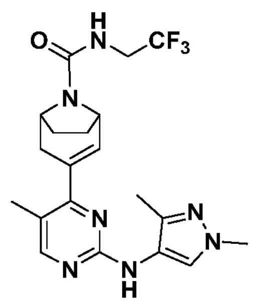

**26**

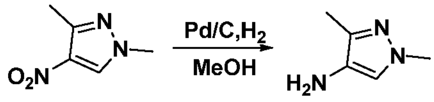


26a 26b

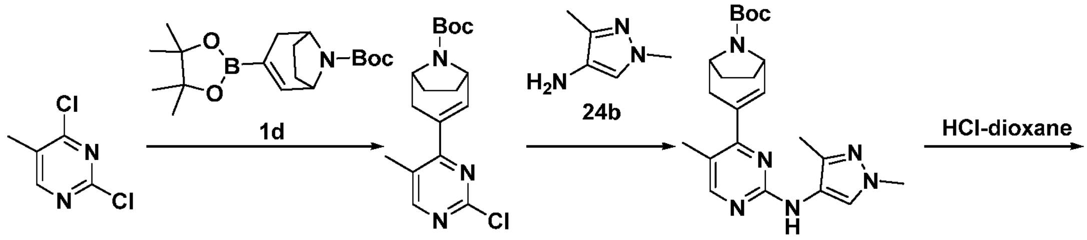


26c 26d 26e

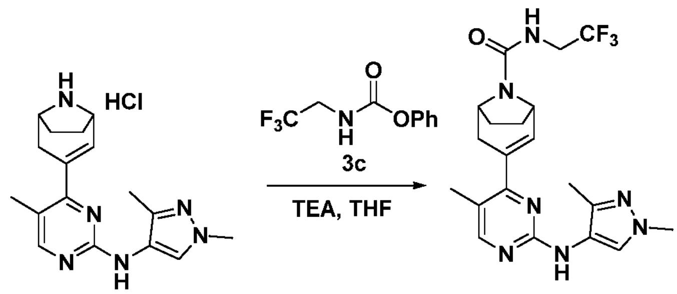


26f 26

**Step 1:** Synthesis of 1,3-dimethyl-1*H*-pyrazol-4-amine (**26b**)

Pd/C (100 mg, 10%) was added to a solution of 1,3-dimethyl-4-nitro-1*H*-pyrazole (1.00 g, 7.08 mmol) in methanol (100 mL) under nitrogen atmosphere at room temperature. The mixture was purged with hydrogen three times and stirred at room temperature overnight. The reaction solution was filtered through Celite and rinsed with methanol. The filtrate was concentrated under reduced pressure and rotary evaporated to dry to give 700 mg of the title compound as a brown oil, yield: 88.9%.

Synthesis steps 2 to 5: The same preparation method as that in Examples 1 and 3 was used to give the title compound **26**, except that 2,4-dichloro-5-methylpyrimidine was used instead of 2,4-dichloropyrimidine and 1,3-dimethyl-1*H*-pyrazol-4-amine was used instead of 1-methyl-1*H*-pyrazol-4-amine (**1g**).

Purification method by preparative liquid chromatography: column: 30 mm $\times$ 250 mm; packing: C18, 10 $\mu$m; process: 0-2-22 min, acetonitrile 10-10-50%; wavelength: 220 nm; flow rate: 45 mL/min; mobile phase: acetonitrile, water.

LC-MS: m/z 436 $[M+H]^+$.

$^1$H NMR (400 MHz, $CDCl_3$): $\delta$ppm8.17 (s, 1H), 7.74 (s, 1H), 6.41 (s, 1H), 6.29-6.28 (m, 1H), 4.85-4.81 (m, 1H), 4.50-4.43 (m, 2H),4.10-3.92 (m, 2H), 3.83 (s, 3H), 3.06-3.00 (m, 1H), 2.38-2.31 (m, 2H), 2.25(s, 3H), 2.15 (s, 3H),2.11-2.07 (m, 2H),1.92-1.87 (m, 1H).

Examples 26-a and 26-b: Preparation of compounds **26-a** and **26-b**

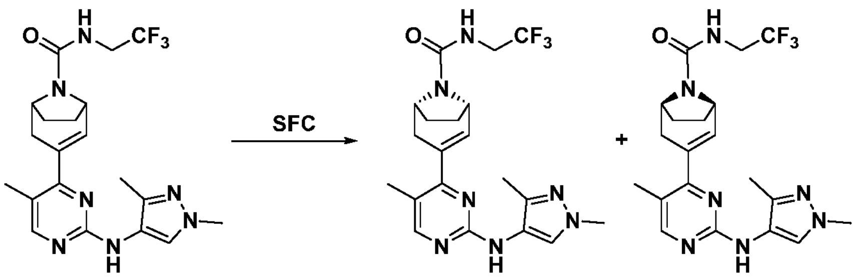


26 26-a and 26-b

Compounds **26-a** and **26-b** were obtained by separation from compound **26** by SFC.

SFC separation conditions:
Column: AD-H 4.6 mm × 250 mm, 5 μm, mobile phase: MeOH (0.2% $NH_3 \cdot H_2O$)/$CO_2$ = 35:65, flow rate: 40 g/min.

**26-a:** retention time: 2.21 min;

LC-MS: m/z 436 $[M+H]^+$.

$^1H$ NMR (400 MHz, $CDCl_3$): $\delta$ppm 8.17 (s, 1H), 7.74 (s, 1H), 6.41 (s, 1H), 6.29-6.28 (m, 1H), 4.85-4.81 (m, 1H), 4.50-4.43 (m, 2H),4.10-3.92 (m, 2H), 3.83 (s, 3H), 3.06-3.00 (m, 1H), 2.38-2.31 (m, 2H), 2.25(s, 3H), 2.15 (s, 3H),2.11-2.07 (m, 2H),1.92-1.87 (m, 1H).

**26-b:** retention time: 2.63 min.

LC-MS: m/z 436 $[M+H]^+$.

$^1H$ NMR (400 MHz, $CDCl_3$): $\delta$ppm8.17 (s, 1H), 7.74 (s, 1H), 6.40 (s, 1H), 6.28-6.27 (m, 1H), 4.92-4.90 (m, 1H), 4.51-4.49 (m, 1H), 4.48-4.44 (m, 1H),3.99-3.89 (m, 2H), 3.82 (s, 3H), 3.06-3.00 (m, 1H), 2.36-2.29 (m, 2H), 2.22(s, 3H), 2.15 (s, 3H),2.09-2.05 (m, 2H),1.92-1.85 (m, 1H).

Example 27: Preparation of 3-(2-((1,5-dimethyl-1*H*-pyrazol-4-yl)amino)-5-methylpyrimidin-4-yl)-*N*-(2,2,2-trifluoroethyl)-8-azabicyclo[3.2.1]oct-2-ene-8-carboxamide (**27**)

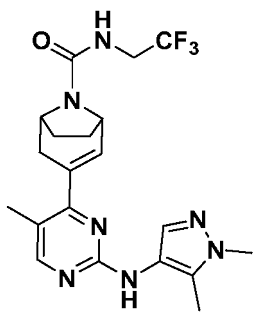

27

The same preparation method as that in Examples 1 and 3 was used to give the title compound **27**, except that 2,4-dichloro-5-methylpyrimidine was used instead of 2,4-dichloropyrimidine (**1e**) and 1,5-dimethyl-1*H*-pyrazol-4-amine was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 20-20-60%; wavelength: 220 nm; flow rate: 45 mL/min; mobile phase: acetonitrile, 0.05% formic acid in water.

LC-MS: m/z 436 $[M+H]^+$.

$^1H$ NMR (400 MHz, DMSO-$d_6$): $\delta$ppm8.43 (s, 1H), 8.12(s, 1H), 7.45 (s, 1H), 7.27-7.24 (m, 1H), 6.28-6.27 (m, 1H),4.55-4.43 (m, 2H), 3.88-3.79 (m, 2H),3.68 (s, 3H),2.87-2.83 (m, 1H), 2.19-2.13 (m, 5H), 2.07(s, 3H),1.94-1.91 (m, 2H),1.72-1.65 (m, 1H).

Example 28: Preparation of 3-(2-((1-(2-hydroxyethyl)-1*H*-pyrazol-4-yl)amino)-5-methylpyrimidin-4-yl)-*N*-(2,2,2-trifluoroethyl)-8-azabicyclo[3.2.1]oct-2-ene-8-carboxamide (**28**)

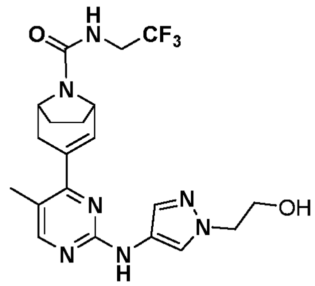

**28**

The same preparation process as that in Examples 1 and 3 was used to give the title compound **28**, except that 2,4-dichloro-5-methylpyrimidine was used instead of 2,4-dichloropyrimidine (**1e**) and 2-(4-amino-1*H*-pyrazol-1-yl)ethane-1-ol was used instead of 1-methyl-1*H*-pyrazol-4-amine (**1g**).

Purification method by preparative liquid chromatography: column: 30 mm × 250 mm; packing: C18, 10 μm; process: 0-2-27 min, acetonitrile 10-10-60%; wavelength: 220 nm; flow rate: 45 mL/min; mobile phase: acetonitrile, 0.05% formic acid in water.

LC-MS: m/z 452 $[M+H]^+$.

$^1H$ NMR (400 MHz, DMSO-$d_6$): $\delta$ppm9.22 (s, 1H), 8.22(s, 1H), 7.83 (s, 1H), 7.47 (s, 1H), 7.31-7.27 (m, 1H), 6.32-6.30 (m, 1H), 4.88-4.86 (m, 1H),4.55-4.45 (m, 2H), 4.09-4.05 (m, 2H), 3.90-3.78 (m, 2H), 3.76-3.70 (m, 2H), 2.93-2.87 (m, 1H),2.26-2.20 (m, 2H), 2.17(s, 3H),2.09-1.95(m, 2H),1.78-1.68 (m, 1H).

Example 29: Preparation of 3-(2-((1-(2-hydroxy-2-methylpropyl)-1*H*-pyrazol-4-yl)amino)-5-methylpyrimidin-4-yl)-*N*-(2,2,2-trifluoroethyl)-8-azabicyclo [3.2.1]oct-2-ene-8-carboxamide (**29**)

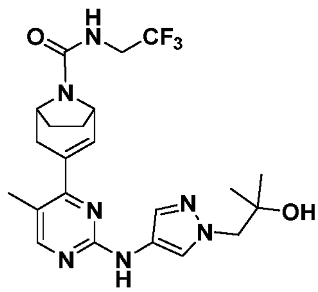

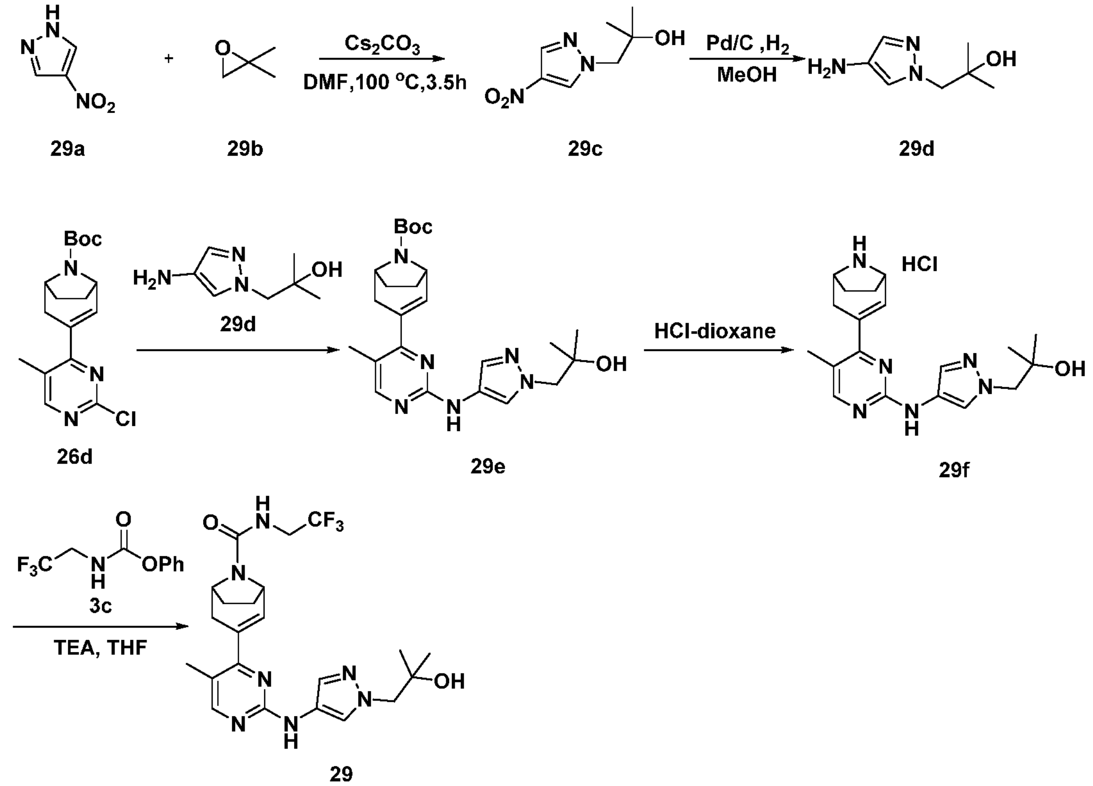


29a 29b 29c 29d

26d 29d 29e 29f

3c 29

**Step 1:** Preparation of 2-methyl-1-(4-nitro-1*H*-pyrazol-1-yl)propan-2-ol (**29c**).

2,2-Dimethyloxirane (1.91 g, 26.5 mmol) was added to a mixture of 4-nitro-1*H*-pyrazole (1.00 g, 8.84 mmol) and cesium carbonate (5.76 g, 17.7 mmol) in *N*,*N*-dimethylformamide (20 mL) at room temperature, and the mixture was stirred at 100°C overnight. Water was added (100 mL) and the reaction solution was extracted with EA (30 mL*3). The organic phase was washed with brine, dried over anhydrous sodium sulfate and concentrated under reduced pressure. The residues were purified by flash column chromatography (mobile phase: petroleum ether/ethyl acetate = 20/1 to 2/1) to give 1.23 g of the title compound as a yellow oil, yield: 75.1%.

**Step 2:** Synthesis of 1-(4-amino-1*H*-pyrazol-1-yl)-2-methylpropan-2-ol (**29d**).

The same preparation process as that in Example **26b** was used to give the title compound **29d**, except that 2-methyl-1-(4-nitro-1*H*-pyrazol-1-yl)propan-2-ol was used instead of 1,3-dimethyl-4-nitro-1*H*-pyrazole (**26a**).

Synthesis steps 3 to 5: The same preparation process as that in Examples 1 and 3 was used to give the title compound **29**, except that 2,4-dichloro-5-methylpyrimidine was used instead of 2,4-dichloropyrimidine and 1-(4-amino-1*H*-pyrazol-1-yl)-2-methylpropan-2-ol was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 10-10-50%; wavelength: 220 nm; flow rate: 45 mL/min; mobile phase: acetonitrile, water.

LC-MS: m/z 480 $[M+H]^+$.

$^1H$ NMR (400 MHz, $CDCl_3$): δppm8.18 (s,1H), 7.88 (s, 1H), 7.54 (s, 1H),6.90 (s, 1H), 6.33-6.32 (m, 1H), 4.86-4.83 (m, 1H),4.47-4.43 (m, 2H), 4.04 (s, 2H), 3.94-3.84 (m, 3H), 3.15-3.11 (m, 1H), 2.38-2.30 (m, 2H),2.17 (s, 3H), 2.12-2.05 (m, 2H), 1.91-1.84 (m, 1H),1.19 (s, 6H).

Examples 29-a and 29-b: Preparation of compounds **29-a** and **29-b**

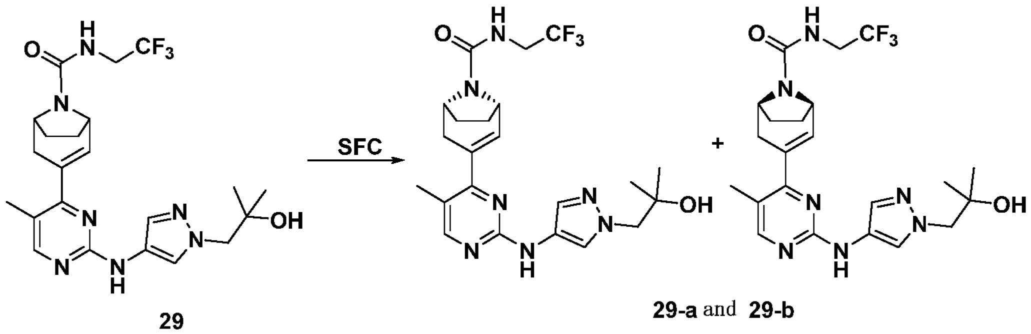


29 29-a and 29-b

Compounds **29-a** and **29-b** were obtained by separation from compound **29** by SFC.

SFC separation conditions:

Column: AD-H 4.6 mm × 250 mm, 5 μm, mobile phase: MeOH (0.2% $NH_3H_2O$)/$CO_2$ = 35:65, flow rate: 40 g/min.

**29-a:** retention time: 2.39 min;

LC-MS: m/z 480 $[M+H]^+$.

$^1H$ NMR (400 MHz, $CDCl_3$): $\delta$ppm8.18 (s,1H), 7.88 (s, 1H), 7.54 (s, 1H),6.90 (s, 1H), 6.33-6.32 (m, 1H), 4.86-4.83 (m, 1H),4.47-4.43 (m, 2H), 4.04 (s, 2H), 3.94-3.84 (m, 3H), 3.15-3.11 (m, 1H), 2.38-2.30 (m, 2H),2.17 (s, 3H), 2.12-2.05 (m, 2H), 1.91-1.84 (m, 1H),1.19 (s, 6H).

**29-b:** retention time: 3.67 min.

LC-MS: m/z 480 $[M+H]^+$.

$^1H$ NMR (400 MHz, $CDCl_3$): $\delta$ppm8.18 (s,1H), 7.88 (s, 1H), 7.54 (s, 1H),6.88 (s, 1H), 6.32-6.31 (m, 1H), 4.88-4.85 (m, 1H), 4.47-4.43 (m, 2H), 4.04 (s, 2H), 3.97-3.83 (m, 3H), 3.15-3.11 (m, 1H), 2.36-2.28 (m, 2H), 2.14 (s, 3H), 2.11-2.06 (m, 2H), 1.88-1.83 (m, 1H),1.19 (s, 6H).

Example 30: Preparation of 3-(5-methyl-2-((1-(oxetan-3-yl)-1*H*-pyrazol-4-yl) amino)pyrimidin-4-yl)-*N*-(2,2,2-trifluoroethyl)-8-azabicyclo[3.2.1]oct-2-ene-8-1-carboxamide (**30**)

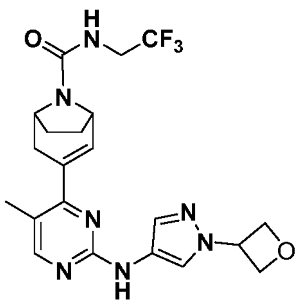

**30**

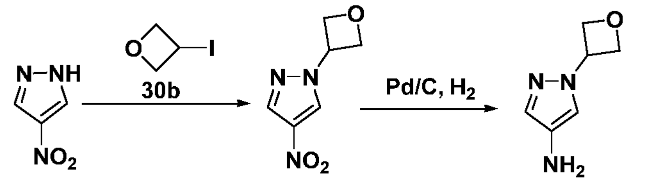


30a 30c 30d

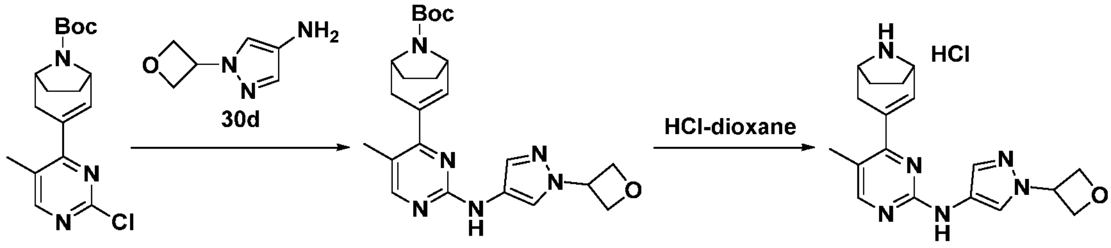


26d 30e 30f

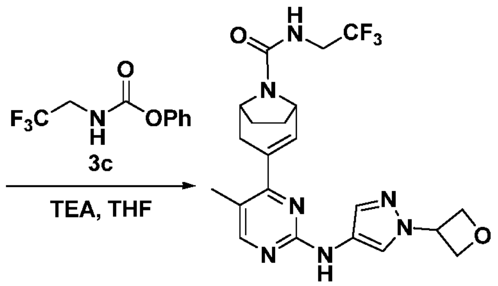


30

**Step 1:** Synthesis of 4-nitro-1-(oxetan-3-yl)-1*H*-pyrazole (**30c**)

Cesium carbonate (11.5 g, 35.4 mmol) was added to a solution of 4-nitro-1*H*-pyrazole (2.00 g, 17.7 mmol) and 3-iodo-oxetane (3.91 g, 21.2 mmol) in DMF (10 mL) at room temperature, and the mixture was stirred at 100°C for 2 hours. The reaction solution was cooled, diluted with ethyl acetate (200 mL) and washed with water (100 mL*3). The organic phase was washed with saturated brine, dried over anhydrous sodium sulfate and concentrated under reduced pressure. The residues were purified by flash column chromatography (mobile phase: petroleum ether/ethyl acetate, 10/1 to 1/1) to give 2.35 g of the title compound as a yellow solid, yield: 78.5%.

**Step 2:** Synthesis of 1-(oxetan-3-yl)-1*H*-pyrazol-4-amine (**30d**)

Palladium on carbon (1.00 g) was added to a solution of 4-nitro-1-(oxetan-3-yl)-1*H*-pyrazole (2.35 g, 13.9 mmol) in methanol (100 mL) under nitrogen atmosphere at room temperature. The mixture was purged with hydrogen for three times and stirred at room temperature overnight. The reaction solution was suction filtered through Celite, and the filtrate was concentrated under reduced pressure to give 2.1 g of the crude title compound as a brown solid.

Synthesis steps 3 to 5: The same preparation method as that in Examples 1 and 3 was used to give the title compound **30**, except that 2,4-dichloro-5-methylpyrimidine was used instead of 2,4-dichloropyrimidine and 1-(oxetan-3-yl)-1*H*-pyrazol-4-amine was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 40% isocratic; wavelength: 220 nm; flow rate: 45 mL/min; mobile phase: acetonitrile, 0.05% formic acid in water.

LC-MS: m/z 464$[M+H]^+$.

$^1H$ NMR (400 MHz, DMSO-$d_6$) : δ ppm 9.31 (s, 1H), 8.24 (s, 1H), 7.96 (s, 1H), 7.63 (s, 1H), 7.29 (t, *J* = 6.3 Hz, 1H), 6.32 (d, *J* = 5.1 Hz, 1H), 5.59-5.46 (m, 1H), 4.96-4.80 (m, 4H), 4.58-4.43 (m, 2H), 3.90-3.76 (m, 2H), 3.00-2.86 (m, 1H), 2.28-2.16 (m, 2H), 2.10 (s, 3H), 2.01-1.92 (m, 2H), 1.80-1.68 (m, 1H).

Example 31: Preparation of 3-(2-((1-cyclopropyl-1*H*-pyrazol-4-yl)amino)-5-methylpyrimidin-4-yl)-*N*-(2,2,2-trifluoroethyl)-8-azabicyclo[3.2.1]oct-2-ene-8-carboxamide (**31**)

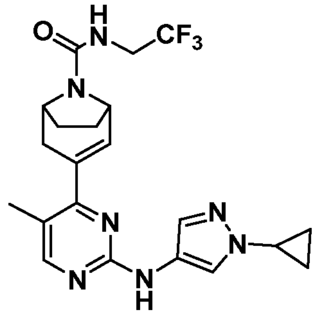

**31**

The same preparation method as that in Examples 1 and 3 was used to give the title compound **31**, except that 2,4-dichloro-5-methylpyrimidine was used instead of 2,4-dichloropyrimidine (**1e**) and 2-(4-amino-1*H*-pyrazol-1-yl)ethane-1-ol was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 40% isocratic; wavelength: 220 nm; flow rate: 45 mL/min; mobile phase: acetonitrile, 0.05% formic acid in water.

LC-MS: m/z 448 $[M+H]^+$.

$^1H$ NMR (300 MHz, DMSO-$d_6$):δppm9.22 (s,1H), 8.21 (s, 1H),7.82 (s, 1H), 7.42 (s, 1H),7.30-7.26 (m1H),6.31-6.29 (m, 1H), 4.57-4.51 (m, 1H), 4.48-4.44 (m, 1H), 3.89-3.77 (m, 2H), 3.67-3.60 (m, 1H), 2.94-2.88 (m, 1H), 2.22-2.16 (m, 2H), 2.08 (s, 3H), 1.99-1.89 (m, 2H),1.76-1.66 (m, 1H), 0.99-0.87(m, 4H).

Example 32: Preparation of 3-(2-((5-chloro-1-methyl-1*H*-pyrazol-4-yl)amino)-5-methylpyrimidin-4-yl)-*N*-(2,2,2-trifluoroethyl)-8-azabicyclo[3.2.1]oct-2-ene-8-carboxamide (**32**)

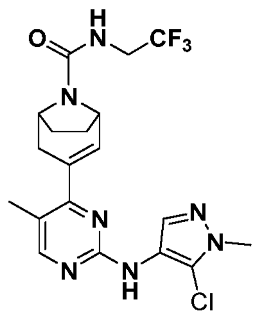

**32**

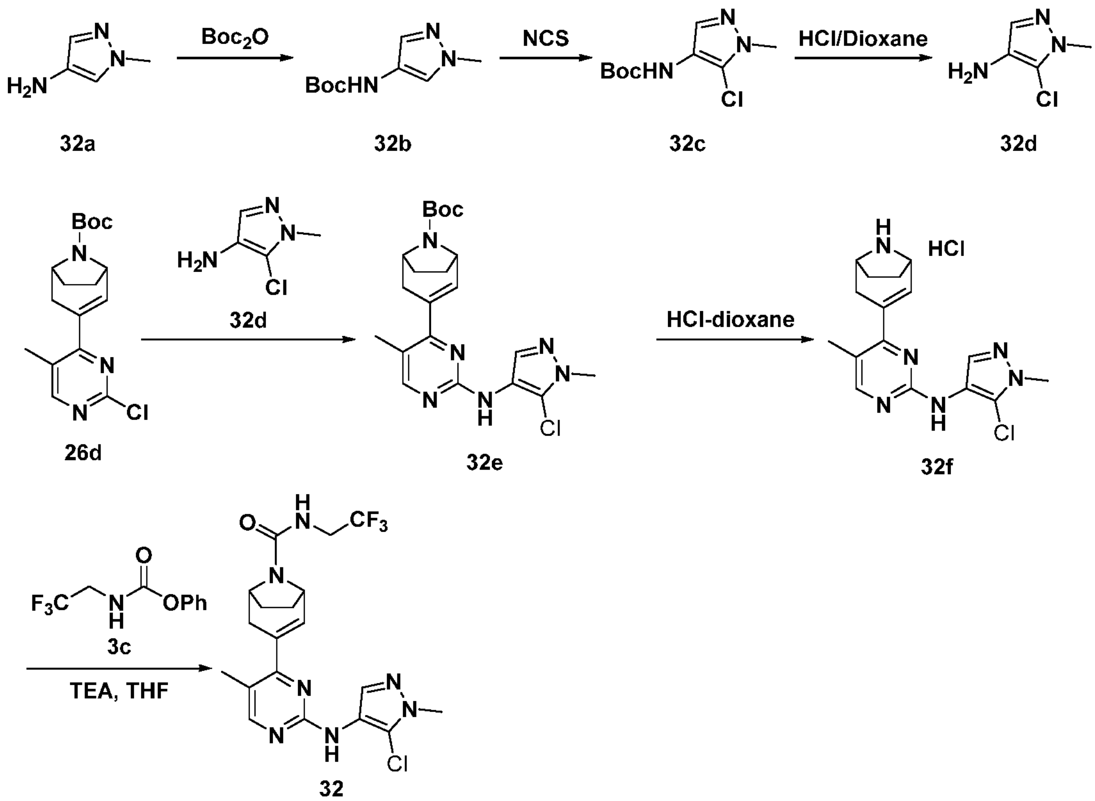


32a 32b 32c 32d 26d 32d 32e 32f 3c 32

**Step 1:** Synthesis of tert-butyl (1-methyl-1*H*-pyrazol-4-yl)carbamate (**32b**).

Triethylamine (8.32 g, 82.4 mmol) was added to a solution of 1-methyl-1*H*-pyrazole-4-amine (4.00 g, 41.2 mmol) and di-tert-butyl dicarbonate (10.8 g, 49.4 mmol) in dichloromethane (200 mL) at room temperature, and the mixture was stirred at room temperature overnight. Water was added (100 mL) and the reaction solution was extracted with dichloromethane (100 mL*3). The organic phase was washed with brine, dried over anhydrous sodium sulfate and concentrated under reduced pressure. The residues were purified by flash column chromatography (mobile phase: petroleum ether/ethyl acetate = 20/1 to 2/1) to give 7.90 g of the title compound as a yellow oil, yield: 97.3%.

LC-MS: m/z 198[M+H]$^+$.

**Step 2:** Synthesis of tert-butyl (5-chloro-1-methyl-1*H*-pyrazol-4-yl)carbamate (**32c**).

NCS (2.24 g, 16.8 mmol) was added to a solution of tert-butyl (1-methyl-1*H*-pyrazol-4-yl)carbamate (3.00 g, 15.2 mmol) in dichloromethane (60 mL) under nitrogen atmosphere at room temperature, and the mixture was stirred at 30°C overnight. The reaction solution was concentrated under reduced pressure and rotary evaporated to dry. The residues were purified by flash column chromatography (mobile phase: petroleum ether/ethyl acetate = 20/1 to 2/1) to give 2.97 g of the title compound as a brown solid, yield: 84.2%.

LC-MS: m/z 232[M+H]$^+$.

**Step 3:** Synthesis of 5-chloro-1-methyl-1*H*-pyrazol-4-amine (**32d**).

HCl/dioxane (10 mL, 4 M) was added to a solution of tert-butyl (5-chloro-1-methyl-1*H*-pyrazol-4-yl)carbamate (2.00 g, 8.62 mmol) in dichloromethane (10 mL) under nitrogen atmosphere at room temperature, and the mixture was stirred at room temperature for 30 minutes. The reaction solution was concentrated under reduced pressure and rotary evaporated to dry, and then $NH_3$/methanol (10 mL, 4 M) was added and rotary evaporated to dry. The residues were purified by flash column chromatography (mobile phase: petroleum ether/ethyl acetate = 100/1 to 20/1) to give 910 mg of the title compound as a brown solid, yield: 80.2%.

LC-MS: m/z 132[M+H]$^+$.

Synthesis steps 4 to 6: The same preparation method as that in Examples 1 and 3 was used to give the title compound **32**, except that 2,4-dichloro-5-methylpyrimidine was used instead of 2,4-dichloropyrimidine and 5-chloro-1-methyl-1*H*-pyrazol-4-amine was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 20-20-70%; wavelength: 220 nm; flow rate: 45 mL/min; mobile phase: acetonitrile, 0.05% formic acid in water.

LC-MS: m/z 456 $[M+H]^+$.

$^1H$ NMR (400 MHz, DMSO): δppm8.59 (s,1H), 8.16(s, 1H), 7.64 (s, 1H),7.28-7.25 (m, 1H), 6.31-6.30 (m, 1H), 4.54-4.43 (m, 2H),3.85-3.81 (m, 2H),3.77 (s, 2H), 2.87-2.83 (m, 1H), 2.25-2.15 (m, 2H), 2.09 (s, 3H), 2.01-1.93 (m, 2H), 1.72-1.65 (m, 1H).

Example 33: Preparation of 3-(2-((1-methyl-1*H*-pyrazol-4-yl)amino)pyrimidin-4-yl)-*N*-(1-(trifluoromethyl)cyclopropyl)-8-azabicyclo[3.2.1]oct-2-ene-8-carboxamide (**33**)

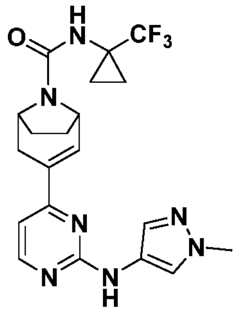

**33**

The same preparation method as that in Example 3 was used to give the title compound **33**, except that 1-(trifluoromethyl)cycloprop-1-amine hydrochloride was used instead of 2-aminoacetonitrile (**2a**).

Purification method by preparative liquid chromatography: column: 30 mm × 250 mm; packing: C18, 10 μm; process: 0-2-22 min, acetonitrile 20-20-60%; wavelength: 220 nm; flow rate: 45 mL/min; mobile phase: acetonitrile, 0.05% formic acid in water.

LC-MS: m/z 434$[M+H]^+$.

$^1H$ NMR (300 MHz, DMSO-d6) δppm 9.33 (s, 1H), 8.32 (d, *J* = 5.2 Hz, 1H), 7.81 (s, 1H), 7.49 (d, *J* = 18.1 Hz, 2H), 7.18 (d, *J* = 5.2 Hz, 1H), 6.80 (d, *J* = 5.2 Hz, 1H), 4.65-4.43 (m, 2H), 3.81 (s, 3H), 2.92 (d, *J* = 18.0 Hz, 1H), 2.30-2.11 (m, 2H), 2.05-1.90 (m, 2H), 1.65-1.61 (m, 1H), 1.26-1.07 (m, 2H), 1.05-0.98 (m, 2H).

Example 34: Preparation of 3-(2-((1-methyl-1*H*-pyrazol-4-yl)amino)-5-(methylsulfonyl)pyrimidin-4-yl)-*N*-(2,2,2-trifluoroethyl)-8-azabicyclo[3.2.1]oct-2-ene-8 -carboxamide (**34**)

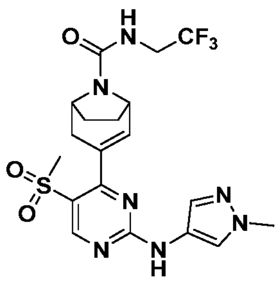

**34**

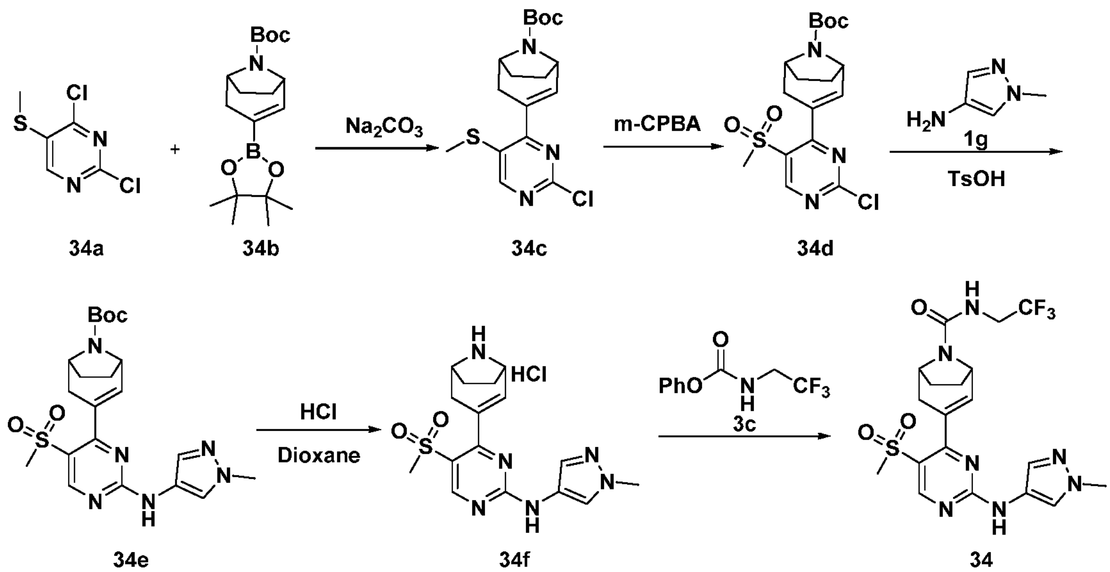


**Step 1:** Synthesis of tert-butyl-3-(2-chloro-5-(methylthio)pyrimidin-4-yl)-8-azabicyclo[3.2.1]oct-2-ene-8-carboxylate (**34c**).

[1,1'-Bis(diphenylphosphino)ferrocene]palladium dichloride (754 mg, 1.03 mmol) and sodium carbonate (2.70 g, 25.8 mmol) were added to a mixture of tert-butyl 3-(4,4,5,5-tetramethyl-1,3,2-dioxin-2-yl)-8-azabicyclo[3.2.1]oct-2-ene-8-carboxylate (3.70 g, 11.3 mmol) and 2,4-dichloro-5-(methylthio)pyrimidine (2.00 g, 10.3 mmol) in dioxane and water (60 mL, v/v=5:1) at room temperature. The mixture was purged with nitrogen for three times and stirred at 90°C overnight. The reaction solution was concentrated under reduced pressure. Water was added (150 mL). The mixture was extracted with EA (100 mL*3). The organic phase was washed with brine, dried over anhydrous sodium sulfate and concentrated under reduced pressure. The residues were separated and purified by silica gel column chromatography (mobile phase: petroleum ether/ethyl acetate = 100/1 to 4/1) to give 1.30 g of the title compound as a yellow solid, yield: 34.0%, LC-MS: m/z 368$[M+H]^+$.

**Step 2:** Synthesis of tert-butyl-3-(2-chloro-5-(methylsulfonyl)pyrimidin-4-yl)-8-azabicyclo[3.2.1]oct-2-ene-8-carboxylate (**34d**).

m-Chloroperoxybenzoic acid (2.15 g, 12.5 mmol) was added to a solution of tert-butyl-3-(2-chloro-5-(methylthio)pyrimidin-4-yl)-8-azabicyclo[3.2.1]oct-2-ene-8-carboxylate (1.15 g, 3.13 mmol) in dichloromethane (20 mL) at room temperature, and the mixture was stirred at room temperature overnight. The reaction system was filtered. The filtrate was concentrated under reduced pressure and extracted with ethyl acetate (100 mL*3). The organic phase was washed with brine, dried over anhydrous sodium sulfate and concentrated under reduced pressure. The residues were purified by silica gel column chromatography (mobile phase: petroleum ether/ethyl acetate = 100/1 to 2/1) to give 640 mg of the title compound as a pale yellow solid, yield: 51.4%. LC-MS: m/z 400$[M+H]^+$.

Synthesis steps 3 to 5: The same preparation process as that in Examples 1 and 3 was used to give the title compound **34**, except that tert-butyl-3-(2-chloro-5-(methylsulfonyl)pyrimidin-4-yl)-8-azabicyclo[3.2.1]oct-2-ene-8-carboxylate was used instead of tert-butyl 3-(2-chloropyrimidin-4-yl)-8-azabicyclo[3.2.1]oct-2-ene-8-carboxylate (**1f**).

Purification method by preparative liquid chromatography: column: 30 mm × 250 mm; packing: C18, 10 μm; process: 0-2-22 min, acetonitrile 20-20-70%; wavelength: 220 nm; flow rate: 45 mL/min; mobile phase: acetonitrile, 0.05% formic acid in water.

LC-MS: m/z 486$[M+H]^+$.

$^1H$ NMR (300 MHz, DMSO-$d_6$): $\delta$ppm10.48-10.43(d, 1H), 8.77-8.71(d, 1H), 7.92-7.79(m, 1H),7.55-7.52(m, 1H),7.35-7.21(m, 1H), 6.40-6.29(m, 1H), 4.54-4.46(m, 2H), 3.85-3.81 (m,5H), 3.21-3.20 (m,3H), 3.09-2.92 (m,1H),2.18-1.94 (m,5H).

Example 35: Preparation of 3-(5-cyano-2-((1-methyl-1*H*-pyrazol-4-yl)amino) pyrimidin-4-yl)-*N*-(2,2,2-trifluoroethyl)-8-azabicyclo[3.2.1]oct-2-ene-8-carboxamide (**35**)

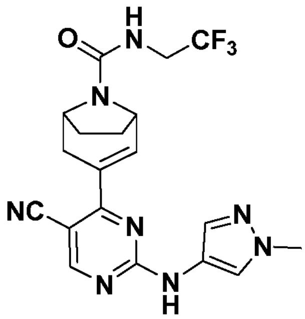

35

The same preparation method as that in Example 25 was used to give the title compound **35**, except that 5-cyano-2,4-dichloropyrimidine was used instead of 2,4-dichloro-5-(trifluoromethyl)pyrimidine (**25a**).

Purification method by preparative liquid chromatography: column: 30 mm × 250 mm; packing: C18, 10 μm; process: 0-2-22 min, acetonitrile 10-10-60%; wavelength: 220 nm; flow rate: 45 mL/min; mobile phase: acetonitrile, 0.05% formic acid in water.

LC-MS: m/z 433 $[M+H]^+$.

$^1H$ NMR (300 MHz, DMSO-$d_6$) $\delta$ppm 9.94 (s, 1H), 8.67 (s, 1H), 7.92 (s, 1H), 7.75 (s, 1H), 7.53 (d, *J* = 5.3 Hz, 1H), 7.31-7.22 (m, 1H), 4.69-4.58 (m, 1H), 4.56-4.47 (m, 1H), 3.85 (s, 3H), 3.84-3.71 (m, 2H), 3.04-2.91 (m, 1H), 2.40-2.28 (m, 1H), 2.23-2.05 (m, 1H), 2.01-1.87 (m, 2H), 1.75-1.54 (m, 1H).

Example 36: Preparation of (3-(2-((1-cyclopropyl-1*H*-pyrazol-4-yl)amino) pyrimidin-4-yl)-8-azabicyclo[3.2.1]oct-2-en-8-yl)((*S*)-2,2-difluorocyclopropyl) methanone (**36**)

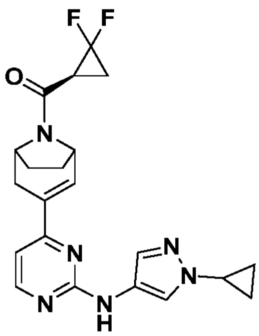

36

The same preparation process as that in Example 1 was used to give the title compound **36**, except that 1-cyclopropyl-1*H*-pyrazol-4-amine was used instead of 1-methyl-1*H*-pyrazol-4-amine (**1g**).

Preparation method: column: 30 mm × 250 mm; packing: C18, 10 μm; process: 0-2-22 min, acetonitrile 15-15-55%; wavelength: 220 nm; flow rate: 45 mL/min; mobile phase: acetonitrile, 0.05% formic acid in water.

LC-MS: m/z 413 $[M+H]^+$.

$^1H$ NMR (300 MHz, DMSO-$d_6$): $\delta$ppm9.34 (s,1H), 8.35-8.32 (m, 1H), 7.87 (s, 1H), 7.51-7.48 (m, 1H), 7.22-7.16 (m1H),6.85-6.80 (m, 1H),4.89-4.84 (m, 1H),4.77-4.70 (m, 1H), 3.69-3.65 (m, 1H),3.20-3.15 (m, 1H), 2.96-2.85 (m, 1H), 2.63-2.57 (m, 1H), 2.44-2.35 (m, 1H), 2.31-2.24 (m, 1H),2.14-2.05 (m, 2H),1.94-1.85 (m, 2H), 1.00-0.96 (m, 2H), 0.95-0.92 (m, 2H).

Example 37: Preparation of ((*S*)-2,2-difluorocyclopropyl)(3-(2-((1-(oxetan-3-yl)-1*H*-pyrazol-4-yl)amino)pyrimidin-4-yl)-8-azabicyclo[3.2.1]oct-2-en-8-yl)methanone (**37**)

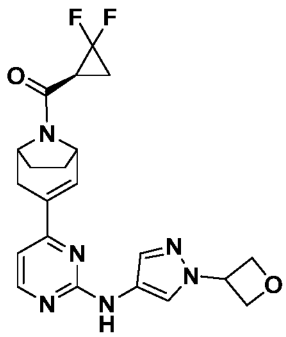

37

The same preparation method as that in Example 1 was used to give the title compound **37**, except that 1-(oxetan-3-yl)-1*H*-pyrazole-4-amine (**30d**) was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 15-15-55%; wavelength: 220 nm; flow rate: 45 mL/min; mobile phase: acetonitrile, 0.05% formic acid in water.

LCMS[M+H]: 429.

$^1$H NMR (300MHz, DMSO-$d_6$) :δppm9.44 (s, 1H), 8.40-8.23 (m, 1H), 7.98 (s, 1H), 7.67 (s, 1H), 7.28-7.15 (m, 1H), 6.88-6.79 (m, 1H), 5.59-5.47 (m, 1H), 4.99-4.64(m, 6H), 3.25-3.08 (m, 1H), 2.98-2.80 (m, 1H), 2.71-2.55 (m, 1H), 2.36-2.20 (m, 1H), 2.10-1.63 (m, 5H).

Example 38: Preparation of 2-(4-((4-(8-((*S*)-2,2-difluorocyclopropane-1-formyl)-8-azabicyclo[3.2.1]oct-2-en-3-yl)pyrimidin-2-yl)amino)-1*H*-pyrazol-1-yl)acetamide (**38**)

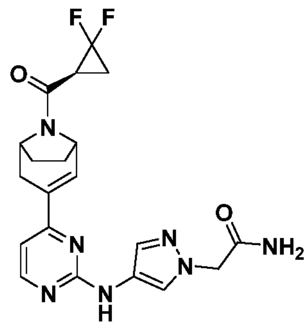

38

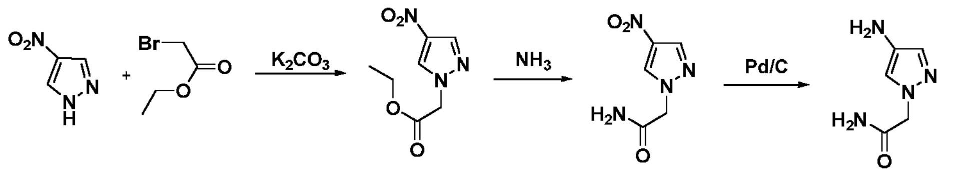


38a 38b 38c 38d 38e

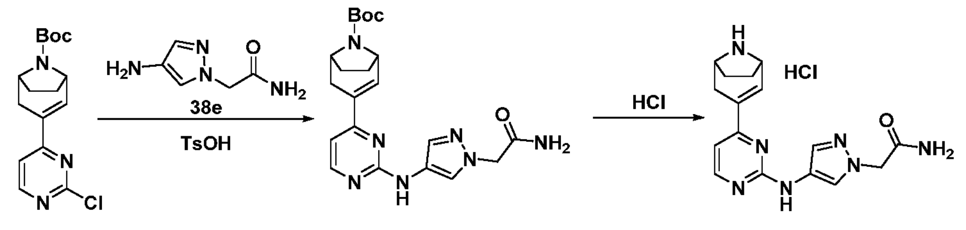


1f 38f 38g

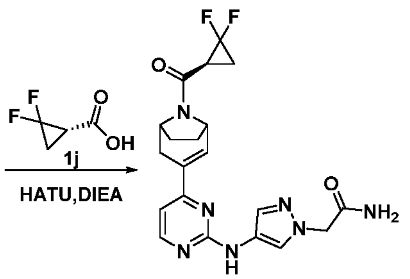


38

**Step 1:** Synthesis of ethyl 2-(4-nitro-1*H*-pyrazol-1-yl)acetate (**38c**).

4-Nitro-1*H*-pyrazole (10.0 g, 88.0 mmol) and potassium carbonate (24.0 g, 88.0 mmol) were added to a solution of ethyl 2-bromoacetate in *N,N*-dimethylformamide (200 mL) at room temperature. The mixture was replaced with nitrogen three times and stirred at 90°C overnight. Water was added to the reaction system. The mixture was extracted with EA (100 mL*3). The organic phase was washed with brine, dried over anhydrous sodium sulfate and concentrated under reduced pressure. The residues were purified by flash column chromatography (mobile phase: petroleum ether/ethyl acetate = 100/1 to 4/1) to give 16.0 g of the title compound as a yellow solid, yield: 90.0%

LC-MS: m/z 200$[M+H]^+$

**Step 2:** Synthesis of 2-(4-nitro-1*H*-pyrazol-1-yl)acetamide (**38d**).

Ammonium hydroxide (2.4 g, 17.2 mmol, 25-28%) was added to a solution of ethyl 2-(4-nitro-1*H*-pyrazol-1-yl)acetate (1.60 g, 8.60 mmol) in methanol (10 mL) at room temperature, and the mixture was stirred at room temperature overnight. The reaction solution was concentrated under reduced pressure to give 1.10 g of the title compound as a white solid, yield: 74.8%

LC-MS: m/z 171 $[M+H]^+$.

**Step 3:** Synthesis of 2-(4-amino-1*H*-pyrazol-1-yl)acetamide (**38e**).

Palladium on carbon (50 mg) was added to a solution of 2-(4-nitro-1*H*-pyrazol-1-yl)acetamide (300 mg, 1.76 mmol) in methanol (10 mL) at room temperature, and the mixture was stirred under hydrogen atmosphere at room temperature overnight. The reaction solution was filtered, and the filtrate was concentrated under reduced pressure to give 240 mg of the title compound as a red solid, yield: 96.0%.

Synthesis steps 4 to 6: The same preparation method as that in Example 1 was used to give the title compound **38**, except that 2-(4-amino-1*H*-pyrazol-1-yl)acetamide was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 10-10-50%; wavelength: 220 nm; flow rate: 45 mL/min; mobile phase: acetonitrile, 0.05% formic acid in water.

LC-MS: m/z 430$[M+H]^+$.

$^1H$ NMR (300 MHz, DMSO-$d_6$): δppm10.34-10.27(m, 1H), 9.35(s, 1H), 8.14 (s, 1H), 7.94-7.85(m, 1H),7.54-7.17(m, 3H),6.82-6.79(m, 1H), 4.89-4.67(m, 4H), 3.25-2.75(m, 3H), 2.45-1.60 (m,6H).

Example 39: Preparation of 1-(4-((4-(8-((*S*)-2,2-difluorocyclopropane-1-carbonyl)-8-azabicyclo[3.2.1]oct-2-en-3-yl)pyrimidin-2-yl)amino)-1*H*-pyrazol-1-yl))cyclopropane -1-carboxamide (**39**)

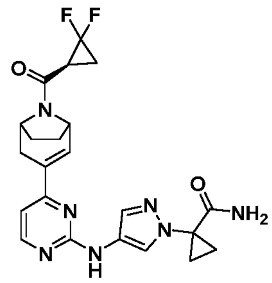

**39**

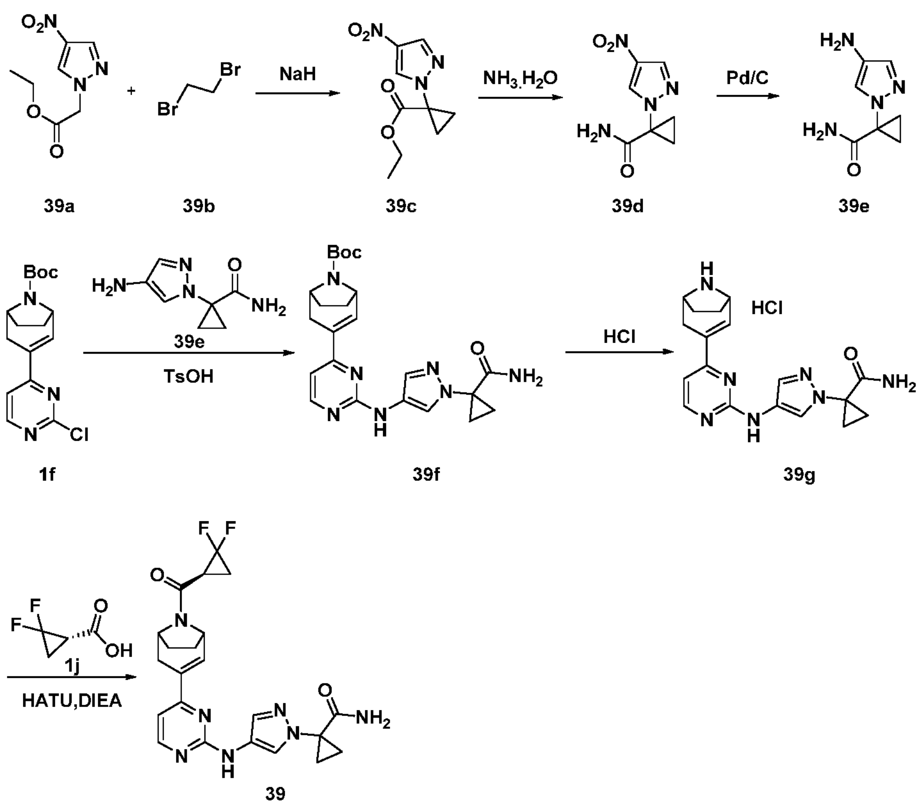


39a 39b 39c 39d 39e

1f 39f 39g

39

**Step 1:** Synthesis of ethyl 1-(4-nitro-1*H*-pyrazol-1-yl)cyclopropane-1-carboxylate (**39c**)

Sodium hydride (2.15 g, 57.6 mmol) was added to a solution of ethyl 2-(4-nitro-1*H*-pyrazol-1-yl)acetate (5.00 g, 26.8 mmol) in anhydrous *N*,*N*-dimethylformamide (150 mL) under nitrogen atmosphere at 0°C and reacted for 10 minutes. 1,2-dibromoethane (6.03 g, 32.0 mmol) was added dropwise. After completion of the addition, the mixture was naturally warmed to room temperature and stirred overnight. The reaction solution was added to water (650 mL) and extracted with EA (100 mL*3). The organic phase was washed with brine, dried over anhydrous sodium sulfate and concentrated under reduced pressure. The residues were purified by flash column chromatography (mobile phase: petroleum ether/ethyl acetate = 100/1 to 4/1) to give 1.2 g of the title compound as a yellow solid, yield: 20.0%.
LC-MS: m/z 226[M+H]$^+$.

**Step 2:** Synthesis of 1-(4-nitro-1*H*-pyrazol-1-yl)cyclopropane-1-carboxamide (**39d**)

Ammonium hydroxide (743 mg, 5.31 mmol, 25-28%) was added to a solution of ethyl 1-(4-nitro-1*H*-pyrazol-1-yl)cyclopropane-1-carboxylate (400 mg, 1.77 mmol) in methanol (10 mL) at room temperature, and the mixture was stirred at room temperature overnight. The reaction solution was concentrated under reduced pressure to give 300 mg of the title compound as a white solid, yield: 86.0%

LC-MS: m/z 197[M+H]$^+$.

**Step 3:** Synthesis of 1-(4-amino-1*H*-pyrazol-1-yl)cyclopropane-1-carboxamide (**39e**)

Palladium on carbon (50 mg) was added to a solution of 1-(4-nitro-1*H*-pyrazol-1-yl) cyclopropane-1-carboxamide (300 mg, 1.52 mmol) in methanol (10 mL) at room temperature, and the mixture was stirred under hydrogen atmosphere at room temperature overnight. The reaction solution was filtered, and the filtrate was concentrated under reduced pressure to give 250 mg of the title compound as a red solid, yield: 99.1%.

LC-MS: m/z 167[M+H]$^+$.

Synthesis steps 4 to 6: The same preparation process as that in Example 1 was used to give the title compound **39**, except that 1-(4-amino-1*H*-pyrazol-1-yl) cyclopropane-1-carboxamide was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 20-20-40%; wavelength: 220 nm; flow rate: 45 mL/min; mobile phase: acetonitrile, 0.05% formic acid in water.

LC-MS: m/z 456[M+H]$^+$.

$^1$H NMR (300 MHz, DMSO-$d_6$): δppm9.48 (s, 1H), 8.37-8.34(m, 1H), 7.90 (s, 1H), 7.63(s, 1H), 7.34(s, 1H),7.23-7.15(m, 1H), 6.89-6.83(m, 1H), 6.06(s, 1H), 4.89-4.69 (m,2H), 3.22-2.52(m, 3H), 2.50-1.65 (m,6H), 1.50 (s,2H),1.37(s,2H).

Example 40: Preparation of ((*S*)-2,2-difluorocyclopropyl)(3-(2-((1-(2-hydroxyethyl)-1*H*-pyrazol-4-yl)amino)pyrimidin-4-yl)-8-azabicyclo[3.2.1]oct-2-en-8-yl)methanone (**40**)

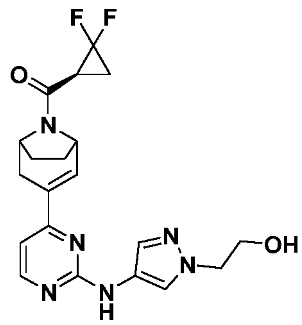

**40**

The same preparation method as that in Example 1 was used to give the title compound **40**, except that 2-(4-amino-1*H*-pyrazol-1-yl)ethane-1-ol was used instead of 1-methyl-1*H*-pyrazol-4-amine (**1g**).

Purification method by preparative liquid chromatography: column: 30 mm × 250 mm; packing: C18, 10 μm; process: 0-2-22 min, acetonitrile 10-10-50%; wavelength: 220 nm; flow rate: 45 mL/min; mobile phase: acetonitrile, 0.05% formic acid in water.

LC-MS: m/z 417 [M+H]$^+$.

$^1$H NMR (300 MHz, DMSO-$d_6$): δppm9.33 (s,1H), 8.33 (s, 1H), 7.88 (s, 1H), 7.52 (s, 1H), 7.24-7.18 (m1H), 6.81 (s, 1H), 4.88-4.70 (m, 3H), 4.09-4.05 (m, 2H), 3.77-3.71 (m, 2H), 3.17-3.14 (m, 1H), 2.95-2.83 (m, 1H), 2.62-2.58 (m, 1H), 2.26-2.09 (m, 2H), 1.94-1.67 (m, 4H).

Example 41: Preparation of ((*S*)-2,2-difluorocyclopropyl)(3-(2-((1-(2-hydroxy-2-methylpropyl)-1*H*-pyrazol-4-yl)amino)pyrimidin-4-yl)-8-azabicyclo[3.2.1]oct-2-en-8-yl)methanone (**41**)

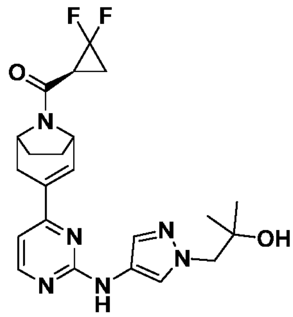

**41**

The same preparation method as that in Example 1 was used to give the title compound **41**, except that 1-(4-amino-1*H*-pyrazol-1-yl)-2-methylpropan-2-ol was used instead of 1-methyl-1*H*-pyrazol-4-amine (**1g**).

Purification process by preparative liquid chromatography: column: 30 mm × 250 mm; packing: C18, 10 μm; process: 0-2-22 min, acetonitrile 20-20-60%; wavelength: 220 nm; flow rate: 45 mL/min; mobile phase: acetonitrile, 0.05% formic acid in water.

LC-MS: m/z 445 $[M+H]^+$.

$^1H$ NMR (300 MHz, DMSO-$d_6$): δppm9.36 (s,1H), 8.37-8.31 (m, 1H), 7.92-7.86 (m, 1H), 7.51-7.49 (m1H), 7.23-7.18(m1H), 6.82-6.78 (m, 1H), 4.87-4.81 (m, 2H), 4.70-4.67 (m, 1H), 3.95 (s, 2H),3.20-3.09 (m, 2H),2.93-2.87 (m, 1H), 2.48-2.25 (m, 1H), 2.08-2.02 (m, 1H), 1.96-1.80 (m, 4H), 1.04 (s, 6H).

Example 42: Preparation of 3-(2-((1-(2-hydroxyethyl)-1*H*-pyrazol-4-yl)amino) pyrimidin-4-yl)-*N*-(2,2,2-trifluoroethyl)-8-azabicyclo[3.2.1]oct-2-ene-8-carboxamide (**42**)

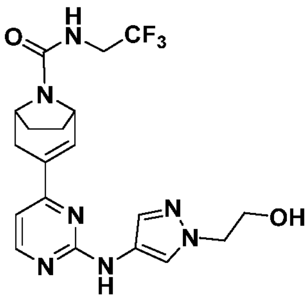

**42**

The same preparation method as that in Examples 1 and 3 was used to give the title compound **42**, except that 2-(4-amino-1*H*-pyrazol-1-yl)ethane-1-ol was used instead of 1-methyl-1*H*-pyrazol-4-amine (**1g**).

Purification method by preparative liquid chromatography: column: 30 mm × 250 mm; packing: C18, 10 μm; process: 2-22 min, acetonitrile 10-50%; wavelength: 220 nm; flow rate: 45 mL/min; mobile phase: acetonitrile, aqueous solution of 0.05% formic acid.

LC-MS: m/z 438 $[M+H]^+$.

$^1H$ NMR (400 MHz, DMSO-$d_6$):δppm9.34 (s, 1H),8.32 (*d,J* = 6Hz, 1H), 7.87 (s, 1H), 7.54 (s,1H), 7.29-7.21 (m, 2H), 6.80 (*d,J* = 6Hz, 1H),4.92-4.88 (m, 1H), 4.61-4.52 (m, 2H),4.11-4.07 (m, 2H), 3.87-2.68 (m, 4H), 2.97-2.92 (m, 1H), 2.33-2.27 (m, 1H), 2.15-2.13 (m, 1H), 1.90-1.70 (m, 2H), 1.65-1.60 (m, 1H).

Example 43: Preparation of 3-(2-((1-cyclopropyl-1*H*-pyrazol-4-yl)amino) pyrimidin-4-yl)-*N*-(2,2,2-trifluoroethyl)-8-azabicyclo[3.2.1]oct-2-ene-8-carboxamide (**43**)

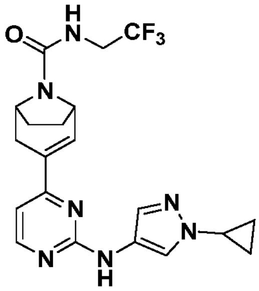

**43**

The same preparation method as that in Examples 1 and 3 was used to give the title compound **43**, except that 1-cyclopropyl-1*H*-pyrazol-4-amine was used instead of 1-methyl-1*H*-pyrazol-4-amine (**1g**).
Purification method by preparative liquid chromatography: column: 30 mm × 250 mm; packing: C18, 10 μm; process: 0-22 min, acetonitrile 30-70%; wavelength: 220 nm; flow rate: 45 mL/min; mobile phase: acetonitrile, 0.05% aqueous solution of formic acid.
LC-MS: m/z 434[M+H]$^+$.
$^1$H NMR (300 MHz, DMSO-*d*$_6$): δppm9.33 (s, 1H), 8.40-8.32(m, 1H), 7.87(s, 1H), 7.52(s, 1H),7.29-7.20(m, 2H),6.83-6.81(m, 1H), 4.63-4.51(m, 2H), 3.84-3.70(m, 3H), 2.98-2.94(m, 1H), 2.32-2.10 (m,2H),1.90-1.70 (m,2H) ,1.68-1.65(m,1H),1.00-0.90 (m,4H).

Example 44: Preparation of *N*-(2,2-difluoroethyl)-3-(2-((1-methyl-1*H*-pyrazol-4-yl) amino)pyrimidin-4-yl)-8-azabicyclo[3.2.1]oct-2-ene-8-carboxamide (**44**)

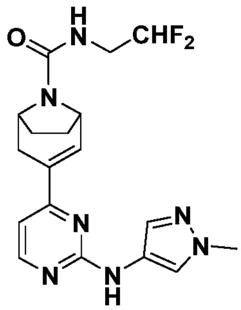

**44**

The same preparation method as that in Example 2 was used to give the title compound **44**, except that 2,2-difluoroethane-1-amine hydrochloride was used instead of 2-aminoacetonitrile (**2a**).
Purification method by preparative liquid chromatography: column: 30 mm × 250 mm; packing: C18, 10 μm; process: 0-22 min, acetonitrile 10-50%; wavelength: 220 nm; flow rate: 45 mL/min; mobile phase: acetonitrile, 0.05% aqueous solution of formic acid.
LC-MS: m/z 390 [M+H]$^+$.
$^1$H NMR (301 MHz, DMSO-*d*$_6$) δppm9.33 (s, 1H), 8.32 (d, *J* = 5.2 Hz, 1H), 7.80 (s, 1H), 7.51 (s, 1H), 7.21 (s, 1H), 7.10-7.01 (m, 1H), 6.83-6.76 (m, 1H), 6.15-5.68 (m, 1H), 4.65-4.43 (m, 2H), 3.80 (s, 3H), 3.48-3.35 (m, 2H), 3.01-2.88 (m, 1H), 2.33-2.22 (m, 1H), 2.20-2.06(m, 1H), 1.99-1.84 (m, 2H), 1.73-1.57 (m, 1H).

Example 45: Preparation of ((*S*)-2,2-difluorocyclopropyl)(3-(2-((1-ethyl-1*H*-pyrazol-4-yl)amino)pyrimidin-4-yl)-8-azabicyclo[3.2.1]oct-2-en-8-yl)methanone (**45**)

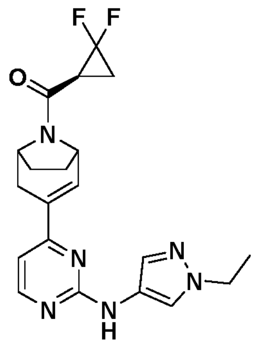

**45**

The same preparation method as that in Example 1 was used to give the title compound **45,** except that 1-ethyl-1*H*-pyrazol-4-amine was used instead of 1-methyl-1*H*-pyrazol-4-amine (**1g**).

Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.

LC-MS: m/z 401 $[M+H]^+$

$^1$H NMR (400 MHz, DMSO-$d_6$):$\delta$ppm9.37 (s, 1H),8.36-8.32 (m, 1H), 7.86-7.85 (m, 1H), 7.53-7.50 (m,1H), 7.24-7.19 (m, 1H), 6.85-6.80 (m, 1H),4.90-4.71 (m, 2H), 4.12-4.05 (m, 2H), 3.32-3.18 (m, 1H), 3.07-2.62 (m, 1H), 2.51-2.44 (m, 1H), 2.26-2.23 (m, 1H), 2.13-1.80 (m, 5H), 1.38-1.23 (m, 3H).

Example 46: Preparation of ((*S*)-2,2-difluorocyclopropyl)(3-(2-((l-isopropyl-1H-pyrazol-4-yl)amino)pyrimidin-4-yl)-8-azabicyclo[3.2.1]oct-2-en-8-yl)methanone (46)

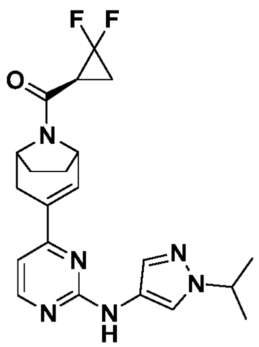

**46**

The same preparation method as that in Example 1 was used to give the title compound **46,** except that 1-isopropyl-1*H*-pyrazol-4-amine was used instead of 1-methyl-1*H*-pyrazol-4-amine (**1g**).

Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, aqueous solution of 0.05% formic acid.

LC-MS: m/z 415 $[M+H]^+$.

$^1$H NMR (400 MHz, DMSO-$d_6$): $\delta$ppm9.35 (s,1H), 8.36-8.33 (m, 1H), 7.89-7.87 (m, 1H),7.55-7.51 (m, 1H), 7.25-7.17 (m1H), 6.86-6.82 (m, 1H),4.97-4.71 (m, 2H), 4.48-4.41 (m, 1H), 3.22-3.07 (m, 1H),2.96-2.87 (m, 1H),2.63-2.54 (m, 1H), 2.41-2.27 (m, 1H),2.13-1.80 (m, 5H), 1.41-1.39 (m, 6H).

Example 47: Preparation of (1*R*,2*R*)-2-(3-(2-((1-methyl-1*H*-pyrazol-4-yl)amino) pyrimidin-4-yl)-8-azabicyclo[3.2.1]oct-2-ene-8-formyl)cyclopropane-1-carbonitrile (47)

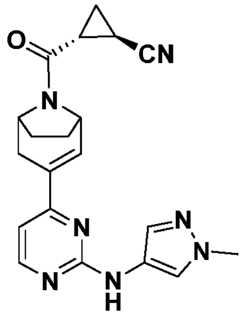

**47**

The same preparation method as that in Example 1 was used to give the title compound **47,** except that (1*R*,2*R*)-2-cyanocyclopropane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 10-60%; wavelength: 230 nm; flow rate: 45 mL/min; mobile phase: acetonitrile, 0.05% aqueous solution of formic acid.
LC-MS: m/z 376 $[M+H]^+$.
$^1H$ NMR (400 MHz, DMSO-$d_6$):δppm9.37 (s, 1H),8.35-8.33 (m, 1H), 7.84-7.82 (m, 1H), 7.50 (s,1H), 7.26-7.18 (m, 1H), 6.86-6.81 (m, 1H),5.10-4.97 (m, 1H), 4.84-4.73 (m, 1H),3.80 (s, 3H), 3.01-2.83 (m, 2H), 2.41-2.27 (m, 1H), 2.09-1.83 (m, 3H), 1.79-1.68 (m, 1H), 1.45-1.22 (m, 3H).

Example 48: Preparation of (2,2-difluoro-1-methylcyclopropyl)(3-(2-((1-methyl-1*H*-pyrazol-4-yl)amino)pyrimidin-4-yl)-8-azabicyclo[3.2.1]oct-2-en-8-yl)methanone (48)

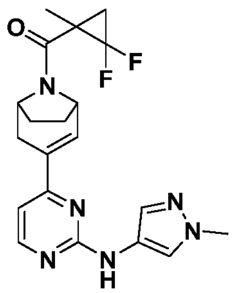

**48**

The same preparation method as that in Example 1 was used to give the title compound **48,** except that 2,2-difluoro-1-methylcyclopropane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 30-70%; wavelength: 230 nm; flow rate: 45 mL/min; mobile phase: acetonitrile, 0.05% aqueous solution of formic acid.
LC-MS: m/z 401 $[M+H]^+$.
$^1H$ NMR (300 MHz, DMSO-$d_6$):δppm9.36 (s,1H), 8.36-8.34 (m, 1H), 7.85-7.83 (m, 1H), 7.50 (s, 1H), 7.30-7.18 (m1H),6.84-6.83 (m, 1H), 4.96-4.79 (m, 1H),4.85-4.47 (m, 1H), 3.80 (s, 3H),2.99-2.61 (m, 2H), 2.14-2.02 (m, 2H),1.97-1.86 (m, 1H),1.80-1.70 (m, 2H), 1.59-1.52 (m, 1H), 1.45-1.33 (m, 3H).

Example 49: Preparation of (3-(2-((1-methyl-1*H*-pyrazol-4-yl)amino)pyrimidin-4-yl)-8-azabicyclo[3.2.1]oct-2-en-8-yl)(2-(trifluoromethyl)cyclopropyl)methanone (49)

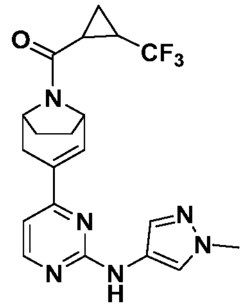

**49**

The same preparation method as that in Example 1 was used to give the title compound 49, except that 2-(trifluoromethyl)cyclopropane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-70%; wavelength: 230 nm; flow rate: 45 mL/min; mobile phase: acetonitrile, 0.05% aqueous solution of formic acid.

LC-MS: m/z 419 $[M+H]^+$.

$^1$H NMR (300 MHz, DMSO-$d_6$): δppm9.35 (s,1H), 8.35-8.33 (m, 1H), 7.82(s, 1H), 7.51-7.50 (m, 1H), 7.25-7.18 (m1H), 6.84-6.81 (m, 1H), 5.06-4.73 (m, 2H),3.80 (s 3H),2.95-2.90 (m, 1H),2.60-2.53 (m, 1H), 2.42-2.21 (m, 2H), 2.12-2.06 (m, 2H),1.94-1.66 (m, 2H), 1.29-1.07 (m, 2H).

Example 50: Preparation of (2-chloro-2-fluorocyclopropyl)(3-(2-((1-methyl-1*H*-pyrazol-4-yl)amino)pyrimidin-4-yl)-8-azabicyclo[3.2.1]oct-2-en-8-yl)methanone (50)

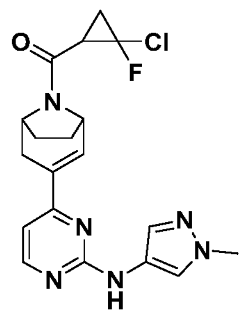

**50**

The same preparation method as that in Example 1 was used to give the title compound **50,** except that 2-chloro-2-fluorocyclopropane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-70%; wavelength: 230 nm; flow rate: 45 mL/min; mobile phase: acetonitrile, 0.05% aqueous solution of formic acid.

LC-MS: m/z 403 $[M+H]^+$.

$^1$H NMR (300 MHz, DMSO-d6) δppm9.36 (s, 1H), 8.36-8.27 (m, 1H), 7.85-7.83(m, 1H), 7.51 (d, *J* = 5.1 Hz, 1H), 7.30-7.14 (m, 1H), 6.88- 6.77 (m, 1H), 4.97-4.66 (m, 2H), 3.81 (d, *J*= 3.6 Hz, 3H), 3.19-2.83 (m, 2H), 2.45-1.59 (m, 7H).

Example 51: Preparation of (2-fluorocyclopropyl)(3-(2-((1-methyl-1*H*-pyrazol-4-yl) amino)pyrimidin-4-yl)-8-azabicyclo[3.2.1]oct-2-en-8-yl)methanone (51)

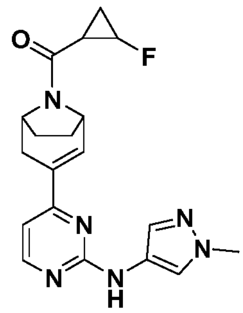

**51**

The same preparation method as that in Example 1 was used to give the title compound **51,** except that 2-fluorocyclopropane-1-carboxylic acid was used instead of (*S*)-2,2-difluorocyclopropane-1-carboxylic acid (**1j**).

Preparation method: column: 30 mm × 250 mm; packing: C18, 10 μm; process: 0-22 min, acetonitrile 10-60%; wavelength: 230 nm; flow rate: 45 mL/min; mobile phase: acetonitrile, 0.05% aqueous solution of formic acid.

LC-MS: m/z 369 $[M+H]^+$.

1H NMR (300 MHz, DMSO-$d_6$) δppm9.40-9.34 (m, 1H), 8.4-8.28 (m, 1H), 7.83-7.75 (m, 1H), 7.60-7.45 (m, 1H), 7.35-7.15 (m, 1H), 6.87-6.75 (m, 1H), 5.15-4.60 (m, 3H), 3.81 (s, 3H), 3.00-2.75 (m, 1H), 2.32-1.43 (m, 7H), 1.11-1.00 (m, 1H).

Example 52: Preparation of (3-(2-((1-methyl-1*H*-pyrazol-4-yl)amino)pyrimidin-4-yl)-8-azabicyclo[3.2.1]oct-2-en-8-yl)(1-(trifluoromethyl)cyclopropyl)methanone (52)

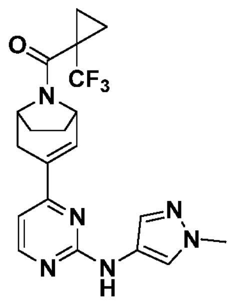

**52**

The same preparation method as that in Example 1 was used to give the title compound **52,** except that 1-(trifluoromethyl)cyclopropane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-65%; wavelength: 230 nm; flow rate: 45 mL/min; mobile phase: acetonitrile, aqueous solution of 0.05% formic acid.

LC-MS: m/z 419 $[M+H]^+$.

$^1$H NMR (300 MHz, DMSO-$d_6$) δppm9.35 (s, 1H), 8.35 (d, *J*= 5.2 Hz, 1H), 7.83 (s, 1H), 7.50 (s, 1H), 7.20 (d, *J* = 5.5 Hz, 1H), 6.82 (d, *J* = 5.2 Hz, 1H), 4.96-4.85 (m, 1H), 4.83-4.71 (m, 1H), 3.81 (s, 3H), 3.00-2.85 (m, 1H), 2.49-2.39 (m, 1H), 2.20-1.80 (m, 3H), 1.79-1.62 (m, 1H), 1.34-1.10 (m, 4H).

Example 53: Preparation of 1-(3-(2-((1-methyl-1*H*-pyrazol-4-yl)amino)pyrimidin-4-yl)-8-azabicyclo[3.2.1]oct-2-en-8-yl)but-3-yn-1-one (**53**)

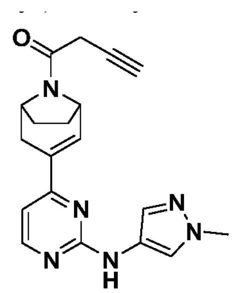

**53**

The same preparation method as that in Example 1 was used to give the title compound **53,** except that 3-butynoic acid was used instead of (*S*)-2,2-difluorocyclopropane-1-carboxylic acid (**1j**).

Purification method by preparative liquid chromatography: column: 30 mm × 250 mm; packing: C18, 20 μm; process: 0-22 min, acetonitrile 10-60%; wavelength: 230 nm; flow rate: 45 mL/min; mobile phase: acetonitrile, 0.05% aqueous solution of formic acid.

LC-MS: m/z 349 $[M+H]^+$.

$^1H$ NMR (300 MHz, DMSO-$d_6$) δppm9.34 (s, 1H), 8.34 (d, *J*= 5.2 Hz, 1H), 7.82 (s, 1H), 7.50 (d, *J*= 6.4 Hz, 1H), 7.21 (d, *J*= 5.3 Hz, 1H), 6.83-6.80 (m, 1H), 6.35-6.19 (m, 1H), 5.37-5.19 (m, 2H), 4.95-4.66 (m, 2H), 3.85-3.73 (m, 3H), 3.00-2.88 (m, 1H), 2.28-1.59 (m, 5H).

Example 54: Preparation of *N*-(1-cyanocyclopropyl)-3-(2-((1-methyl-1*H*-pyrazol-4-yl)amino)pyrimidin-4-yl)-8-azabicyclo[3.2.1]oct-2-ene-8-carboxamide **(54)**

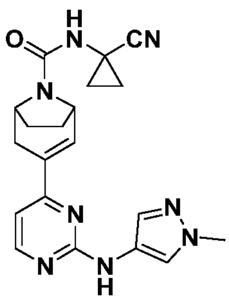

**54**

The same preparation method as that in Example 2 was used to give the title compound **54,** except that 1-aminocyclopropane-1-carbonitrile hydrochloride was used instead of 2-aminoacetonitrile **(2a).**

Purification method by preparative liquid chromatography: column: 30 mm × 250 mm; packing: C18, 10 μm; process: 0-22 min, acetonitrile 10-60%; wavelength: 230 nm; flow rate: 45 mL/min; mobile phase: acetonitrile, 0.05% aqueous solution of formic acid.

LC-MS: m/z 391 $[M+H]^+$.

$^1H$ NMR (300 MHz, DMSO-$d_6$):δppm9.33 (s,1H), 8.33 (d, *J*= 3.0 Hz,1H),7.81 (s, 1H),7.63 (s, 1H), 7.51 (s,1H),7.20-7.17 (m, 1H),6.81(d, *J* = 3.0 Hz,1H), 4.57-4.44 (m, 2H), 3.81 (s, 3H), 2.94-2.88 (m, 1H), 2.33-2.27 (m, 1H),2.16-2.07 (m, 1H), 1.93-1.88 (m, 2H), 1.68-1.59 (m, 1H), 1.41-1.37 (m, 2H), 1.09-1.04 (m, 2H).

Example 55: Preparation of 3-(2-((1-methyl-1*H*-pyrazol-4-yl)amino)pyrimidin-4-yl)-*N*-((*S*)-1,1,1-trifluoropropan-2-yl)-8-azabicyclo[3.2.1]oct-2-ene-8-carboxamide **(55)**

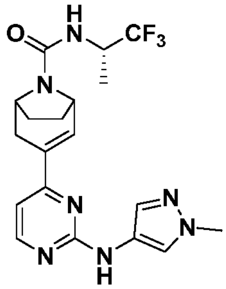

55

The same preparation method as that in Example 2 was used to give the title compound **55,** except that (*S*)-1,1,1-trifluoropropane-2-amine hydrochloride was used instead of 2-aminoacetonitrile **(2a).**

Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.

LC-MS: m/z 422[M+H]+.

$^1$H NMR (300 MHz, DMSO-d6) δppm9.32 (s, 1H), 8.33 (d, *J* = 5.2 Hz, 1H), 7.81 (s, 1H), 7.52 (s, 1H), 7.20 (s, 1H), 7.00 (t, *J* = 8.9 Hz, 1H), 6.81 (d, *J* = 5.2 Hz, 1H), 4.79-4.31 (m, 3H), 3.80 (s, 3H), 3.00-2.90 (m, 1H), 2.32-2.12 (m, 2H), 1.25-1.21 (m, 2H), 1.70-1.60 (m, 1H), 1.25-1.21 (m, 3H).

Example 56: Preparation of 3-(2-((1-methyl-1H-pyrazol-4-yl)amino)pyrimidin-4-yl)-*N*-((*R*)-1,1,1-trifluoropropan-2-yl)-8-azabicyclo[3.2.1]oct-2-ene-8-carboxamide (**56**)

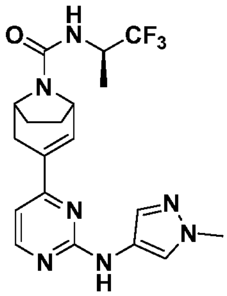

56

The same preparation method as that in Example 2 was used to give the title compound **56,** except that (*R*)-1,1,1-trifluoropropane-2-amine hydrochloride was used instead of 2-aminoacetonitrile **(2a).**

Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.

LC-MS: m/z 422 [M+H]+.

$^1$H NMR (300 MHz, DMSO-$d_6$): δppm9.32 (s,1H),8.31 (d, *J*= 3.0 Hz,1H),7.81 (s, 1H),7.52 (s, 1H),7.20 (s1H),7.00(t, *J* = 9.0 Hz,1H), 6.82-6.80 (m, 1H),4.66-4.47 (m, 3H), 3.80 (s, 3H),2.99-2.89 (m, 1H),2.32-2.26 (m, 1H),2.18-2.08 (m, 1H), 1.95-1.89 (m, 2H), 1.70-1.61 (m, 1H), 1.25-1.22 (m, 3H).

Example 57: Preparation of 3-(2-((1-methyl-1*H*-pyrazol-4-yl)amino)pyrimidin-4-yl)-*N*-(3,3,3-trifluoropropyl)-8-azabicyclo[3.2.1]oct-2-ene-8-carboxamide **(57)**

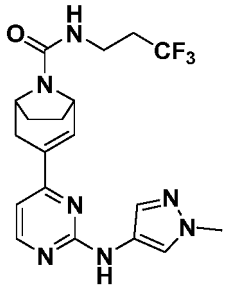

57

The same preparation method as that in Example 2 was used to give the title compound **57,** except that 3,3,3-trifluoropropan-1-amine hydrochloride was used instead of 2-aminoacetonitrile **(2a).**

Purification method by preparative liquid chromatography: column: 30 mm × 250 mm; packing: C18, 10 μm; process: 0-22 min, acetonitrile 10-55%; wavelength: 230 nm; flow rate: 45 mL/min; mobile phase: acetonitrile, 0.05% aqueous solution of formic acid.

LC-MS: m/z 422 $[M+H]^+$.

$^1H$ NMR (300 MHz, DMSO-d6) δppm9.32 (s, 1H), 8.32 (d, *J* = 5.2 Hz, 1H), 7.81 (s, 1H), 7.52 (s, 1H), 7.21 (d, *J* = 5.2 Hz, 1H), 6.90-6.75 (m, 2H), 4.54-4.46 (m, 2H), 3.81 (s, 3H), 3.29-3.12 (m, 3H), 2.98-2.92 (m, 1H), 2.49-2.37 (m, 2H), 2.30-2.06 (m, 1H), 1.90-1.87 (m, 2H), 1.66-1.62 (m, 1H).

Example 58: Preparation of 3-(2-((1-methyl-1H-pyrazol-4-yl)amino)pyrimidin-4-yl)-*N*-(2-(trifluoromethoxy)ethyl)-8-azabicyclo[3.2.1]oct-2-ene-8-carboxamide **(58)**

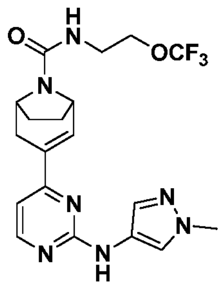

58

The same preparation method as that in Example 2 was used to give the title compound **58,** except that 2-(trifluoromethoxy)ethane-1-amine hydrochloride was used instead of 2-aminoacetonitrile **(2a).**

Purification method by preparative liquid chromatography: column: 30 mm × 250 mm; packing: C18, 10 μm; process: 0-22 min, acetonitrile 10-60%; wavelength: 230 nm; flow rate: 45 mL/min; mobile phase: acetonitrile, 0.05% aqueous solution of formic acid.

LC-MS: m/z 438 $[M+H]^+$.

$^1H$ NMR (300 MHz, DMSO) δppm 10.64 (s, 1H), 9.31 (s, 1H), 8.37-8.24 (m, 1H), 7.81 (s, 1H), 7.52 (s, 1H), 7.21-7.07 (m, 1H), 6.94-6.76 (m, 1H), 4.63-4.31 (m, 2H), 3.86-3.62 (m, 5H), 3.10-2.91 (m, 2H), 2.40-2.07 (m, 3H), 2.02-1.78 (m, 2H), 1.1.72-1.54 (m, 1H).

Example 59: Preparation of 3-(2-((1-methyl-1*H*-pyrazol-4-yl)amino)pyrimidin-4-yl)-8-azabicyclo[3.2.1]oct-2-en-8-yl)(2-phenylcyclopropyl)methanone **(59)**

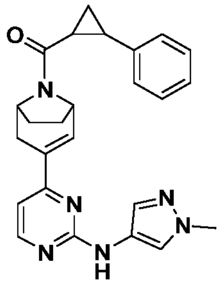

**59**

The same preparation method as that in Example 1 was used to give the title compound **59,** except that 2-phenylcyclopropane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 30-70%; wavelength: 230 nm; flow rate: 45 mL/min; mobile phase: acetonitrile, 0.05% aqueous solution of formic acid.
LC-MS: m/z 427 $[M+H]^+$.
$^1H$ NMR (300 MHz, DMSO-$d_6$): δppm9.33-9.31 (m,1H), 8.33 (dd, *J* = 6.0 Hz, 3.0 Hz,1H), 7.82-7.78 (m, 1H), 7.52-7.46 (m, 1H),7.31-7.10 (m6H),6.80(dd, *J* = 12.0 Hz, 3.0 Hz,1H), 4.97-4.75 (m, 2H), 3.81-3.75 (m, 3H), 3.02-2.91 (m, 1H),2.37-2.18 (m, 3H), 2.12-1.86 (m, 3H),1.74-1.59 (m, 1H), 1.45-1.18 (m, 2H).

Example 60: Preparation of 2-methyl-3-(3-(2-((1-methyl-1*H*-pyrazol-4-yl)amino) pyrimidin-4-yl)-8-azabicyclo[3.2.1]oct-2-en-8-yl)-3-oxopropionitrile **(60)**

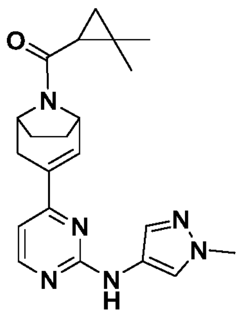

**60**

The same preparation method as that in Example 1 was used to give the title compound **60,** except that 2,2-dimethylcyclopropane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 25-65%; wavelength: 230 nm; flow rate: 45 mL/min; mobile phase: acetonitrile, 0.05% aqueous solution of formic acid.
LC-MS: m/z 379 $[M+H]^+$.
$^1H$ NMR (300 MHz, DMSO-$d_6$): δppm9.34 (s,1H), 8.35-8.31 (m, 1H), 7.82 (s, 1H), 7.51 (s, 1H), 7.32-7.16 (m1H),6.84-6.80 (m, 1H), 4.91-4.61 (m, 2H),3.81-3.78 (m, 3H), 2.98-2.85 (m, 1H),2.41-2.26 (m, 1H), 2.10-2.02 (m, 1H), 1.97-1.16 (m, 3H), 1.70-1.60 (m, 1H), 1.19-1.12 (m, 3H), 1.02-0.98 (m, 1H), 0.92-0.88 (m, 1H), 0.81 (s, 1H), 0.73(s, 1H), 0.67-0.58 (m, 1H).

Example 61: Preparation of 3-(3-(2-((1-methyl-1*H*-pyrazol-4-yl)amino)pyrimidin-4-yl)-8-azabicyclo[3.2.1]oct-2-en-8-yl)-3-oxopropionitrile **(61)**

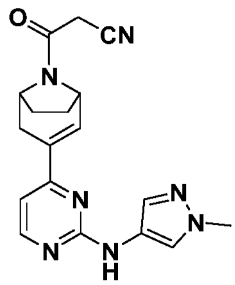

**61**

The same preparation method as that in Example 1 was used to give the title compound **61,** except that 2-cyanoacetic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.

LC-MS: m/z 350 $[M+H]^+$.

$^1H$ NMR (300 MHz, DMSO-$d_6$): δppm9.33 (s,1H), 8.34 (d, *J* = 3.0 Hz,1H),7.83-7.81 (m, 1H), 7.50 (s, 1H), 7.20-7.17 (m1H), 6.81 (d, *J* = 3.0 Hz,1H), 4.89-4.72 (m, 1H), 4.60-4.45 (m, 1H), 4.12-3.99 (m, 2H), 3.81 (s, 3H),3.09-2.84 (m, 2H),2.23-2.00 (m, 2H), 1.91-1.63 (m, 2H).

Example 62: Preparation of 2-methyl-3-(3-(2-((1-methyl-1*H*-pyrazol-4-yl)amino) pyrimidin-4-yl)-8-azabicyclo[3.2.1]oct-2-en-8-yl)-3-oxopropionitrile **(62)**

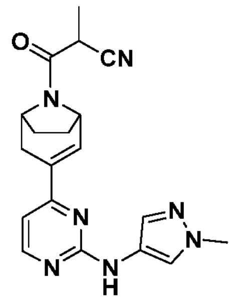

**62**

The same preparation process as that in Example 1 was used to give the title compound **62,** except that 2-cyanopropionic acid was used instead of (*S*)-2,2-difluorocyclopropane-1-carboxylic acid (**1j**).

Purification process by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.

LC-MS: m/z 364 $[M+H]^+$.

$^1H$ NMR (300 MHz, DMSO-$d_6$): δppm9.35 (s,1H), 8.36-8.34 (m, 1H), 7.85-7.81 (m, 1H), 7.50 (s, 1H), 7.23-7.17 (m1H),6.85-6.81 (m, 1H),4.94-4.70 (m, 2H),4.39-4.27 (m, 1H),3.80 (s, 3H),3.17-2.83 (m, 2H), 2.28-2.06 (m, 2H), 1.93-1.66 (m, 2H),1.42-1.31 (m, 3H).

Example 63: Preparation of 1-(3-(2-((1-methyl-1*H*-pyrazol-4-yl)amino)pyrimidin-4-yl)-8-azabicyclo[3.2.1]oct-2-ene-8-formyl)cyclopropane-1-carbonitrile **(63)**

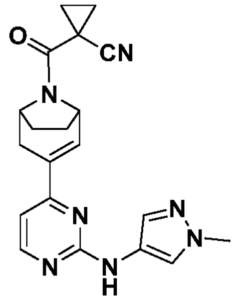

63

The same preparation method as that in Example 1 was used to give the title compound **63,** except that 1-cyanocyclopropane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.
LC-MS: m/z 376 $[M+H]^+$.
$^1H$ NMR (300 MHz, DMSO-$d_6$): δppm9.37 (s,1H), 8.35 (d, *J*= 3.0 Hz,1H),7.84 (s, 1H), 7.49 (s, 1H), 7.31-7.15 (m1H), 6.84 (d, *J* = 6.0 Hz,1H),5.10-4.69 (m, 2H), 3.80 (s, 3H), 3.17-2.72 (m, 2H), 2.27-1.98 (m, 3H),1.82-1.70 (m, 1H), 1.65-1.52 (m, 3H), 1.45-1.29 (m, 1H).

Example 64: Preparation of 4-(3-(2-((1-methyl-1*H*-pyrazol-4-yl)amino)pyrimidin-4-yl)-8-azabicyclo[3.2.1]oct-2-ene-8-carbonyl)tetrahydro-2*H*-pyran-4-cyano (64)

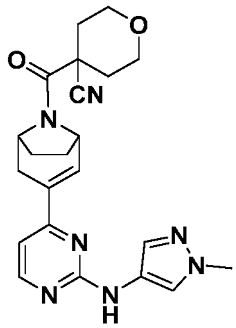

64

The same preparation method as that in Example 1 was used to give the title compound **64,** except that 4-cyanotetrahydro-2H-pyran-4-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.
LC-MS: m/z 420 $[M+H]^+$.
$^1H$ NMR (300 MHz, DMSO-$d_6$): δppm9.37 (s,1H), 8.35 (d, *J* = 3.0 Hz, 1H), 7.84 (s, 1H), 7.49 (s, 1H),7.26-7.20 (m1H), 6.85-6.79 (m, 1H),5.18-4.79 (m, 2H),4.00-3.72 (m, 5H),3.62-3.50 (m, 2H),3.05-2.88 (m, 1H), 2.41-2.22 (m, 1H),2.17-1.94 (m, 5H),1.91-1.77 (m, 2H),1.74-1.56 (m, 1H).

Example 65: Preparation of 3,3,3-trifluoro-1-(3-(2-((1-methyl-1*H*-pyrazol-4-yl) amino)pyrimidin-4-yl)-8-azabicyclo[3.2.1]oct-2-en-8-yl)-1-acetone (65)

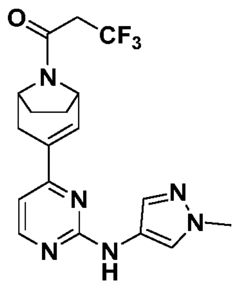

65

The same preparation method as that in Example 1 was used to give the title compound **65,** except that 3,3,3-trifluoropropionic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.

LC-MS: m/z 393 $[M+H]^+$.

$^1$H NMR (300 MHz, DMSO-$d_6$): δppm9.33 (s,1H), 8.34 (d, *J* = 3.0 Hz,1H), 7.83-7.81 (m, 1H), 7.51 (s, 1H), 7.21-7.17 (m1H), 6.83-6.81 (m, 1H), 4.93-4.63 (m, 2H),3.81 (s, 3H), 3.73-3.49 (m, 2H), 3.04-2.89 (m, 1H),2.47-2.32 (m, 1H), 2.24-2.04(m, 2H),1.96-1.84 (m, 1H), 1.76-1.63 (m, 1H).

Example 66: Preparation of 3,3-difluoro-1-(3-(2-((1-methyl-1*H*-pyrazol-4-yl) amino)pyrimidin-4-yl)-8-azabicyclo[3.2.1]oct-2-en-8-yl)-1-acetone **(66)**

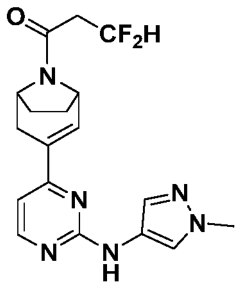

66

The same preparation method as that in Example 1 was used to give the title compound **66,** except that 3,3-difluoropropionic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.

LC-MS: m/z 375 $[M+H]^+$.

$^1$H NMR (300 MHz, DMSO-$d_6$) δppm 9.34 (s, 1H), 8.34 (d, *J* = 5.2 Hz, 1H), 7.82 (d, *J*= 5.0 Hz, 1H), 7.50 (s, 1H), 7.20 (s, 1H), 6.82 (d, *J*= 5.2 Hz, 1H), 6.51 - 6.06 (m, 1H), 4.91-4.61 (m, 2H), 3.81 (s, 3H), 3.26 - 3.06 (m, 2H), 3.02-2.95 (m, 1H), 2.49 - 2.34 (m, 1H), 2.22-2.05 (m, 1H), 2.02-1.92 (m, 2H), 1.70 -1.61(m, 1H).

Example 67: Preparation of 3,3,3-trifluoro-2-methyl-1-(3-(2-((1-methyl-1*H*-pyrazol-4-yl)amino)pyrimidin-4-yl)-8-azabicyclo[3.2.1]oct-2-en-8-yl)-1-acetone **(67)**

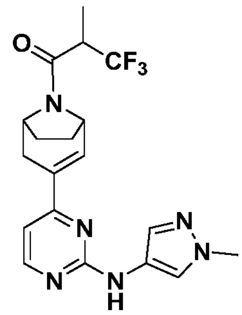

67

The same preparation method as that in Example 1 was used to give the title compound **67,** except that 3,3,3-trifluoro-2-methylpropionic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.

LC-MS: m/z 407 $[M+H]^+$.

$^1H$ NMR (300 MHz, DMSO-$d_6$) $\delta$ppm9.35 (s, 1H), 8.35 (d, *J* = 5.2 Hz, 1H), 7.82-7.81 (m, 1H), 7.51 (s, 1H), 7.19-7.17 (m, 1H), 6.84-6.82 (m, 1H), 4.95-4.70 (m, 2H), 4.04-3.88 (m, 1H), 3.81 (s, 3H), 2.95-2.89 (m, 1H), 2.45-2.39 (m, 1H), 2.22-1.66 (m, 4H), 1.30-1.16 (m, 3H).

Example 68: Preparation of 3,3,3-trifluoro-2,2-dimethyl-1-(3-(2-((1-methyl-1*H*-pyrazol-4-yl)amino)pyrimidin-4-yl)-8-azabicyclo[3.2.1]oct-2-en-8-yl)acetone **(68)**

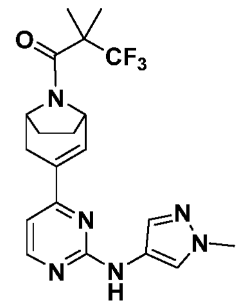

68

The same preparation method as that in Example 1 was used to give the title compound **68,** except that 3,3,3-trifluoro-2,2-dimethylpropionic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 30-70%; wavelength: 230 nm; flow rate: 45 mL/min; mobile phase: acetonitrile, 0.05% aqueous solution of formic acid.

LC-MS: m/z 421 $[M+H]^+$.

$^1H$ NMR (300 MHz, DMSO-$d_6$) $\delta$ppm9.37 (s, 1H), 8.35 (d, *J*= 5.2 Hz, 1H), 7.82 (s, 1H), 7.51 (s, 1H), 7.22 (s, 1H), 6.82 (d, *J*= 5.2 Hz, 1H), 5.02-4.84 (m, 2H), 3.79 (s, 3H), 2.99-2.94 (m, 1H), 2.48-2.46 (m, 1H), 2.18 - 1.94 (m, 3H), 1.69-1.65 (m, 1H), 1.46-1.42 (m, 6H).

Example 69: Preparation of 1-(3-(2-((1-methyl-1*H*-pyrazol-4-yl)amino)pyrimidin-4-yl)-8-azabicyclo[3.2.1]oct-2-en-8-yl)-2-(2,2,2-trifluoroethoxy)-1-one **(69)**

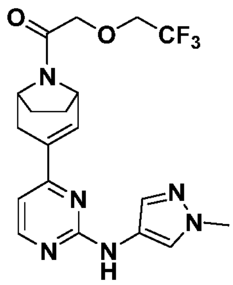

69

The same preparation method as that in Example 1 was used to give the title compound **69,** except that 2-(2,2,2-trifluoroethoxy)acetic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.
LC-MS: m/z 423 $[M+H]^+$.
$^1H$ NMR (300 MHz, DMSO-$d_6$):δppm9.34 (s,1H), 8.35-8.33 (m, 1H),7.83-7.82 (m, 1H), 7.50 (s, 1H),7.21-7.17 (m1H), 6.82-6.80 (m, 1H), 4.91-4.75 (m, 1H), 4.62-4.51 (m, 1H), 4.43-4.27 (m, 2H), 4.22-4.08 (m, 2H), 3.81 (s, 3H), 3.01-2.91 (m, 1H), 2.45-2.37 (m, 1H), 2.26-2.10 (m, 1H), 2.04-1.92 (m, 2H), 1.77-1.62 (m, 1H).

Example 70: Preparation of (3,3-difluorocyclopentyl)(3-(2-((1-methyl-1*H*-pyrazol-4-yl)amino)pyrimidin-4-yl)-8-azabicyclo[3.2.1]oct-2-en-8-yl)methanone **(70)**

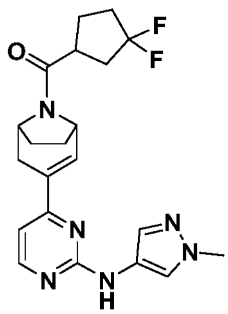

70

The same preparation method as that in Example 1 was used to give the title compound **70,** except that 3,3-difluorocyclopentane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 2-22 min, acetonitrile 30-70%; wavelength: 230 nm; flow rate: 45 mL/min; mobile phase: acetonitrile, 0.05% aqueous solution of formic acid.
LC-MS: m/z 415 $[M+H]^+$.
$^1H$ NMR (300 MHz, DMSO-$d_6$) δppm 9.34 (s, 1H), 8.34 (d, *J* = 5.3 Hz, 1H), 7.83 (s, 1H), 7.51 (s, 1H), 7.21 (s, 1H), 6.81 (d, *J*= 5.2 Hz, 1H), 4.94- 4.63 (m, 2H), 3.81 (s, 3H), 3.32-3.20 (m, 1H), 2.94-2.88 (m, 1H), 2.41-2.29 (m, 3H), 2.20-2.07 (m, 5H), 1.93-1.81 (m, 1H), 1.81-1.54 (m, 2H).

Example 71: Preparation of (3-(2-((1-methyl-1*H*-pyrazol-4-yl)amino)pyrimidin-4-yl)-8-azabicyclo[3.2.1]oct-2-en-8-yl)(3-(trifluoromethyl)bicyclo[1.1.1]pentan-1-yl)me thanone **(71)**

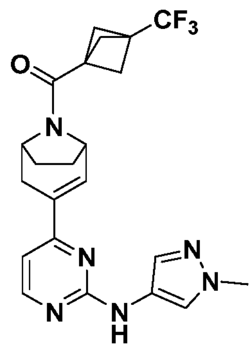

71

The same preparation method as that in Example 1 was used to give the title compound **71,** except that 3-(trifluoromethyl)bicyclo[1.1.1]pentane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 30-70%; wavelength: 230 nm; flow rate: 45 mL/min; mobile phase: acetonitrile, 0.05% aqueous solution of formic acid.

LC-MS: m/z 445 $[M+H]^+$.

$^1$H NMR (300 MHz, DMSO-$d_6$) $\delta$ppm 9.34 (s, 1H), 8.34 (d, $J$ = 5.0 Hz, 1H), 7.82 (d, $J$ = 3.6 Hz, 1H), 7.50 (s, 1H), 7.22-7.17 (m, 1H), 6.82-6.64 (m, 1H), 4.87-4.71 (m, 2H), 3.80 (s, 3H), 2.99-2.78 (m, 1H), 2.42-2.22 (m, 7H), 2.12-1.92 (m, 2H), 1.92-1.57 (m, 2H).

Example 72: Preparation of bicyclo[1.1.1]pentan-1-yl(3-(2-((1-methyl-1*H*-pyrazol-4-yl)amino)pyrimidin-4-yl)-8-azabicyclo[3.2.1]oct-2-en-8-yl)methanone (**72**)

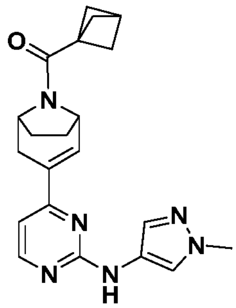

72

The same preparation method as that in Example 1 was used to give the title compound **72,** except that bicyclo[1.1.1]pentane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 20-60%; wavelength: 230 nm; flow rate: 45 mL/min; mobile phase: acetonitrile, 0.05% aqueous solution of formic acid.

LC-MS: m/z 377 $[M+H]^+$.

$^1$H NMR (300 MHz, DMSO-$d_6$) $\delta$ppm 9.34 (s, 1H), 8.33 (d, $J$ = 5.2 Hz, 1H), 7.82 (d, $J$ = 4.0 Hz, 1H), 7.50 (s, 1H), 7.24-7.17 (m, 1H), 6.83-6.62 (m, 1H), 4.89-4.68 (m, 2H), 3.80 (s, 3H), 2.94-2.82 (m, 1H), 2.55-2.51 (m, 1H), 2.45-2.43 (m, 1H), 2.38-2.19 (m, 1H), 2.15-2.00 (m, 7H), 1.98-1.56 (m, 2H).

Example 73: Preparation of 3-(3-(2-((1-methyl-1*H*-pyrazol-4-yl)amino)pyrimidin-4-yl)-8-azabicyclo[3.2.1]oct-2-ene-8-formyl)bicyclo[1.1.1]pentane-1-carbonitrile **(73)**

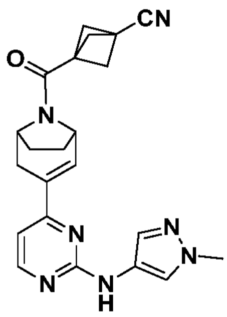

73

The same preparation method as that in Example 1 was used to give the title compound **73,** except that 3-cyanobicyclo[1.1.1]pentane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 10-50%; wavelength: 230 nm; flow rate: 45 mL/min; mobile phase: acetonitrile, 0.05% aqueous solution of formic acid.

LC-MS: m/z 402 $[M+H]^+$.

$^1H$ NMR (300 MHz, DMSO-$d_6$) $\delta$ppm 9.35 (s, 1H), 8.34 (d, *J* = 5.2 Hz, 1H), 7.82 (d, *J* = 5.1 Hz, 1H), 7.50 (s, 1H), 7.20-7.16 (m, 1H), 6.80 (t, *J* = 5.5 Hz, 1H), 4.82-4.70 (m, 2H), 3.81 (s, 3H), 2.92-2.71 (m, 1H), 2.66-2.52 (m, 6H), 2.40-2.20 (m, 1H), 2.01-1.98 (m, 2H), 1.92-1.58 (m, 2H).

Example 74: Preparation of (3-fluorobicyclo[1.1.1]pentan-1-yl)(3-(2-((1-methyl-1*H*-pyrazol-4-yl)amino)pyrimidin-4-yl)-8-azabicyclo[3.2.1]oct-2-en-8-yl) methanone **(74)**

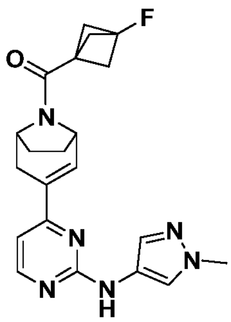

74

The same preparation method as that in Example 1 was used to give the title compound **74,** except that 3-fluorobicyclo[1.1.1]pentane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 20-60%; wavelength: 230 nm; flow rate: 45 mL/min; mobile phase: acetonitrile, 0.05% aqueous solution of formic acid.

LC-MS: m/z 395 $[M+H]^+$.

$^1H$ NMR (300 MHz, DMSO-$d_6$) $\delta$ppm 9.34 (s, 1H), 8.34 (d, *J* = 5.2 Hz, 1H), 7.82 (d, *J* = 5.3 Hz, 1H), 7.50 (s, 1H), 7.20-7.18 (m, 1H), 6.84-6.76 (m, 1H), 4.90-4.63 (m, 2H), 3.80 (s, 3H), 2.96-2.80 (m, 1H), 2.49-2.38 (m, 6H), 2.33-2.26 (m, 1H), 2.19-1.82 (m, 2H), 1.79-1.63 (m,2H).

Example 75: Preparation of 2-cyclopropyl-2,2-difluoro-1-(3-(2-((1-methyl-1*H*-pyrazol-4-yl)amino)pyrimidin-4-yl)-8-azabicyclo[3.2.1]oct-2-en-8-yl)ethan-1-one **(75)**

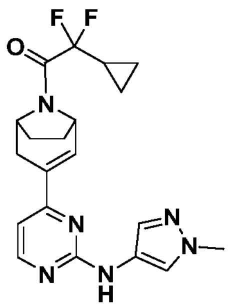

75

The same preparation method as that in Example 1 was used to give the title compound **75,** except that 2-cyclopropyl-2,2-difluoroacetic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 30-70%; wavelength: 230 nm; flow rate: 45 mL/min; mobile phase: acetonitrile, 0.05% aqueous solution of formic acid.

LC-MS: m/z 401 $[M+H]^+$.

1H NMR (300 MHz, DMSO-$d_6$) δppm9.36 (s, 1H), 8.35 (d, *J* = 5.2 Hz, 1H), 7.82 (s, 1H), 7.50 (s, 1H), 7.22 (s, 1H), 6.83 (t, *J*= 4.8 Hz, 1H), 4.97-4.83 (m, 2H), 3.81 (s, 3H), 2.97-2.83 (m, 1H), 2.62-2.56 (m, 1H), 2.16-1.81 (m, 3H), 1.80-1.52 (m, 2H), 0.74-0.56 (m, 4H).

Example 76: Preparation of (3-(2-((1-methyl-1*H*-pyrazol-4-yl)amino)pyrimidin-4-yl)-8-azabicyclo[3.2.1]oct-2-en-8-yl)(tetrahydrofuran-3-yl)methanone **(76)**

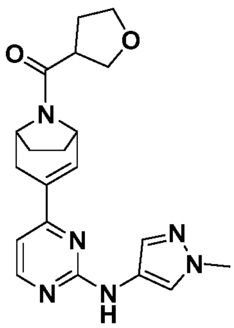

76

The same preparation method as that in Example 1 was used to give the title compound **76,** except that tetrahydrofuran-3-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 10-50%; wavelength: 230 nm; flow rate: 45 mL/min; mobile phase: acetonitrile, 0.05% aqueous solution of formic acid.

LC-MS: m/z 381 $[M+H]^+$.

$^1$H NMR (300 MHz, DMSO-$d_6$) δppm9.33 (s, 1H), 8.34 (d, *J* = 5.2 Hz, 1H), 7.83-7.81 (m, 1H), 7.50 (s, 1H), 7.21-7.19 (m, 1H), 6.88-6.75 (m, 1H), 4.92-4.62 (m, 2H), 3.96-3.78 (m, 4H), 3.76-3.59 (m, 3H), 2.93-2.91 (m, 1H), 2.37-2.35 (m, 1H), 2.30-1.55 (m, 7H).

Example 77: Preparation of 2-(3-(2-((1-methyl-1*H*-pyrazol-4-yl)amino)pyrimidin-4-yl)-8-azabicyclo[3.2.1]oct-2-ene-8-formyl)cyclobutane-1-carbonitrile **(77)**

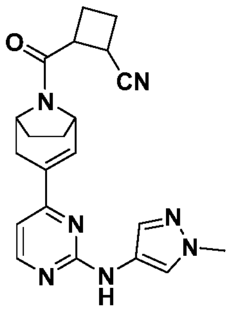

77

The same preparation method as that in Example 1 was used to give the title compound **77,** except that 2-cyanocyclobutane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 10-50%; wavelength: 230 nm; flow rate: 45 mL/min; mobile phase: acetonitrile, 0.05% aqueous solution of formic acid.

LC-MS: m/z 390 $[M+H]^+$.

$^1H$ NMR (300 MHz, DMSO-$d_6$) $\delta$ppm9.34 (s, 1H), 8.35-8.32 (m, 1H), 7.89-7.77 (m, 1H), 7.49 (d, *J*= 2.2 Hz, 1H), 7.20-7.16 (m, 1H), 6.86-6.75 (m, 1H), 4.89-4.45 (m, 2H), 3.90-3.75 (m, 4H), 3.59 -3.43 (m, 1H), 2.95-2.90 (m, 1H), 2.42-2.36 (m, 1H), 2.25 -1.64 (m, 8H).

Example 78: Preparation of 3-(2-((1-methyl-1*H*-pyrazol-4-yl)amino)pyrimidin-4-yl)-8-azabicyclo[3.2.1]oct-2-en-8-yl)(tetrahydro-2*H*-pyran-4-yl)methanone **(78)**

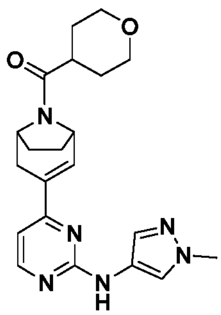

78

The same preparation method as that in Example 1 was used to give the title compound **78,** except that tetrahydropyran-4-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 2-22 min, acetonitrile 10-40%; wavelength: 230 nm; flow rate: 45 mL/min; mobile phase: acetonitrile, 0.05% aqueous solution of formic acid.

LC-MS: m/z 395 $[M+H]^+$.

$^1H$ NMR (300 MHz, DMSO-$d_6$) $\delta$ppm9.33 (s, 1H), 8.33 (d, *J* = 5.2 Hz, 1H), 7.83-7.80 (m, 1H), 7.51 (s, 1H), 7.24-7.20 (m, 1H), 6.81 (d, *J* = 5.2 Hz, 1H), 4.89-4.66 (m, 2H), 3.86-3.80 (m, 5H), 2.92-2.78(m, 3H), 2.40-2.17 (m, 1H), 2.08-2.00 (m, 2H), 1.92 -1.34 (m, 7H).

Example 79: Preparation of 2-(3,3-difluorocyclobutyl)-1-(3-(2-((1-methyl-1*H*-pyrazol-4-yl)amino)pyrimidin-4-yl)-8-azabicyclo[3.2.1]oct-2-en-8-yl)-1-acetone **(79)**

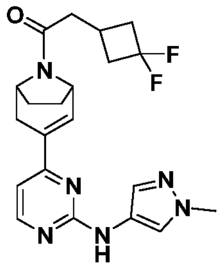

**79**

The same preparation method as that in Example 1 was used to give the title compound **79,** except that 2-(3,3-difluorocyclobutyl)acetic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 20-60%; wavelength: 230 nm; flow rate: 45 mL/min; mobile phase: acetonitrile, 0.05% aqueous solution of formic acid.
LC-MS: m/z 415 $[M+H]^+$.
$^1$H NMR (300 MHz, DMSO-$d_6$) δppm9.34 (s, 1H), 8.34 (d, *J* = 5.2 Hz, 1H), 7.83-7.81 (m, 1H), 7.51 (s, 1H), 7.25-7.15 (m, 1H), 6.82-6.80 (m, 1H), 4.88-4.52 (m, 2H), 3.81 (s, 3H), 2.95-2.78(m, 1H),2.75-2.55 (m, 4H), 2.41-2.14 (m, 4H), 2.15-1.95 (m, 2H), 1.94-1.78 (m, 1H), 1.79-1.61 (m, 1H).

Example 80: Preparation of (3-methoxycyclobutyl)(3-(2-((1-methyl-1*H*-pyrazol-4-yl)amino)pyrimidin-4-yl)-8-azabicyclo[3.2.1]oct-2-en-8-yl)methanone **(80)**

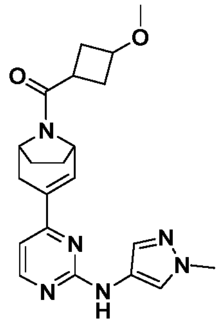

**80**

The same preparation method as that in Example 1 was used to give the title compound **80,** except that 3-methoxycyclobutane-1-carboxylic acid was used instead of (*S*)-2,2-difluorocyclopropane-1-carboxylic acid (**1j**).
Purification method by preparative liquid chromatography: column: 30 mm × 250 mm; packing: C18, 10 μm; process: 0-22 min, acetonitrile 10-50%; wavelength: 220 nm; flow rate: 45 mL/min; mobile phase: acetonitrile, 0.05% aqueous solution of formic acid.
LC-MS: m/z 395 $[M+H]^+$.
$^1$H NMR (300 MHz, DMSO-$d_6$) δppm9.33 (s, 1H), 8.33 (d, *J* = 5.2 Hz, 1H), 7.87-7.76 (m, 1H), 7.57-7.41 (m, 1H), 7.22-7.14 (m, 1H), 6.89-6.68 (m, 1H), 4.90-4.43 (m, 2H), 3.88-3.68 (m, 4H), 3.13-3.05 (m, 3H), 2.89-2.85 (m, 2H), 2.40-2.29 (m, 2H), 2.25-1.58 (m, 7H).

Example 81: Preparation of (3-(hydroxymethyl)cyclobutyl)(3-(2-((1-methyl-1*H*-pyrazol-4-yl)amino)pyrimidin-4-yl)-8-azabicyclo[3.2.1]oct-2-en-8-yl)methanone (**81**)

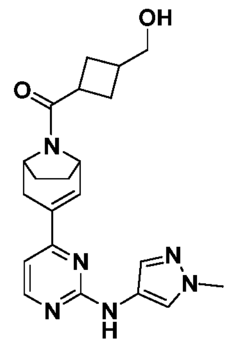

81

The same preparation method as that in Example 1 was used to give the title compound **81,** except that 3-(hydroxymethyl)cyclobutane-1-carboxylic acid was used instead of (*S*)-2,2-difluorocyclopropane-1-carboxylic acid (**1j**).

Purification method by preparative liquid chromatography: column: 30 mm × 250 mm; packing: C18, 10 μm; process: 0-22 min, acetonitrile 10-50%; wavelength: 220 nm; flow rate: 45 mL/min; mobile phase: acetonitrile, 0.05% aqueous solution of formic acid.

LC-MS: m/z 395 $[M+H]^+$.

$^1$H NMR (300 MHz, DMSO-$d_6$) δppm9.34 (s, 1H), 8.33 (d, *J* = 5.2 Hz, 1H), 7.85-7.80 (m, 1H), 7.50 (s, 1H), 7.22-7.15 (m, 1H), 6.82-6.79 (m, 1H), 4.86-4.40 (m, 2H), 3.81 (s, 3H), 3.29-3.10 (m, 3H), 3.03-2.77 (m, 2H), 2.42-1.54 (m, 10H).

Example 82: Preparation of 2-(3-(2-((1-methyl-1*H*-pyrazol-4-yl)amino)pyrimidin-4-yl)-8-azabicyclo[3.2.1]oct-2-en-8-yl)-2-oxo-*N*-(2,2,2-trifluoroethyl)acetamide (82)

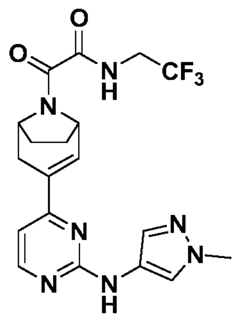

82

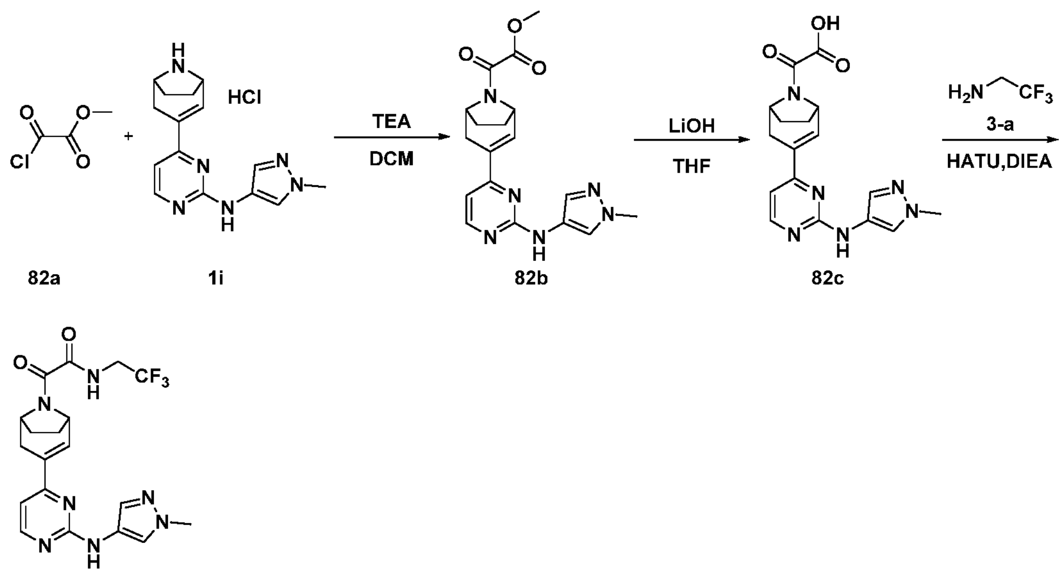


82a 1i 82b 82c

82

Step 1: Preparation of methyl 2-(3-(2-((1-methyl-1*H*-pyrazol-4-yl)amino) pyrimidin-4-yl)-8-azabicyclo[3.2.1]oct-2-en-8-yl)-2-oxoacetate **(82b).**

Methyl 2-chloro-2-oxoacetate (174 mg, 1.42 mmol) was added to a solution of 4-(8-azabicyclo[3.2.1]oct-2-en-8-yl)-*N*-(1-methyl-1*H*-pyrazol-4-yl)pyrimidin-2-amine (400 mg, 1.42 mmol) and triethylamine (286 mg, 2.83 mmol) in dichloromethane (15 mL) at 0°C, and the mixture was stirred at 0°C for 2 hours. The reaction solution was quenched by adding methanol (10 mL) and concentrated under reduced pressure. The residues were purified by silica gel column chromatography (mobile phase: DCM/MeOH=50:1-20:1) to give 390 mg of the title compound as a yellow oil, yield: 74.5%.

LC-MS: m/z 369 $[M+H]^+$.

Step 2: Preparation of 2-(3-(2-((1-methyl-1*H*-pyrazol-4-yl)amino)pyrimidin-4-yl)-8-azabicyclo[3.2.1]oct-2-en-8-yl)-2-oxoacetic acid **(82c).**

Lithium hydroxide monohydrate (120 mg, 2.85 mmol) was added to a solution of methyl 2-(3-(3-(2-((1-methyl-1*H*-pyrazol-4-yl)amino)pyrimidin-4-yl)-8-azabicyclo [3.2.1]oct-2-en-8-yl)-2-oxoacetate (350 mg, 0.95 mmol) in tetrahydrofuran (10 mL) and water (5 mL) at 0°C, and the mixture was stirred at 0°C for 1 hour. Dilute hydrochloric acid-dioxane solution was added to the reaction solution to adjust pH to 7. The mixture was concentrated under reduced pressure and rotary evaporated to dry to give 375 mg of the crude title compound as a yellow oil.

LC-MS: m/z 355 $[M+H]^+$.

Step 3: Preparation of 2-(3-(2-((1-methyl-1*H*-pyrazol-4-yl)amino)pyrimidin-4-yl) -8-azabicyclo[3.2.1]oct-2-en-8-yl)-2-oxo-*N*-(2,2,2-trifluoroethyl)acetamide **(82).**

2-(3-(2-((1-Methyl-1*H*-pyrazol-4-yl)amino)pyrimidin-4-yl)-8-azabicyclo[3.2.1]oct-2-en-8-yl)-2-oxoacetic acid (300 mg, crude) was dissolved in acetonitrile (6 mL) at room temperature. 2-(7-Azobenzotriazole)-*N,N,N',N'*-tetramethylurea hexafluorophosphate (338 mg, 0.890 mmol) was added and reacted at room temperature for 30 min. 2,2,2-Trifluoroethane-1-amine (59 mg, 0.539 mmol) and *N,N*-diisopropylethylamine (229 mg, 1.78 mmol) were added and the mixture was reacted at room temperature overnight. The reaction solution was separated by preparative liquid chromatography to give 23 mg of the title compound as a pale yellow solid, yield: 8.9%.

Purification method by preparative liquid chromatography: column: 30 mm × 250 mm; packing: C18, 10 μm; process: 2-22 min, acetonitrile 20-60%; wavelength: 254 nm; flow rate: 45 ml/min; mobile phase: acetonitrile, water.

LC-MS: m/z 436 $[M+H]^+$.

$^1H$ NMR (400 MHz, DMSO-$d_6$): δppm9.43-9.35 (m,2H), 8.35-8.34 (m, 1H), 7.82 (s, 1H),7.50 (s, 1H), 7.20 (s1H), 6.84-6.82 (m, 1H), 5.13-4.92 (m, 1H), 4.85-4.83 (m, 1H), 4.01-3.91 (m, 2H),3.80 (s3H), 3.01-2.94 (m, 1H),2.58-2.53 (m, 1H),2.28-2.15 (m, 1H), 2.07-2.03 (m, 1H), 2.00-1.88 (m, 1H), 1.80-1.69 (m, 1H).

Example 83: Preparation of *N*-(cyanomethyl)-2-(3-(2-((1-methyl-1*H*-pyrazol-4-yl) amino)pyrimidin-4-yl)-8-azabicyclo[3.2.1]oct-2-en-8-yl)-2-oxoacetamide **(83)**

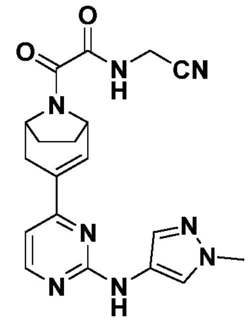

**83**

The same preparation method as that in Example 82 was used to give the title compound **83,** except that 2-aminoacetonitrile hydrochloride was used instead of 2,2,2-trifluoroethane-1-amine **(82d).**

Purification method by preparative liquid chromatography: column: 30 mm × 250 mm; packing: C18, 10 μm; process: 0-22 min, acetonitrile 10-50%; wavelength: 220 nm; flow rate: 45 mL/min; mobile phase: acetonitrile, 0.05% aqueous solution of formic acid.

LC-MS: m/z 393 $[M+H]^+$.

$^1H$ NMR (400 MHz, DMSO-$d_6$): δppm9.45-9.43 (m,1H), 9.35 (s,1H),8.36-8.34 (m, 1H),7.83 (s, 1H),7.50 (s, 1H), 7.22-7.20 (m1H),6.84-6.82 (m, 1H), 5.29-4.99 (m, 1H),4.96-4.83 (m, 1H), 4.20 (dd, *J* = 12.0 Hz, 4.0 Hz,2H), 3.81 (s, 3H),3.04-2.93 (m, 1H), 2.58-2.54 (m, 1H), 2.32-2.14 (m, 1H), 2.08-2.02 (m, 1H), 2.00-1.87 (m, 1H),1.81-1.67 (m, 1H).

Example 84: Preparation of *N*-(1-methyl-1*H*-pyrazol-4-yl)-4-(8-(1-(propansulfonyl)azetidin-3-yl)-8-azabicyclo[3.2.1]oct-2-en-3-yl)pyrimidin-2-amine (84)

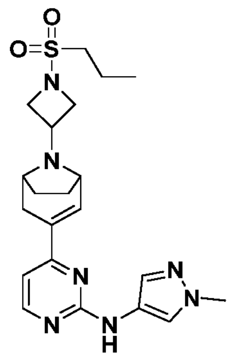

**84**

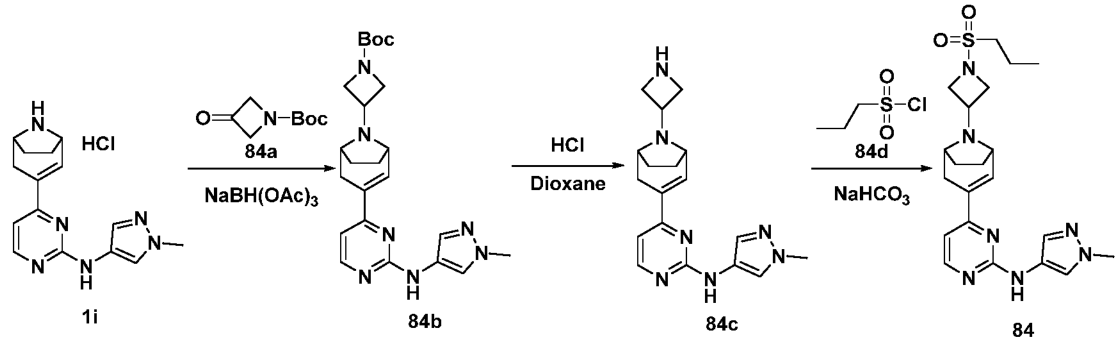

**Step 1:** Synthesis of tert-butyl 3-(3-(2-((1-methyl-1*H*-pyrazol-4-yl)amino) pyrimidin-4-yl)-8-azabicyclo[3.2.1]oct-2-en-8-yl)azetidine-1-carboxylate **(84b)**

Tetraisopropyl titanate (148 mg, 0.521 mmol) was added to a mixed solution of 4-(8-azabicyclo[3.2.1]oct-2-en-3-yl)-*N*-(1-methyl-1H-pyrazol-4-yl)pyrimidin-2-amine hydrochloride (400 mg, 1.05 mmol) and tert-butyl 3-oxoazetidine-1-carboxylate (180 mg, 1.05 mmol) in anhydrous dichloromethane (10 mL) at room temperature. The mixture was stirred at room temperature for 30 minutes. Sodium borohydride acetate (267 mg, 1.26 mmol) was added and the mixture was stirred at room temperature overnight. Dichloromethane (20 mL) and an aqueous solution of sodium hydroxide (2 M, 10 mL) were added. The mixture was extracted, subjected to phase separation, dried and concentrated under reduced pressure. The residues were subjected to flash column chromatography (mobile phase: DCM/MeOH=100:1-10:1) to give 100 mg of brownish yellow solid, yield: 21.8%.

LC-MS: m/z 438 $[M+H]^+$.

**Step 2:** Synthesis of 4-(8-(azetidin-3-yl)-8-azabicyclo[3.2.1]oct-2-en-3-yl)-*N*-(1-methyl-1*H*-pyrazol-4-yl)pyrimidin-2-amine hydrochloride **(84c)**

A solution of HCl in dioxane (4 M, 10 mL) was added to a solution of tert-butyl 3-(3-(2-((1-methyl-1*H*-pyrazol-4-yl)amino)pyrimidin-4-yl)-8-azabicyclo[3.2.1]oct-2-en-8-yl)azetidine-1-carboxylate (100 mg, 0.229 mmol) in dioxane (2 mL) at room temperature, and the mixture was stirred at room temperature for 1 hour. The mixture was concentrated under reduced pressure at low temperature and rotary evaporated to dry to give 70 mg of crude product as a brownish red oil, yield: 81.8%.

LC-MS: m/z 338 $[M+H]^+$.

Step 3: Synthesis of *N*-(1-methyl-1*H*-pyrazol-4-yl)-4-(8-(1-(propansulfonyl) azetidin-3-yl)-8-azabicyclo[3.2.1]oct-2-en-3-yl)pyrimidin-2-amine **(84)**

Propylsulfonyl chloride (32.0 mg, 0.224 mmol) was added to a solution of 4-(8-(azetidin-3-yl)-8-azabicyclo[3.2.1]oct-2-en-3-yl)-*N*-(1-methyl-1*H*-pyrazol-4-yl)pyr imidin-2-amine hydrochloride (70 mg, 0.187 mmol) and sodium bicarbonate (47.1 mg, 0.561 mmol) in tetrahydrofuran and water (5 mL, V/V=4/1) at 0°C, and the reaction was continued for 2 hours. Ethyl acetate (10 mL) and water (10 mL) were added and the phases were separated. The aqueous phase was extracted twice with ethyl acetate, 10 mL each time. The organic phases were pooled, washed with saturated brine, dried and concentrated under reduced pressure. The residues were purified by preparative liquid chromatography to give 32 mg of brown solid, yield: 32.2%.

Purification method by preparative liquid chromatography: column: 30 mm × 250 mm; packing: C18, 10 μm; process: 0-22 min, acetonitrile 10-60%; wavelength: 220 nm; flow rate: 45 mL/min; mobile phase: acetonitrile, 0.05% aqueous solution of formic acid.

LC-MS: m/z 444 $[M+H]^+$.

$^1$H NMR (400 MHz, DMSO-$d_6$): $\delta$ ppm 9.31 (s, 1H), 8.35 (d, *J* =4.8 Hz, 1H),7.85 (s, 1H),7.54 (s, 1H),7.03 (s, 1H), 6.82 (d, *J*= 4.8 Hz, 1H), 3.96-3.94 (m, 1H), 3.89-3.88 (m, 1H), 3.86 (s, 3H), 3.76-3.73(m, 2H), 3.57-3.51(m, 3H), 3.13-3.09 (m, 2 H), 2.72-2.68 (m, 1H), 2.18-2.14 (m, 2H), 2.02-1.97 (m, 1H), 1.96-1.86 (m, 1H), 1.77-1.69 (m, 2H), 1.67-1.55(m, 1H), 1.02-0.99 (m, 3H).

Example 85: Preparation of 2-(1-((*S*)-2,2-difluorocyclopropane-1-formyl)-3-(3-2-((1-methyl-1*H*-pyrazol-4-yl)amino)pyrimidin-4-yl)-8-azabicyclo[3.2.1]oct-2-en-8-yl) azetidin-3-yl)acetonitrile (**85**)

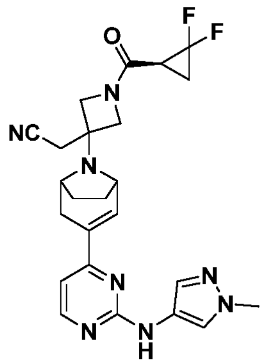

85

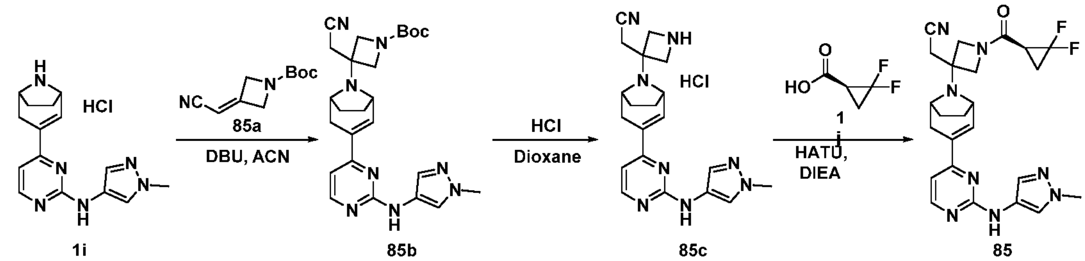


**Step 1:** Synthesis of tert-butyl-9-(cyanomethyl)-3-(3-(2-((1-methyl-1H-pyrazol-4-yl)amino)pyrimidin-4-yl)-8-azabicyclo[3.2.1]oct-2-en-8-yl) azetidine -1-carboxylate **(85b)**

Tert-butyl 3-(cyanomethylenyl)azetidine-1-carboxylate (1.72 g, 8.87 mmol) was added to a solution of 4-(8-azabicyclo[3.2.1]oct-2-en-3-yl)-amino-(1-methyl-1H-pyrazol-4-yl)pyrimidin-2-amine (500 mg, 1.77 mmol) and 1,8-diazabicyclo[5.4.0] undec-7-ene (1.35 g, 8.87 mmol) in acetonitrile (50 mL), and the mixture was stirred at 50°C for 72 hours. Saturated brine (50 mL) was added. The mixture was extracted with ethyl acetate (50 mL*3). The organic phase was dried over anhydrous sodium sulfate and concentrated under reduced pressure. The residues were subjected to flash column chromatography (mobile phase: dichloromethane/methanol, 100/1 to 20/1) to give 450 mg of the title compound as a yellow oil, yield: 53%.
LCMS[M+H]: 477.

**Step 2:** Synthesis of 2-(3-(3-(2-((1-methyl-1H-pyrazol-4-yl)amino)pyrimidin-4-yl)-8-azabicyclo[3.2.1]oct-2-en-8-yl)azetidin-3-yl)acetonitrile hydrochloride **(85c)**

HCl/1,4 dioxane (10 mL, 4 mol/L) was added to a solution of tert-butyl-9(cyanomethyl)-3-(3-(2-((1-methyl-1H-pyrazol-4-yl)amino )pyrimidin-4-yl)-8 -azabicyclo[3.2.1]oct-2-en-8-yl)azetidine-1-carboxylate (450 mg, 0.943 mmol) in dichloromethane (20 mL) at room temperature. The mixture was stirred at room temperature for 30 minutes and concentrated under reduced pressure at low temperature to give 500 mg of the crude title compound as a yellow solid.
LCMS[M+H]: 377.

Step 3: Synthesis of 2-(1-((*S*)-2,2-difluorocyclopropane-1-carbonyl)-3-(3-(2-((1-methyl-1*H*-pyrazol-4-yl)amino)pyrimidin-4-yl)-8-azabicyclo[3.2.1]oct-2-en-8-yl) azetidin-3-yl)acetonitrile **(85)**

2-(7-Oxidobenzotriazole)-*N,N,N',N'*-tetramethylurea hexafluorophosphate (276 mg, 0.728 mmol) was added to a solution of (*S*)-2,2-difluorocyclopropane-1-carboxylic acid (54.4 mg, 0.485 mmol) in tetrahydrofuran (10 mL) at room temperature, and the mixture was stirred at room temperature for 30 minutes. 2-(3-(3-(2-((1-Methyl-1H-pyrazol-4-yl)amino)pyrimidin-4-yl)-8-azabicyclo[3.2.1]oct-2-en-8-yl)azetidin-3-yl)acet onitrile hydrochloride (200 mg, 0.485 mmol) and *N,N*-diisopropylethylamine (187 mg, 1.45 mmol) were added, and the mixture was stirred at room temperature overnight. The mixture was added to water (50 mL) and extracted with ethyl acetate (30 mL*3). The organic phase was washed with saturated brine, dried over anhydrous sodium sulfate and concentrated under reduced pressure. The residues were separated by preparative liquid chromatography to give 20 mg of the title compound as a yellow solid.
Purification method by preparative liquid chromatography: column: 30 mm × 250 mm; packing: C18, 10 μm;

process: 0-2-27 min, acetonitrile 10-10-50%; wavelength: 220 nm; flow rate: 45 mL/min; mobile phase: acetonitrile, 0.05% aqueous solution of formic acid.

LCMS[M+H]: 481.

$^1$H NMR (300 MHz, DMSO-$d_6$) :$\delta$ppm9.32 (s, 1H), 8.32 (d, *J*= 4.7 Hz, 1H), 7.83 (s, 1H), 7.50 (s, 1H), 7.18 (s, 1H), 6.79 (s, 1H), 4.50-4.28 (m, 1H), 4.20-3.85 (m, 4H), 3.80 (s, 3H), 3.71-3.56 (m, 1H), 3.23 (s, 2H), 2.70 (d, *J* = 11.0 Hz, 2H), 2.35-2.21 (m, 1H), 2.13-1.98 (m, 1H), 1.96-1.73 (m, 4H), 1.62-1.46 (m, 1H).

Example 86: Preparation of 3-(cyanomethyl)-3-(3-(2-((1-methyl-1*H*-pyrazol-4-yl) amino)pyrimidin-4-yl)-8-azabicyclo[3.2.1]oct-2-en-8-yl)-*N*-(2,2,2-trifluoroethyl) azetidine-1-carboxamide **(86)**

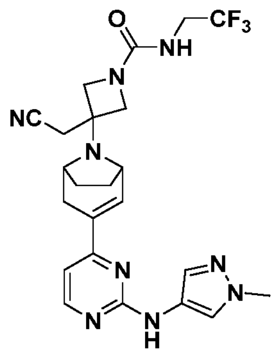

**86**

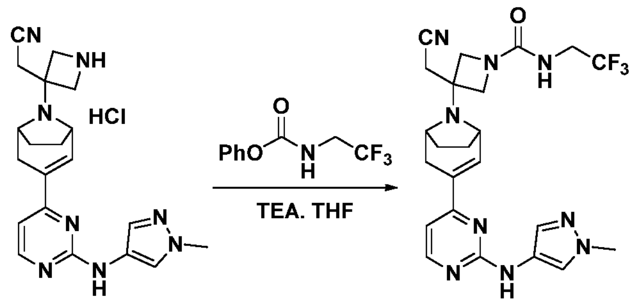


85c 86

The same preparation method as that in Example 3 was used to give the title compound **86,** except that 2-(3-(3-(2-((1-methyl-1H-pyrazol-4-yl)amino)pyrimidin-4-yl)-8-azabicyclo[3.2.1]oct-2-en-8-yl) azetidin-3-yl)acetonitrile hydrochloride **(85c)** was used instead of 4-(8-azabicyclo[3.2.1]oct-2-en-3-yl)-*N*-(1-methyl-1*H*-pyrazol-4-yl) pyrimidin-2-amine (**1i**).

Purification method by preparative liquid chromatography: column: 30 mm × 250 mm; packing: C18, 10 $\mu$m; process: 0-22 min, acetonitrile 10-70%; wavelength: 220 nm; flow rate: 45 mL/min; mobile phase: acetonitrile, 0.05% aqueous solution of formic acid.

LCMS[M+H]: 502.

$^1$H NMR (300 MHz, DMSO-$d_6$): $\delta$ppm9.32 (s, 1H), 8.32 (d, *J* = 5.1 Hz, 1H), 7.83 (s, 1H), 7.50 (s, 1H), 7.18 (d, *J*= 4.8 Hz, 1H), 7.07 (t, *J*= 6.3 Hz, 1H), 6.79 (d, *J*= 5.2 Hz, 1H), 3.96 (d, *J* = 8.5 Hz, 3H), 3.89-3.82 (m, 1H), 3.80 (s, 3H), 3.78-3.66 (m, 3H), 3.61-3.52(m, 1H), 3.25-3.10 (m, 2H), 2.75-2.59 (m, 1H), 2.34-2.19 (m, 1H), 2.12-1.93 (m, 1H), 1.92-1.72 (m, 2H), 1.60-1.50 (m, 1H).

Example 87: Preparation of *N*-(1-methyl-1*H*-pyrazol-4-yl)-4-(8-((3-methyloxetan-3-yl)methyl)-8-azabicyclo[3.2.1]oct-2-en-3-yl)pyrimidin-2-amine **(87)**

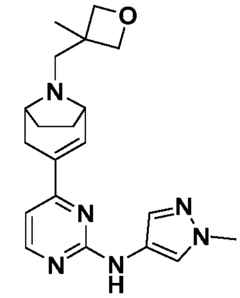

87

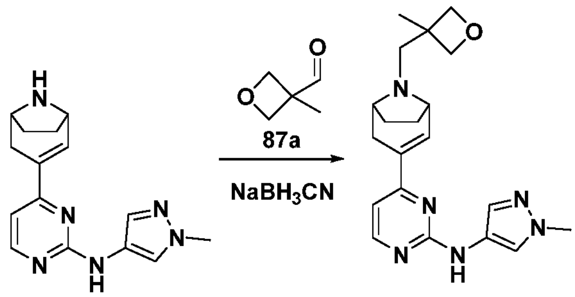


1i 87

Step 1: Preparation of *N*-(1-methyl-1*H*-pyrazol-4-yl)-4-(8-((3-methyloxetan-3-yl)methyl)-8-azabicyclo[3.2.1]oct-2-en-3-yl)pyrimidin-2-amine **(87).**

Sodium cyanoborohydride (133 mg, 2.12 mmol) and tetraisopropyl titanate (101 mg, 0.354 mmol) were added to a mixture of 4-(8-azabicyclo[3.2.1]oct-2-en-3-yl)-*N*-(1-methyl-1*H*-pyrazol-4-yl)pyrimidin-2-amine (200 mg, 0.708 mmol), 3-methyloxetane-3-carbaldehyde (71 mg, 0.708 mmol) and acetic acid (21 mg, 0.354 mmol) in acetonitrile (7 mL) at room temperature, and the mixture was stirred overnight. The reaction solution was concentrated under reduced pressure. The residues were separated by preparative liquid chromatography to give 32 mg of the title compound as a yellow solid, yield: 12.3%.

Purification process by preparative liquid chromatography: column: 30 mm × 250 mm; packing: C18, 10 μm; process: 0-22 min, acetonitrile 10-40%; wavelength: 220 nm; flow rate: 45 mL/min; mobile phase: acetonitrile/water.

LC-MS: m/z 367 $[M+H]^+$.

$^1$H NMR (300 MHz, DMSO-$d_6$): $\delta$ppm9.35 (s,1H),8.36 (d, *J*= 3.0 Hz,1H),7.82 (s, 1H), 7.53 (s, 1H), 7.09-7.05 (m, 1H), 6.86 (d, *J* = 6.0 Hz,1H), 4.40-4.35 (m, 2H), 4.24-4.21 (m, 2H), 3.80 (s, 3H), 3.10-2.73 (m, 4H), 2.33-1.86 (m, 5H),1.73-1.58 (m, 1H),1.36 (s, 3H).

## BIOLOGICAL EVALUATION

Test Example 1: Determination of *in vitro* inhibitory activity of the compounds of the present invention on JAK1 kinase

Experimental materials: JAK1 kinase (Invitrogen, PV4744), ATP (Promega, V915B), ADP-Glo Kinase Assay (Promega, V9101), IRS1 (Signalchem, I40-58-1000).

Sample preparation: The compounds of the present invention and the control product were dissolved in DMSO solvent respectively to formulate into 10 mM initial liquid. The maximum concentration of the compound was 10 μM, 3-fold dilution, 10 concentration gradients, and duplicate wells for each concentration gradient.

Experimental processs: 0.1 μL of the compound to be tested was transferred into a 384 reaction plate (PE, 6007290) via Echo and centrifuged at 1000 rpm/min for 1 min. 5 μL JAK1 kinase (final concentration of 4 nM) was transferred into the 384 reaction plate, which was then centrifuged at 1000 rpm/min for 1 min, and incubated at 25°C for 15 min. 5 μL substrate mixture (1 mM ATP, IRS1 0.05 mg/ml, kinase buffer solution) was transferred into the 384 reaction plate, which was then centrifuged at 1000 rpm/min for 1 min, and incubated at 25°C for 60 min. 10 μL ADP-Glo was transferred into the 384 reaction plate, which was then centrifuged at 1000 rpm/min for 1 min, and incubated at 25°C for 40 min. 20 μL test solution was transferred into the 384 reaction plate, which was then centrifuged at 1000 rpm/min for 1 min and incubated at 25°C for 40 min. RLU (Relative luminescence unit) signal was read by using an Envision multifunctional plate reader. The signal intensity was used to characterize the degree of the kinase activity.

The $IC_{50}$ (half inhibitory concentration) of the compounds was obtained by using the following non-linear fitting equation:

$$Y=Bottom + (Top-Bottom)/(1+10^((LogIC_{50}-X)*HillSlope));$$

X: Log value of the concentration of the compound;
Y: Emission ratio;
Bottom: The minimum value, Top: The maximum value, HillSlope: Slope;

The inhibitory activity of the compounds of the present invention on JAK1 kinase is as shown in Table 1 below. $IC_{50}$ value of 0-100 nM is shown as A, $IC_{50}$ value of 100-300 nM is shown as B, $IC_{50}$ value of 300-1000 nM is shown as C, and $IC_{50}$ value which is greater than 1000 nM is shown as D. NT means not tested.

Table 1: Inhibitory activity of the compounds of the present invention on JAK1 kinase

| Compound No. | $IC_{50}$ (nM) |
|---|---|
| | JAK1 |
| 1 | A |
| 1-a | A |
| 1-b | A |
| 2 | A |
| 3 | B |
| 3-a | A |
| 3-b | A |
| 4 | D |
| 5 | C |
| 6 | D |
| 7 | D |
| 8 | C |
| 9 | D |
| 10 | D |
| 11 | A |
| 12 | A |
| 12-a | A |
| 12-b | A |
| 13 | D |
| 14 | B |
| 15 | C |
| 16 | D |
| 17 | D |
| 18 | D |
| 19 | A |
| 19-a | A |
| 19-b | A |
| 20 | A |
| 21 | A |
| 22 | A |
| 22-a | B |
| 22-b | A |
| 23 | A |
| 23-a | A |
| 23-b | A |
| 24 | A |

(continued)

| Compound No. | $IC_{50}$ (nM) |
|---|---|
| | JAK1 |
| 25 | C |
| 26 | A |
| 26-a | D |
| 26-b | A |
| 27 | C |
| 28 | A |
| 29 | A |
| 29-a | A |
| 29-b | A |
| 30 | A |
| 31 | A |
| 32 | B |
| 33 | A |
| 34 | D |
| 35 | B |
| 36 | B |
| 37 | C |
| 38 | A |
| 39 | D |
| 40 | B |
| 41 | B |
| 42 | A |
| 43 | A |
| 44 | A |
| 45 | B |
| 46 | B |
| 47 | B |
| 48 | D |
| 49 | A |
| 50 | B |
| 51 | A |
| 52 | D |
| 53 | D |
| 54 | D |
| 55 | A |
| 56 | C |
| 57 | C |
| 58 | B |
| 59 | C |
| 60 | D |
| 61 | B |
| 62 | B |
| 63 | C |
| 64 | C |
| 65 | B |
| 66 | B |
| 67 | C |
| 68 | C |

(continued)

| Compound No. | $IC_{50}$ (nM) |
|---|---|
| | JAK1 |
| 69 | C |
| 70 | A |
| 71 | D |
| 72 | D |
| 73 | D |
| 74 | D |
| 75 | B |
| 76 | B |
| 77 | A |
| 78 | D |
| 79 | A |
| 80 | D |
| 81 | D |
| 82 | C |
| 83 | D |
| 84 | D |
| 85 | D |
| 86 | D |
| 87 | D |

It can be seen from the above experimental results that the compounds of the present invention have good *in vitro* anti-JAK1 kinase activity.

Test Example 2: Determination of *in vitro* inhibitory activity of the compounds of the present invention on Tyk2 kinase

Experimental materials: TYK2 (Invitrogen, PV4790), ATP (Promega, V915B), ADP-Glo Kinase Assay (Promega, V9101), IRS1 (Signalchem, 140-58-1000).

Sample preparation: The compounds of the present invention and the control product were dissolved in DMSO solvent respectively to formulate into 10 mM initial liquid. The maximum concentration of the compound was 10 μM, 3-fold dilution, 10 concentration gradients, and duplicate wells for each concentration gradient.

Experimental processs: 0.1 μL of the compound to be tested was transferred into a 384 reaction plate (PE, 6007290) via Echo and centrifuged at 1000 rpm/min for 1 min. 5 μL TYK2 kinase (final concentration of 4 nM) was transferred into the 384 reaction plate, which was then centrifuged at 1000 rpm/min for 1 min, and incubated at 25°C for 15 min. 5 μL substrate mixture (1 mM ATP, IRS1 0.05 mg/ml, kinase buffer solution) was transferred into the 384 reaction plate, which was then centrifuged at 1000 rpm/min for 1 min, and incubated at 25°C for 60 min. 10 μL ADP-Glo was transferred into the 384 reaction plate, which was then centrifuged at 1000 rpm/min for 1 min, and incubated at 25°C for 40 min. 20 μL test solution was transferred into the 384 reaction plate, which was centrifuged at 1000 rpm/min for 1 min and incubated at 25°C for 40 min. RLU (Relative luminescence unit) signal was read by using an Envision multifunctional plate reader. The signal intensity was used to characterize the degree of the kinase activity.

The $IC_{50}$ (half inhibitory concentration) of the compounds was obtained by using the following non-linear fitting equation:

$$Y=Bottom + (Top-Bottom)/(1+10^{\wedge}((LogIC_{50}-X)*HillSlope));$$

X: Log value of the concentration of the compound;
Y: Emission ratio;
Bottom: The minimum value, Top: The maximum value, HillSlope: Slope;

The inhibitory activity of the compounds of the present invention on TYK2 kinase is as shown in Table 2 below. $IC_{50}$ value of 0-100 nM is shown as A, $IC_{50}$ value of 100-300 nM is shown as B, $IC_{50}$ value of 300-1000 nM is shown

as C, and $IC_{50}$ value which is greater than 1000 nM is shown as D. NT means not tested.

Table 2: Inhibitory activity of the compounds of the present invention on Tyk2 kinase

| Compound No. | $IC_{50}$ (nM) |
|---|---|
| | Tyk2 |
| 1 | A |
| 1-a | A |
| 1-b | A |
| 2 | A |
| 3 | A |
| 11 | A |
| 12 | A |

It can be seen from the above experimental results that the compounds of the present invention have good *in vitro* anti-Tyk2 kinase activity.

Test Example 3: Inhibition effect of the compounds of the present invention on STAT3 signaling pathway of human whole blood

Experimental materials: CD3 (BD, 555335), pSTAT3 antibody (BD, 612569), IFN-2α (Biolegend, 592702)

Sample preparation: The compounds of the present invention and the control were dissolved in DMSO solvent respectively to formulate into 10 mM initial liquid. The maximum concentration of the compound was 10 μM, 3-fold dilution, 10 concentration gradients, and duplicate wells for each concentration gradient.

Experimental processs: 20 μL of the compound of the present invention was added to a flow cytometer tube (BD, 352052) containing 180 μL anticoagulant sodium heparin. 20 μL PBS was added to the control. The sample was incubated at 37°C for 30 min. 2 μL stimulating factor was added and then incubated at 37°C for 20 min. 1 mL erythrocyte lysate was rapidly added, and the sample was repeatedly inverted 5-10 times or vortexed and then incubated at 37°C for 10 min. The sample was centrifuged at 600 g for 6-8 min. The supernatant was discarded. The precipitate was vortexed until suspending. The cells were washed with 3 mL PBS and centrifuged at 600 g for 6-8 min. The supernatant was discarded. The precipitate was vortexed until suspending. 1 mL of membrane breaking solution was added, and the system was mixed gently, incubated on ice for 30 min, and centrifuged at 600 g for 6-8 min. The supernatant was discarded. The precipitate was vortexed until suspending. The cells were washed. 3 mL PBS was added. The system was centrifuged at 600 g for 6-8 min. The supernatant was discarded. The precipitate was vortexed untile suspending. The process was repeated twice. 100 μL PBS was added to each staining tube. IFN-2α was added for stimulation, and CD3 and pSTAT3 antibodies (20μL) were added. The system was mixed well, protected from light and incubated at room temperature for 60 min. The cells were washed. 3 mL PBS was added. The system was centrifuged at 600 g for 6-8 min. The supernatant was discarded. The precipitate was vortexed until suspending. The precipitate was suspended in 150 μL in the dark for the flow cytometry analysis. The $IC_{50}$ (half inhibitory concentration) of the compounds was obtained by using the following non-linear fitting equation:

$$Y=Bottom + (Top-Bottom)/(1+10^{\wedge}((LogIC_{50}-X)*HillSlope));$$

X: Log value of the concentration of the compound;
Y: Emission ratio;
Bottom: The minimum value, Top: The maximum value, HillSlope: Slope

The inhibition effect of by the compounds of the present invention on the IFN-2α-stimulated TYK2/JAK1-mediated pathway is shown in Table 3. $IC_{50}$ value of 0-100 nM is shown as A, $IC_{50}$ value of 100-300 nM is shown as B, $IC_{50}$ value of 300-1000 nM is shown as C, $IC_{50}$ value which is greater than 1000 nM is shown as D. NT means not tested.

Table 3: Inhibition effect of the compounds of the present invention on STAT3 signaling pathway of human whole blood

| Cytokine/ pSTAT | JAK signaling pathway | $IC_{50}$ (nM) | | | | | |
|---|---|---|---|---|---|---|---|
| | | Example 1 | Example 1-a | Example 1-b | Example 3 | Example 51 | Example 54 |
| IFN-2α-induced pSTAT3 | TYK2/JAK1 | A | A | A | A | A | A |

As can be seen from Table 3, the compounds of the present invention have very good inhibitory effect on the TYK2/JAK1-mediated signaling pathway of human whole blood.

Test Example 4: Pharmacodynamic studies of the compounds of Example 1, Example 1-b and Example 3 for AIA in rats

Experimental material: complete Freund's adjuvant (Chondrex, 7027)

Experimental process: The *in vivo* efficacy of the compounds of the invention was investigated. 8-Week-old female Lewis rats were selected for modeling by complete Freund's adjuvant (J. of Immunology, 2010, 184: 5298-5307) and grouped after 12 days. 7 groups were divided: the model group, the Example 1 low dose group (3 mg/kg), the Example 1 high dose group (30 mg/kg), the Example 1-b low dose group (1 mg/kg), the Example 1-b high dose group (3 mg/kg), the Example 3 low dose group (3 mg/kg) and the Example 3 high dose group (30 mg/kg). The model group was given 0.5% CMC-Na and the rest groups were given the corresponding dose of the compound. The anmials were scored according to the degree of joint swelling after 7 days of oral administration (Proc Soc Exp Bio Med 1962, 111: 544-547). The scoring results are shown in Table 4.

Table 4: Experimental results of the efficacy of the compounds of the present invention for AIA in rats

| Group | Statistical value | Scoring value | | |
|---|---|---|---|---|
| | | Day 1 | Day 3 | Day 7 |
| Model group | Mean ± SD | 4.11 ± 2.57 | 8.44 ± 2.83 | 6.33 ± 1.22 |
| Example 1 (3 mg/kg) | Mean ± SD | 3.78 ± 2.54 | 2.67 ± 1.22** | 0.22 ± 0.44** |
| Example 1 (30 mg/kg) | Mean ± SD | 4.11 ± 2.52 | 0.44 ± 0.73** | 0.00 ± 0.00** |
| Example 3 (3 mg/kg) | Mean ± SD | 3.78 ± 1.92 | 3.78 ± 1.72** | 1.33 ± 0.87** |
| Example 3 (30 mg/kg) | Mean ± SD | 3.44 ± 1.94 | 0.56 ± 0.53** | 0.00 ± 0.00** |
| Example 1-b (1 mg/kg) | Mean ± SD | 3.30 ± 2.67 | 3.10 ± 3.51** | 3.30 ± 3.71** |
| Example 1-b (3 mg/kg) | Mean ± SD | 3.30 ± 2.67 | 0.30 ± 0.95* | 1.50 ± 3.75** |
| Note: Wherein, "*" p<0.05 represents a significant difference and "**" p<0.01 represents a highly significant difference. | | | | |

From the experimental results, it can be seen that the compounds of Example 1, Example 1-b and Example 3 significantly inhibit the symptoms of arthritis induced by complete Freund's adjuvant, indicating that the compounds of Example 1, Example 1-b and Example 3 have good efficacy on the AIA model.

Experimental Example 5: Pharmacokinetic studies of the compounds of Example 1, Example 1-a, Example 1-b and Example 3 in rats

In order to investigate the blood concentration and pharmacokinetic parameters of the compounds of the present invention after oral administration, the corresponding LC/MS/MS assay and plasma treatment methods were developed for the compounds of the Experimental Examples. The preliminary methodological validation showed that the endogenous substances in the plasma basically did not affect the separation of the compounds to be measured from the internal standard and the determination, and the regression equation of the plasma standard curve had r greater than 0.95 and had good linearity, which satisfied the detection needs of the compounds to be measured in the plasma samples.

Male SD rats were orally administered at a dose of 5 mg/kg. Blood was orbitally collected and anticoagulated with sodium heparin. The plasma was deproteinated with acetonitrile. The samples were analyzed by LC/MS/MS method. The *in vivo* pharmacokinetic parameters (statistical moment parameters of non-atrial model) in SD rats are shown in Table 5. Wherein, AUC <1000 μg/L*h is shown as C, AUC between 1000-2000 μg/L*h is shown as B, AUC >2000 μg/L*h is shown as A; $C_{max}$ <600 μg/L is shown as C, $C_{max}$ between 600-900 μg/L is shown as B, and $C_{max}$ >900 μg/L is shown as A.

Table 5: Pharmacokinetic parameters of the compounds of Example 1, Example 1-a, Example 1-b and Example 3 for oral administration in SD rats

| Example | 1 | 1-a | 1-b | 3 |
|---|---|---|---|---|
| $AUC_{0-t}$, μg/L*h | A | B | A | B |
| $C_{max}$, μg/L | A | B | A | B |

It can be seen from Table 5 that the compounds of Example 1, Example 1-a, Example 1-b and Example 3 have good pharmacokinetic parameters and are suitable for oral administration.

## Claims

**1.** A compound represented by general formula (I),

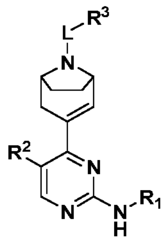

(I)

or a mesomer, racemate, enantiomer, diastereomer thereof, or a mixture thereof, or a prodrug thereof, or a pharmaceutically acceptable salt thereof, wherein:

$R^1$ is selected from the group consisting of cycloalkyl, heterocyclyl, aryl and heteroaryl, wherein the cycloalkyl, heterocyclyl, aryl and heteroaryl are each independently optionally further substituted with one or more of $R^4$;
each of $R^4$ is independently selected from the group consisting of halogen, amino, nitro, cyano, hydroxyl, thiol, oxo, alkyl, alkoxyl, cycloalkyl, heterocyclyl, aryl, heteroaryl, $-C(O)R^a$, $-O(O)CR^a$, $-C(O)OR^a$, $-C(O)NR^aR^b$, $NR^aR^b$, $-NHC(O)R^a$, $-S(O)_nR^a$, $-S(O)_nNR^aR^b$ and $-NHS(O)_nR^a$, wherein the alkyl, alkoxyl, cycloalkyl, heterocyclyl, aryl and heteroaryl are each independently optionally further substituted with one or more groups selected from the group consisting of halogen, amino, nitro, cyano, oxo, hydroxyl, thiol, carboxyl, alkoxycarbonyl, alkyl, haloalkyl, alkoxyl, haloalkoxyl, alkenyl, alkynyl, cycloalkyl, heterocyclyl, aryl and heteroaryl;
$R^2$ is selected from the group consisting of hydrogen, halogen, amino, cyano, hydroxyl, thiol, carboxyl, alkyl, alkoxyl and cycloalkyl, wherein the alkyl, alkoxyl and cycloalkyl are each independently optionally further substituted with one or more groups selected from the group consisting of halogen, amino, nitro, cyano, oxo, hydroxyl, thiol, carboxyl, alkoxycarbonyl, alkyl, alkoxyl, alkenyl, alkynyl, cycloalkyl, heterocyclyl, aryl and heteroaryl;
L is selected from the group consisting of single bond, $-CR^5R^6-$, -C(O)-, -C(S)-, $-N(R^a)-$, $-S(O)_n-$, -O-, -S-, $-C(O)N(R^a)-$, $-C(O)-C(O)-N(R^a)-$ and $-S(O)_nN(R^a)-$;
$R^5$ and $R^6$ are each independently selected from the group consisting of hydrogen, halogen, hydroxyl, alkyl, alkoxyl, alkenyl, alkynyl, cycloalkyl, heterocyclyl, aryl and heteroaryl, wherein the alkyl, alkoxyl, alkenyl, alkynyl, cycloalkyl, heterocyclyl, aryl and heteroaryl are each independently optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;
or $R^5$ and $R^6$ together with the atom to which they are attached form a cycloalkyl or heterocyclyl, wherein the cycloalkyl or heterocyclyl is optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;
$R^3$ is selected from the group consisting of alkyl, cycloalkyl, heterocyclyl, aryl and heteroaryl, wherein the alkyl, cycloalkyl, heterocyclyl, aryl and heteroaryl are each independently optionally further substituted with one or more of $R^7$;

each of $R^7$ is independently selected from the group consisting of halogen, amino, nitro, cyano, hydroxyl, thiol, oxo, alkyl, alkoxyl, cycloalkyl, heterocyclyl, aryl, heteroaryl, $OR^a$, $-C(O)R^a$, $-O(O)CR^a$, $-C(O)OR^a$, $-C(O)NR^aR^b$, $NR^aR^b$, $-NHC(O)R^a$, $-S(O)_nR^a$, $-S(O)_nNR^aR^b$ and $-NHS(O)_nR^a$, wherein the alkyl, alkoxyl, cycloalkyl, heterocyclyl, aryl and heteroaryl are each independently optionally further substituted with one or more groups selected from the group consisting of halogen, amino, nitro, cyano, oxo, hydroxyl, thiol, carboxyl, alkoxycarbonyl, alkyl, haloalkyl, alkoxyl, haloalkoxyl, alkenyl, alkynyl, cycloalkyl, heterocyclyl, aryl and heteroaryl;

$R^a$ and $R^b$ are each independently selected from the group consisting of hydrogen, halogen, hydroxyl, alkyl, alkenyl, alkynyl, cycloalkyl, heterocyclyl, aryl and heteroaryl, wherein the alkyl, alkenyl, alkynyl, cycloalkyl, heterocyclyl, aryl and heteroaryl are each independently optionally further substituted with one or more groups selected from the group consisting of halogen, amino, nitro, cyano, hydroxyl, thiol, carboxyl, alkoxycarbonyl, oxo, alkyl, alkoxyl, alkenyl, alkynyl, cycloalkyl, heterocyclyl, aryl and heteroaryl;

or $R^a$ and $R^b$ together with the atom to which they are attached form a nitrogen-containing heterocyclyl, wherein the nitrogen-containing heterocyclyl is optionally further substituted with one or more groups selected from the group consisting of halogen, amino, nitro, cyano, oxo, hydroxyl, thiol, carboxyl, alkoxycarbonyl, alkyl, alkoxyl, alkenyl, alkynyl, cycloalkyl, heterocyclyl, aryl and heteroaryl; and

n is 0, 1 or 2.

**2.** The compound represented by general formula (I) according to claim 1, which is a compound of formula (II):

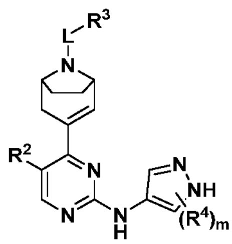

(II)

wherein, $R^2$, $R^3$, $R^4$ and L are as defined in claim 1; and
m is 0, 1, 2 or 3.

**3.** The compound represented by general formula (I) according to claim 1 or 2, wherein,

$R^3$ is selected from the group consisting of alkyl, cycloalkyl and heterocyclyl, wherein the alkyl, cycloalkyl and heterocyclyl are each independently optionally further substituted with one or more of $R^7$; and

$R^7$ is as defined in claim 1, preferably selected from the group consisting of halogen, cyano, aryl, cycloalkyl and alkyl, wherein the cycloalkyl and alkyl are each independently optionally substituted with one or more halogens.

**4.** The compound represented by general formula (I) according to any one of claims 1 to 3, wherein,

L is selected from the group consisting of single bond, $-CR^5R^6-$, -C(O)-, $-S(O)_n-$, -O-, -S-, $-C(O)N(R^a)-$, $C(O)-C(O)-N(R^a)-$ and $-S(O)_nN(R^a)-$, preferably $-S(O)_n-$, -C(O)-, $-C(O)N(R^a)-$ and $-S(O)_nN(R^a)-$, more preferably -C(O)- and $-C(O)N(R^a)-$,

wherein, $R^5$, $R^6$, $R^a$ and n are as defined in claim 1.

**5.** The compound represented by general formula (I) according to any one of claims 1 to 4, wherein,
$R^2$ is selected from the group consisting of hydrogen, halogen, cyano, hydroxyl, carboxyl, alkyl and cycloalkyl, preferably hydrogen, halogen, cyano and alkyl, more preferably hydrogen and halogen.

**6.** The compound represented by general formula (I) according to claim 1, wherein,

$R^1$ is selected from the group consisting of cycloalkyl, heterocyclyl, aryl and heteroaryl, preferably aryl and heteroaryl, more preferably heteroaryl, wherein the cycloalkyl, heterocyclyl, aryl and heteroaryl are each independently optionally further substituted with one or more of $R^4$; and

R⁴ is as defined in claim 1, preferably alkyl.

7. The compound represented by general formula (I) according to any one of claims 1 to 6, wherein, the compound is selected from the group consisting of:

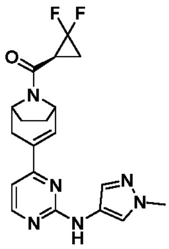 , 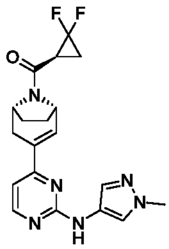 , 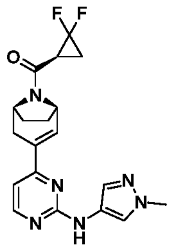 , 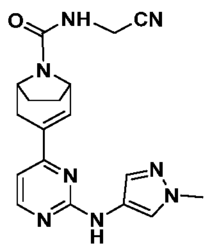 , 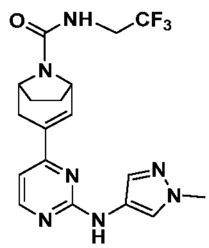 ,

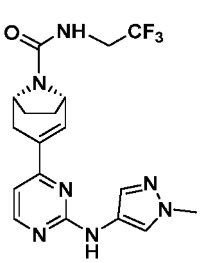 , 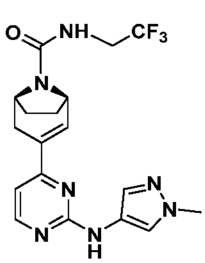 , 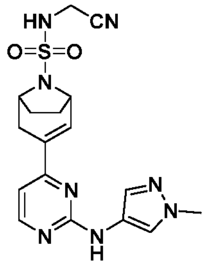 , 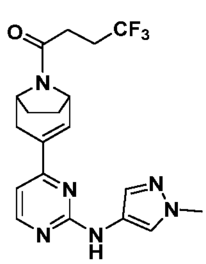 , 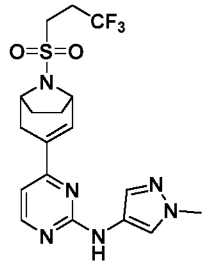 ,

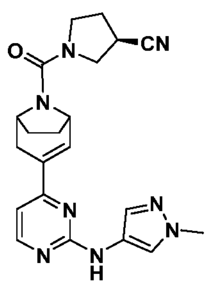 , 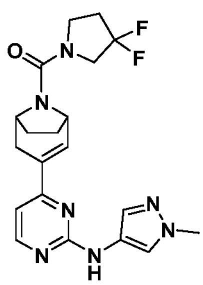 , 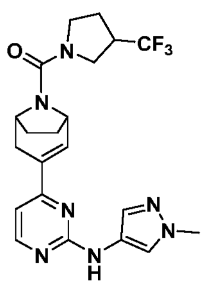 , 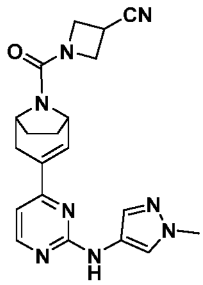 ,

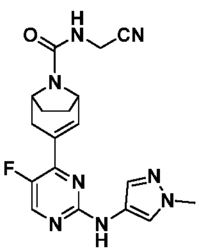 , 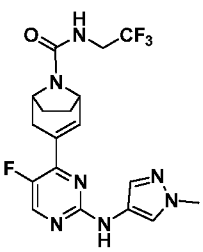 , 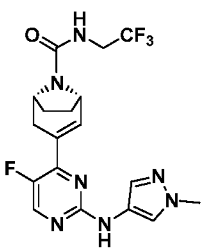 , 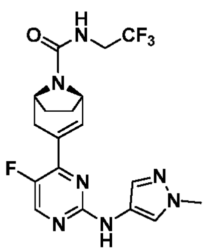 , 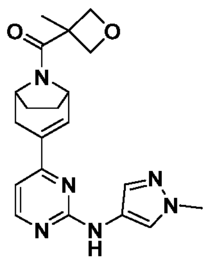 ,

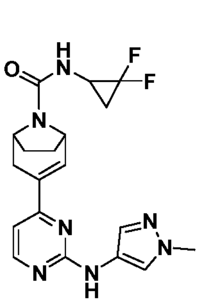 , 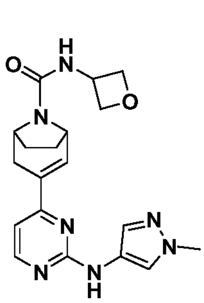 , 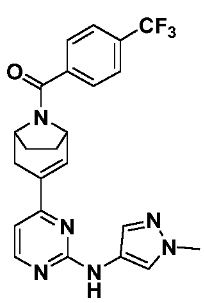 , 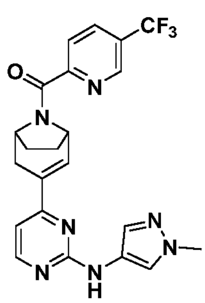 , 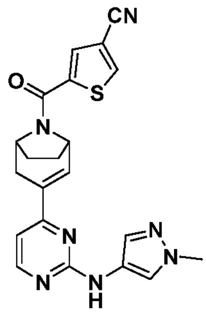 ,

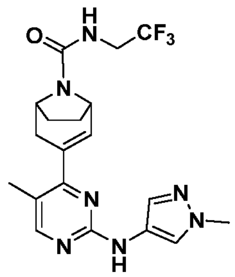 , 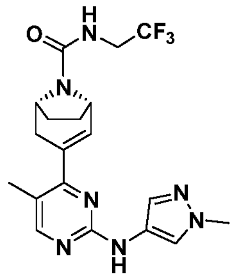 , 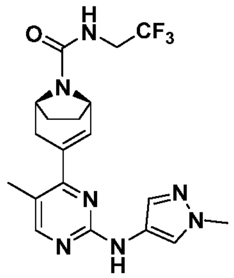 , 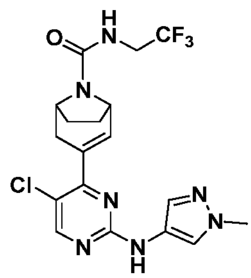 ,

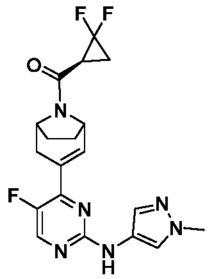 , 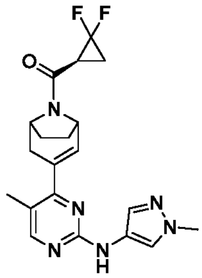 , 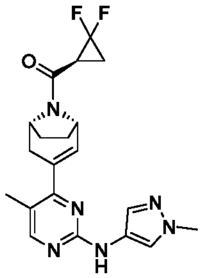 , 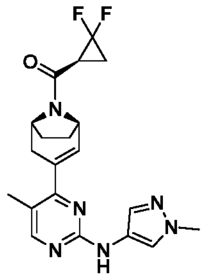 , 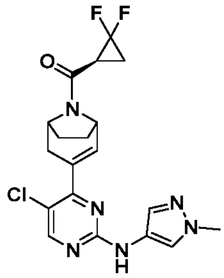 ,

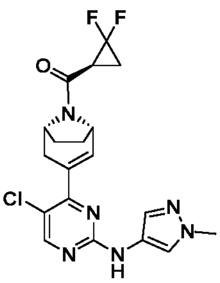 , 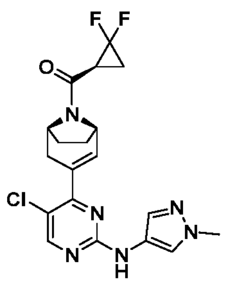 , 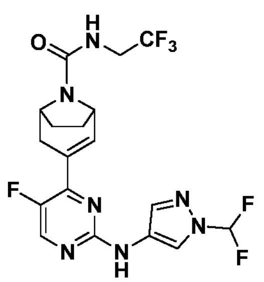 , 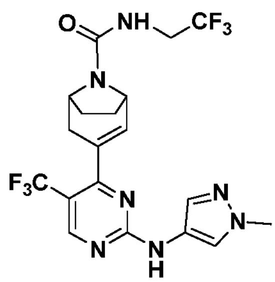 ,

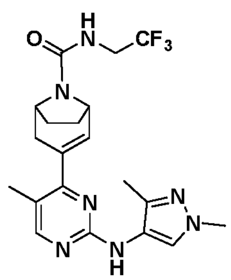 , 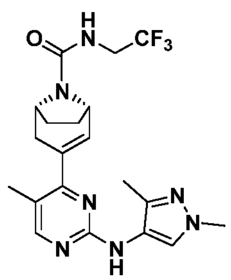 , 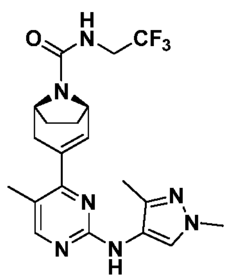 , 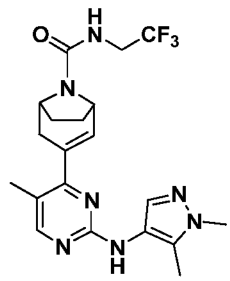 ,

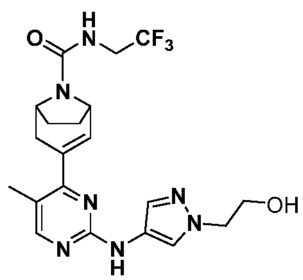 , 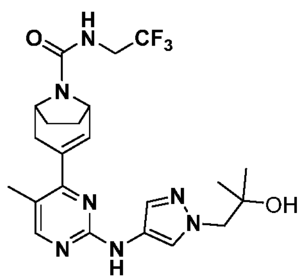 , 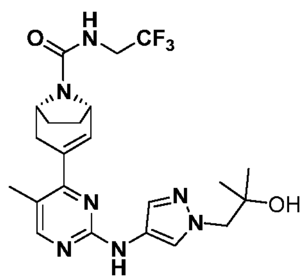 ,

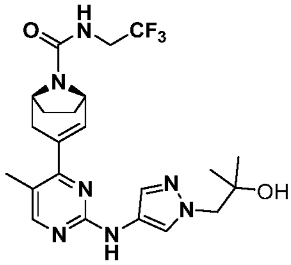 , 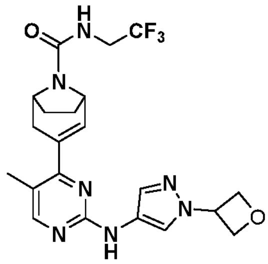 , 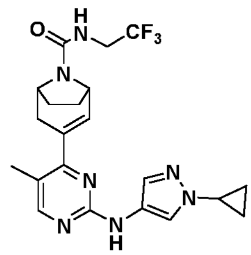 , 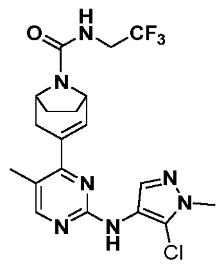 ,

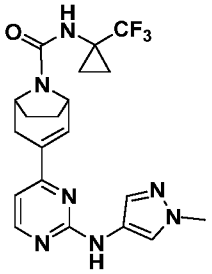 , 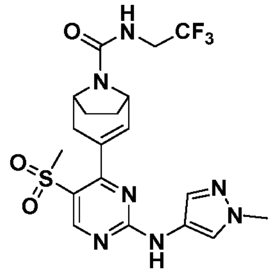 , 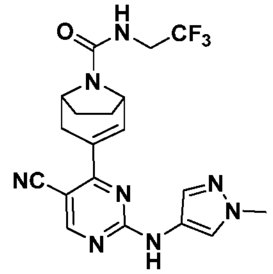 , 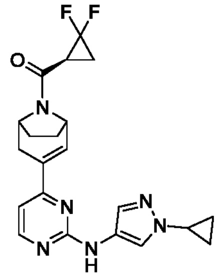 ,

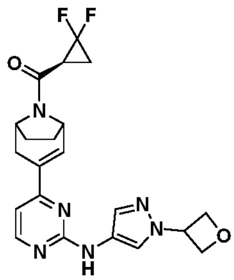 , 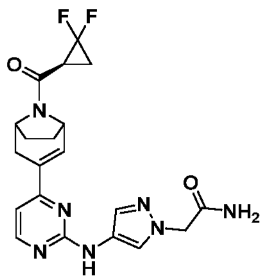 , 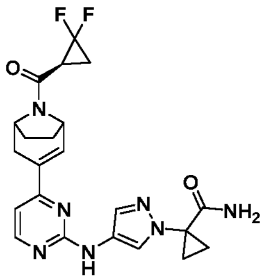 , 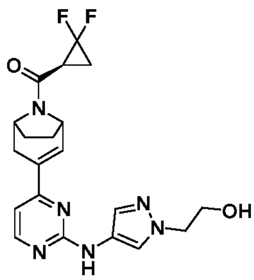 ,

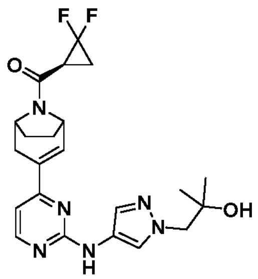 , 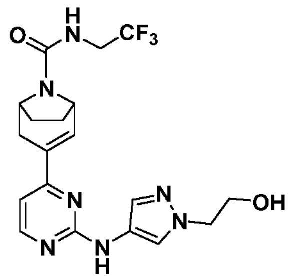 , 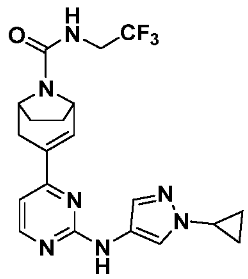 , 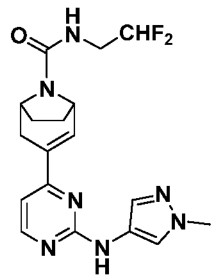 ,

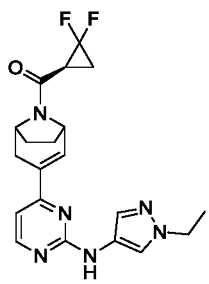 , 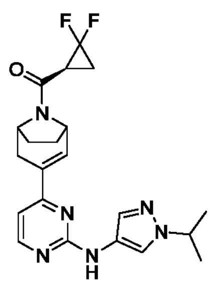 , 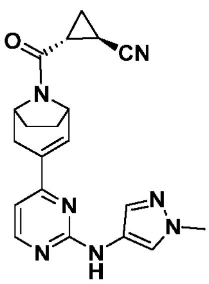 , 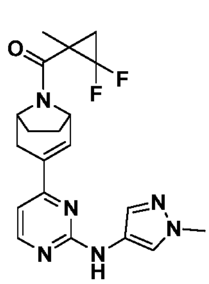 , 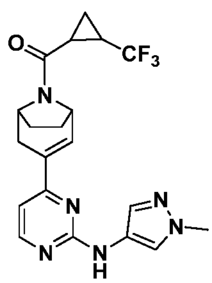 ,

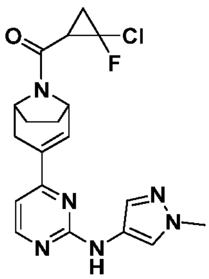,
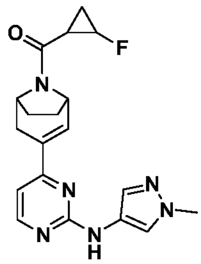,
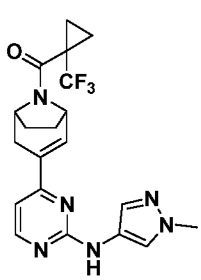,
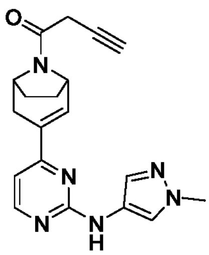,
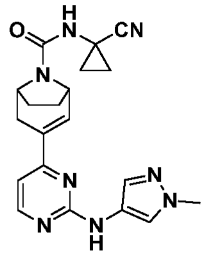,

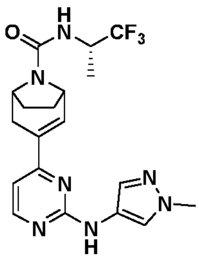,
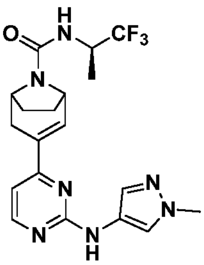,
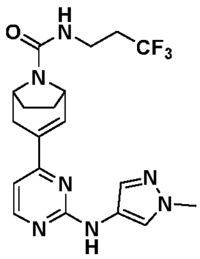,
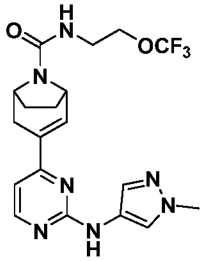,
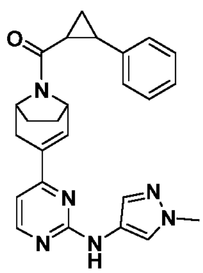,

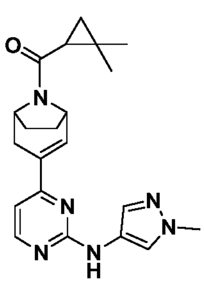,
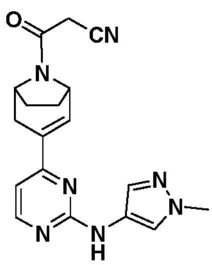,
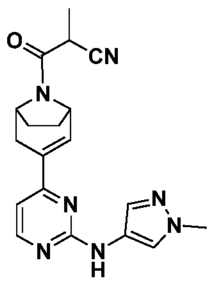,
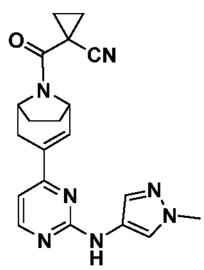,
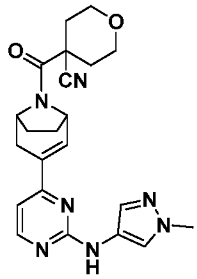,

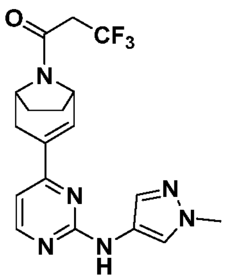,
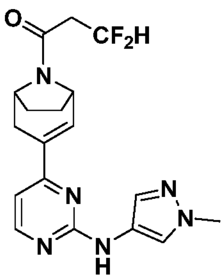,
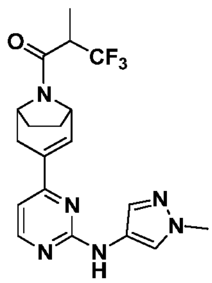,
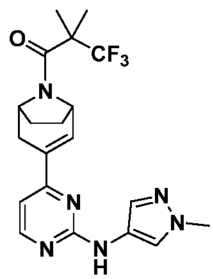,

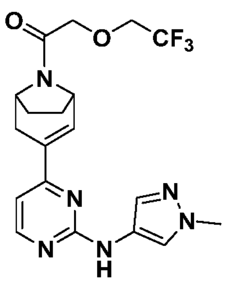,
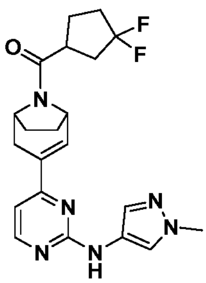,
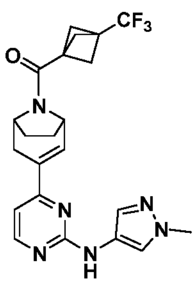,
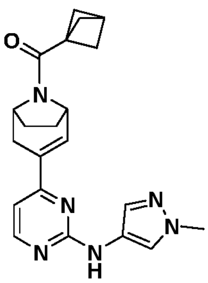,
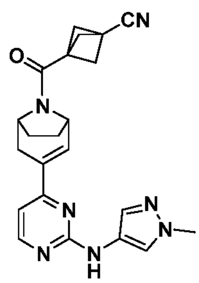,

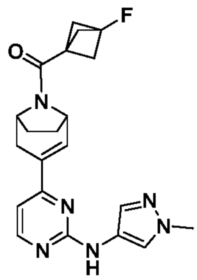, 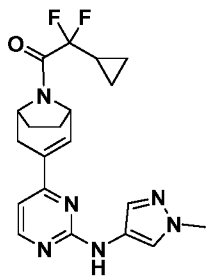, 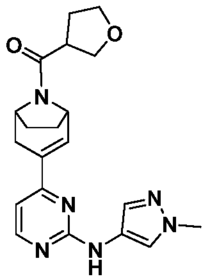, 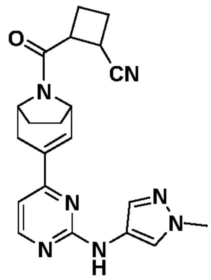, 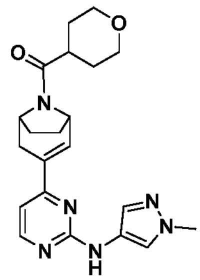,

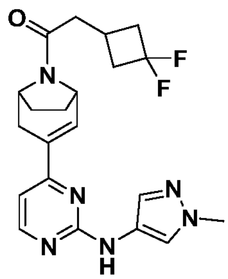, 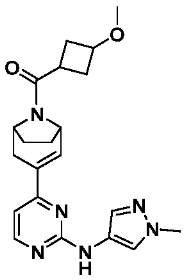, 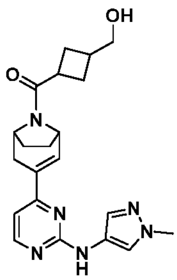, 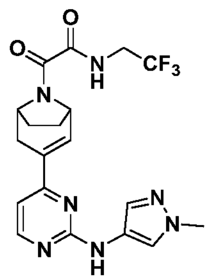, 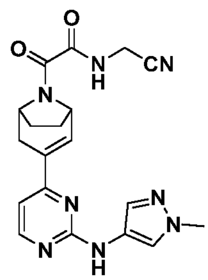,

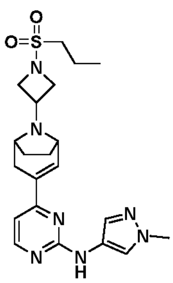, 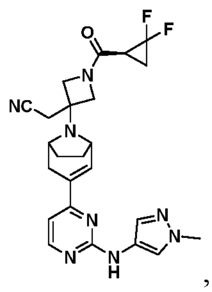, 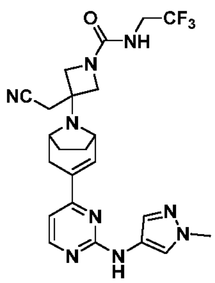 and 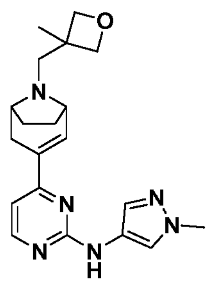.

8. A method for preparing the compound represented by general formula (I) according to any one of claims 1 to 7, comprising the following steps:

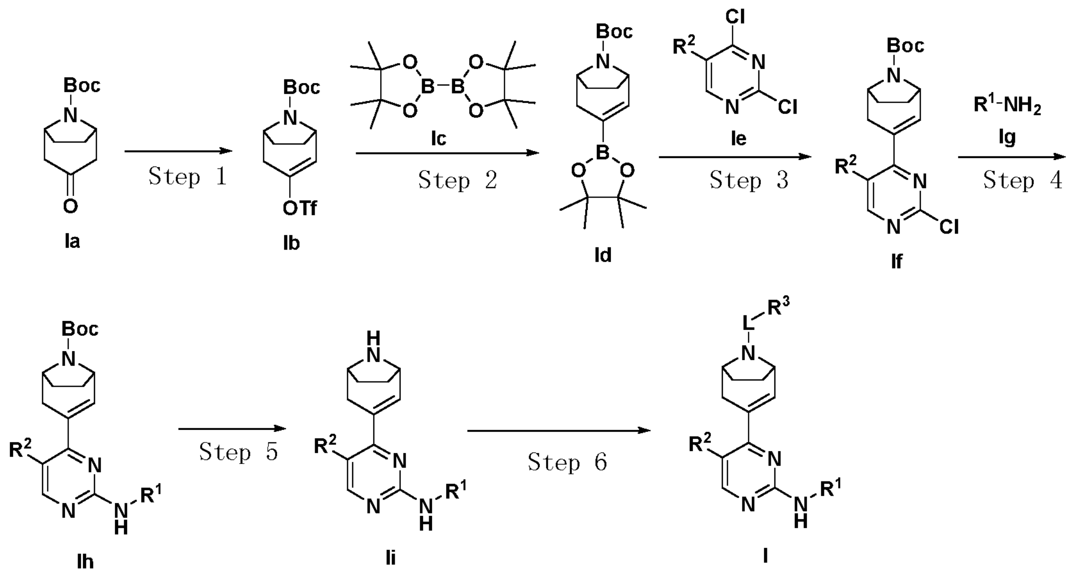


Step 1: Reacting compound Ia with N-phenylbis(trifluoromethanesulfonyl)imide under alkaline conditions to obtain compound Ib, wherein the reagent providing alkaline conditions is preferably potassium hexamethyldisilazide;

Step 2: Reacting compound Ib with pinacol diborate (Ic) under alkaline conditions in the presence of a catalyst to obtain compound Id, wherein the reagent providing alkaline conditions is preferably potassium acetate, and the catalyst is preferably $Pd(dppf)Cl_2$-$CH_2Cl_2$;

Step 3: Reacting compound Id with compound Ie under alkaline conditions in the presence of a catalyst to obtain compound If, wherein the reagent providing alkaline conditions is preferably potassium carbonate, and the catalyst is preferably $Pd(dppf)Cl_2$;

Step 4: Reacting compound If with compound Ig under acidic conditions to obtain compound Ih, wherein the reagent providing acidic conditions is preferably p-toluenesulfonic acid;

Step 5: Compound Ih is subject to a deprotection reaction under acidic conditions to obtain compound Ii, wherein the reagent providing acidic conditions is preferably trifluoroacetic acid;

Step 6: reacting compound Ii with $R^3$-L-X (X=Cl, Br, I, OPh or

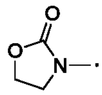
)

under alkaline conditions to obtain a compound of general formula (I), wherein the reagent providing alkaline conditions is preferably triethylamine; or reacting compound Ii with $R^3$-L-OH under alkaline conditions in the presence of a catalyst to obtain a compound of general formula (I), wherein the reagent providing alkaline conditions is preferably DIPEA, and the catalyst is preferably HATU,

wherein, $R^1$, $R^2$, $R^3$ and L are as defined in claim 1.

**9.** A pharmaceutical composition comprising a therapeutically effective amount of the compound represented by general formula (I) according to any one of claims 1 to 7, or a mesomer, racemate, enantiomer, diastereomer thereof, or a mixture thereof, or a prodrug thereof, or a pharmaceutically acceptable salt thereof, as well as a pharmaceutically acceptable carrier.

**10.** Use of the compound represented by general formula (I) according to any one of claims 1 to 7, or a mesomer, racemate, enantiomer, diastereomer thereof, or a mixture thereof, or a prodrug thereof, or a pharmaceutically acceptable salt thereof, or the pharmaceutical composition according to claim 9, in the preparation of JAK1 and TYK2 inhibitors.

**11.** Use of the compound represented by general formula (I) according to any one of claims 1 to 7, or a mesomer, racemate, enantiomer, diastereomer thereof, or a mixture thereof, or a prodrug thereof, or a pharmaceutically acceptable salt thereof, or the pharmaceutical composition according to claim 9, in the preparation of medicaments for the prevention and/or treatment of diseases related to JAK1 and TYK2 activity.

**12.** The use according to claim 11, wherein the disease is selected from the group consisting of inflammation, autoimmune disease and cancer, and the inflammation is preferably selected from the group consisting of rheumatoid arthritis, psoriatic arthritis, inflammatory bowel disease, uveitis, psoriasis and atopic dermatitis; the autoimmune disease is preferably selected from the group consisting of multiple sclerosis and lupus; the cancer is preferably selected from the group consisting of breast cancer, cervical cancer, colon cancer, lung cancer, gastric cancer, rectal cancer, pancreatic cancer, brain cancer, skin cancer, oral cancer, prostate cancer, bone cancer, kidney cancer, ovarian cancer, bladder cancer, liver cancer, fallopian tube tumor, ovarian tumor, peritoneal tumor, melanoma, solid tumor, glioma, glioblastoma, hepatocellular carcinoma, mastoid nephroma, head and neck tumors, leukemia, lymphoma, myeloma and non-small cell lung cancer.

## INTERNATIONAL SEARCH REPORT

International application No.
**PCT/CN2020/107054**

**A. CLASSIFICATION OF SUBJECT MATTER**

C07D 239/42(2006.01)i; C07D 403/12(2006.01)i; A61K 31/506(2006.01)i; A61P 37/00(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

DWPI; SIPOABS; CNKI; CNABS; STNext; ISI Web of Science: 中国医药研究开发中心有限公司, JAK1, TYK2, Janus, 抑制剂, 免疫, 癌症, 肿瘤, immun+, cancer, tumor

**C. DOCUMENTS CONSIDERED TO BE RELEVANT**

| Category* | Citation of document, with indication, where appropriate, of the relevant passages | Relevant to claim No. |
|---|---|---|
| PX | CN 110862376 A (JIAXING TEKELUO BIOTECHNOLOGY CO., LTD.) 06 March 2020 (2020-03-06) description, paragraph 6, claim 5, description, paragraph 87, paragraphs 93-94, paragraphs 261-262, paragraph 281 | 1-12 |
| A | WO 2012062704 A1 (CELLZOME LTD. et al.) 18 May 2012 (2012-05-18) abstract, description, table 7, compound 45, claims 1-30 | 1-12 |
| A | US 2016207906 A1 (CARNA BIOSCIENCES INC.) 21 July 2016 (2016-07-21) abstract, and claims 1-20 | 1-12 |
| A | WO 2017035366 A1 (INCYTE CORP.) 02 March 2017 (2017-03-02) abstract, claims 1-80 | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 October 2020** | **09 November 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2020/107054**

| Patent document cited in search report | Publication date (day/month/year) | Patent family member(s) | Publication date (day/month/year) |
|---|---|---|---|
| CN 110862376 A | 06 March 2020 | None | |
| WO 2012062704 A1 | 18 May 2012 | AU 2011328237 A1 | 23 May 2013 |
| | | CA 2815330 A1 | 18 May 2012 |
| | | CN 103298794 A | 11 September 2013 |
| | | EP 2638018 A1 | 18 September 2013 |
| | | JP 2014500254 A | 09 January 2014 |
| US 2016207906 A1 | 21 July 2016 | WO 2015033888 A1 | 12 March 2015 |
| | | AU 2014316247 A1 | 31 March 2016 |
| | | IL 244393 D0 | 21 April 2016 |
| | | RU 2016112314 A | 05 October 2017 |
| | | EP 3042899 A1 | 13 July 2016 |
| | | CN 105745202 A | 06 July 2016 |
| | | KR 20160046917 A | 29 April 2016 |
| | | JP WO2015033888 A1 | 02 March 2017 |
| | | CA 2922939 A1 | 12 March 2015 |
| WO 2017035366 A1 | 02 March 2017 | US 2020181151 A1 | 11 June 2020 |
| | | US 2017057965 A1 | 02 March 2017 |
| | | US 10053465 B2 | 21 August 2018 |
| | | US 10519163 B2 | 31 December 2019 |
| | | US 2019031663 A1 | 31 January 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *J. Immunol.,* 2015, vol. 194, 21
- *J. Med. Chem.,* 2014, vol. 57, 5023
- *Curr. Opin. Rheumatol.,* 2014, vol. 26, 237
- *J. of Immunology,* 2010, vol. 184, 5298-5307
- *Proc Soc Exp Bio Med,* 1962, vol. 111, 544-547